# EUROPEAN PATENT APPLICATION

(11) **EP 1 584 686 A1**
(43) Date of publication of application: **12.10.2005**
(21) Application number: 05014080.5
(22) Date of filing: 30.12.1999
(51) Int. Cl.: C12N 15/29, C12N 5/06, C07K 14/42, G01N 33/566, A61K 38/16, A61P 39/00

(54) **Progenitor cell preservation factors and related methods and products**

(62) Divisional of application: 99967798.2
(71) Applicant: ImClone Systems Incorporated, New York, NY 10014 (US); The Regents of the University of California, Oakland, CA 94607 (US)
(72) Inventor: Colucci, M. Gabriella, 20122 Napoli (IT); Chrispeels, Maarten J, La Jolla CA 92037 (US); Moore, Jeffrey G., Brookline Massachusetts, 02445 (US)
(74) Representative: Grund, Martin

(57) **Abstract**

Disclosed is the FRIL, family of progenitor cell preservation factors and nucleid acids encoding the same. FRIL family members preserve progenitor cells both *in vivo* and *ex vivo*. FRIL family members find use as therapeutics for alleviating and/or reducing the hematopoietic progenitor cell-depleting activity of many cancer therapeutics. FRIL family members are also useful for isolating rare, primitive progenitor cells.

## Description

This application is a continuation in part of U.S. Ser. No. 08/881,189, filed June 24, 1997, the entire contents of which are hereby incorporated by reference.

### BACKGROUND OF THE INVENTION

### Field of the Invention

The invention relates to the preservation of progenitor cells. More specifically, the invention relates to the *in vivo* or *ex vivo* preservation of progenitor cells, such as hematopoietic progenitor cells.

### Summary of the Related Art

The wide variety of functionally and phenotypically different types of cells in a multi-cellular eukaryotic organism results in part from the proliferation and differentiation of rare and mostly quiescent populations of progenitor cells. For example, hematopoiesis involves the process of producing a balanced supply of different blood cells from such progenitor cells found in the adult bone marrow. The development of other cell types also depends upon production of the differentiated cells from such progenitor cells.

Progenitor cells are activated by signals, such as cell-cell contact or soluble regulators, to generate daughter cells that are identical to the parent (*i*.*e*., self-renewal of the parent) and/or to generate daughter cells that are more differentiated than the parent, thus beginning an irreversible process that ends with the production of differentiated, functional cells. In the process of hematopoiesis, differentiation is coupled to proliferation as a progenitor cell gives rise to more differentiated daughter cells that progressively become committed to producing only one blood cell type. The enormous activation of hematopoietic progenitor cells needed to meet the body's daily requirement for hundreds of billions of new mature blood cells is directed by potent soluble regulators (*e*.*g*., colony stimulating factors and cytokines) acting upon the hematopoietic progenitor cells themselves, and their more differentiated daughter cells.

Although progenitor cells eventually produce so many of the mature cells of the body, they occur only rarely. Moreover, typically the more primitive *(i.e.,* undifferentiated) the progenitor cell, the more rare the progenitor cell. For example, the currently believed most primitive of the hematopoietic progenitor cells, which are called hematopoietic stem cells, occur at a frequency of only from about 1 in 10,000 to about 1 in 100,000 of the cells in the bone marrow. Hematopoietic stem cells have the capacity to generate more than 10¹³ mature blood cells of all lineages, including other progenitor cells which, although more differentiated than hematopoietic stem cells, are themselves capable of giving rise to several different types of mature blood cells.

Hematopoietic stem cells are responsible for sustaining blood cell production over the life of an animal. The small population of hematopoietic stem cells is sufficient to produce all the mature blood cells in a healthy individuals; however, some unhealthy individuals suffer from a lack of a sufficient number of progenitor cells and/or mature blood cells. For example, cancer patients receiving chemotherapeutic or radiotherapy treatments designed to kill the rapidly dividing cancer cells also suffer from the depletion of white blood cells and platelets, thus exposing these patients to life threatening opportunistic infections and bleeding episodes. Indeed, this hematopoietic progenitor cell-depleting activity is the dose-limiting factor for most of these chemotherapeutic and radiotherapeutic agents.

Many cancer patients are routinely treated with cytokines, including G-CSF, GM-CSF, SCF, Erythropoietin, and IL-11, to accelerate restoration of hematopoiesis following chemotherapy (Moore, M.A., *Blood* 78:1-19,1991). However, these cytokines lead to the irreversible differentiation of hematopoietic progenitor cells, including hematopoietic stem cells, into more differentiated daughter cells. Thus, better protection of hematopoietic progenitor cells is needed during chemotherapy.

Workers in the field have attempted to use cytokines in mice to protect progenitors from the toxicity of chemotherapy (Neta et al., *J. Immunol.* 136: 2483-2485,1986; Neta et al., *J. Immunol*. 140: 108-111, 1988; Neta et al., *J. Exp. Med.* 173: 1177-1182,1991; de Haan et al., *Blood* 87: 4581-4588, 1996; Lyman and Jacobsen, *Blood* 91:1101-1134,1998; Dalmau et al., *Bone Marrow Transplant.* 12:551-563,1993; Grzegorzewski et al., *J. Exp. Med*. 180: 1046-1057,1994; Grzegorzewski et al., *Blood* 94:1066a (Abstr.)1999). Marshall et al. (Euro. *J. Cancer* 34:1023-1029,1998) and Gilmore et al. *(Exp. Hematol.* 27: 195-202,1999) describe the use in clinical trials of a chemokine that allegedly inhibits progenitor cell proliferation, MIP1-α, as a chemoprotectant. Marshall (Marshall, A., *Nat.. Biotechnol.* 16: 129, 1999) describes the use of MPIF-1, a chemokine that allegedly inhibits progenitor cell proliferation, in clinical trials as a chemoprotectant.

There are several drawbacks to using chemokines, cytokines, and other immunoregulators as chemoprotectants during the chemotherapeutic or radiotherapeutic treatment of cancer patient. These drawbacks include the cost of production and toxicity to the patient.

Therefore, there is a need for improved reagents that are non-toxic and inexpensive to produce for use in preserving progenitor cells.

### BRIEF SUMMARY OF THE INVENTION

The invention provides a family of factors that preserve progenitor cells. Members of this family, the FRIL family, are non-toxic, inexpensively produced reagents that preserve progenitor cells. The invention provides compositions comprising at least one member of the FRIL family, as well as methods for using members of the FRIL family to preserve progenitor cells both *in vivo* and *ex vivo*.

Accordingly, in a first aspect, the invention provides an essentially pure composition of one or more members of the FRIL family of progenitor cell preservation factors.

In certain embodiments of the first aspect of the invention, the FRIL family member is from a legume. In some embodiments, the legume is *Dolichos lab lab*. In some embodiments, the legume is *Phaseolus vulgaris*. In some embodiments, the legume is *Sphenostylis stenocarpa.*

In certain embodiments of the first aspect of the invention, the FRIL family member is a mutant derived from a second member of the FRIL family, wherein the mutant is selected from the group consisting of a substitution mutant, a deletion mutant, an addition mutant, or a combination thereof.

In certain embodiments of the first aspect of the invention, the FRIL family member is a fusion protein comprising a first portion and a second portion, wherein the first portion is derived from a second member of the FRIL family.

In a second aspect, the invention provides a recombinant nucleic acid molecule encoding a composition of a member of the FRIL family of progenitor cell preservation factors.

In a third aspect, the invention provides a pharmaceutical formulation comprising an essentially pure composition of one or more members of the FRIL family of progenitor cell preservation factors and a pharmaceutically acceptable carrier.

In certain embodiments of the third aspect of the invention, administration of a therapeutically effective amount of the formulation to a patient suffering from a condition whereby the patient's hematopoietic progenitor cells are depleted alleviates and/or reduces the condition in the patient.

In certain embodiments of the third aspect of the invention, administration of a therapeutically effective amount of the formulation to a patient prior to treatment of the patient with a therapeutic treatment having a hematopoietic progenitor cell-depleting activity alleviates and/or reduces the hematopoietic progenitor cell-depleting activity of the therapeutic treatment in the patient. In certain embodiments, the patient is a human or is a domesticated mammal. In some embodiments, the patient has cancer. In some embodiments, the therapeutic treatment is a radiotherapeutic or a chemotherapeutic treatment, including, without limitation, cytarabine (Ara-C), doxorubicin (Dox), or 5-fluorouracil (5-FU), or a combination of a radiotherapeutic and a chemotherapeutic.

In a fourth aspect, the invention provides a method for alleviating or reducing the hematopoietic progenitor cell-depleting activity of a therapeutic treatment in a patient, comprising administering to the animal a therapeutically effective amount of a composition of a FRIL family member prior to administration of the therapeutic treatment to the patient.

In certain embodiments of the fourth aspect, the patient is a human or is a domesticated mammal. In some embodiments, the patient has cancer. In some embodiments, the therapeutic treatment is a radiotherapeutic or a chemotherapeutic treatment, including, without limitation, cytarabine (Ara-C), doxorubicin (Dox), or 5-fluorouracil (5-FU), or a combination of a radiotherapeutic and a chemotherapeutic.

In a fifth aspect, the invention provides a method for isolating a population of progenitor cells, comprising contacting a population of cells with a plurality of FRIL family member molecules, and separating the unbound cells, wherein the cells bound to the FRIL family member molecules are an isolated population of progenitor cells. Preferably, the isolated population of progenitor cells is from a human.

In certain embodiments of the fifth aspect of the invention, the FRIL family member molecules are detectably labeled. In certain embodiments, the detectably labeled FRIL family member molecules are labeled with a chromophore. In certain embodiments, the unbound cells are separated by using a flow cytometry cell sorter to sort the population of cells contacted with the FRIL family member molecules detectably labeled with a chromophore.

In certain embodiments of the fifth aspect, the FRIL family member molecules is immobilized on a solid support. In some embodiments, the solid support is a bead, such as a magnetic bead. In some embodiments, the unbound cells are separated by applying a magnet to the population of cells contacted with the FRIL family member molecules immobilized on the magnetic bead. In further embodiments, the population of cells bound to the FRIL family member molecules immobilized on a magnetic bead is rinsed with a physiologically acceptable solution while the magnet is applied.

In certain embodiments of the fifth aspect, the solid support is the bottom of a tissue culture plate. In some embodiments, the unbound cells are separated by rinsing the population of cells contacted with the FRIL family member immobilized on the bottom of a tissue culture plate with a physiologically acceptable solution.

In preferred embodiments of the fifth aspect of the invention, the isolated population of progenitor cells is a population of hematopoietic progenitor cell. In various embodiments, the population of cells is whole blood, umbilical cord blood, bone marrow cells, or fetal liver cells.

In certain embodiments of the fifth aspect of the invention, the population of cells is a sorted population of cells, wherein a cell of the sorted population does not express a cell surface molecule selected from the group consisting of CD11b, CD11c, and CD38. In certain embodiments, the sorted population of cells is sorted by flow cytometry or by magnetic bead selection.

In a sixth aspect, invention provides an isolated population of progenitor cells isolated by a method comprising contacting a population of cells with a plurality of FRIL family member molecules, and separating the unbound cells, wherein the cells bound to the FRIL family member molecules are an isolated population of progenitor cell. Preferably, the progenitor cell is from a human.

In certain embodiments of the sixth aspect, the cells of the isolated population do not express CD34. In certain embodiments, the cells of the isolated population express a receptor tyrosine kinase selected from the group consisting of from FLK1, FLT1, FLT3, FLT4, and Kit. In some embodiments, the cells of the isolated population express a cell surface molecule selected from the group consisting of CD11b and CD11c. In preferred embodiments, the cells of the isolated population express FLT3.

In various embodiments of the sixth aspect of the invention, the cells of the isolated population are hemangioblasts, messenchymal stem cells, bone progenitor cells, hepatic progenitor cells, endothelial progenitor cells, hematopoietic progenitor cells, embryonal stem cells, brain progenitor cells, or dendritic progenitor cells. Preferably, the cells of the isolated population are hematopoietic progenitor cells.

In certain embodiments of the sixth aspect of the invention, where the cells of the isolated population are hematopoietic progenitor cells, transplantation of the isolated population into an animal lacking a population of hematopoietic progenitor cells sufficient to enable survival of the animal reconstitutes the animal, wherein the transplanted animal survives. In certain embodiments, the hematopoietic progenitor cells are from a human or a mouse and wherein the animal is a mouse. In some embodiments, the mouse is a SCID mouse or the mouse is sublethally irradiated or the mouse is treated with a sublethal dose of a chemotherapeutic. In certain embodiments, the hematopoietic progenitor cells are from a human and the animal is a human. In some embodiments, the human is a cancer patient receiving a treatment that depletes the patient's hematopoietic progenitor cells. In some embodiments, the treatment is a radiotherapeutic or a chemotherapeutic treatment, including, without limitation, cytarabine (Ara-C), doxorubicin (Dox), or 5-fluorouracil (5-FU), or a combination of a radiotherapeutic and a chemotherapeutic.

In a seventh aspect, the invention provides a method for preserving progenitor cells *ex vivo* comprising contacting a population of cells comprising at least one progenitor cell with an effective amount of a composition of a FRIL family member for an effective period of time, wherein the progenitor cells in the population are rendered quiescent.

In certain embodiments of the seventh aspect, the progenitor cells are from a human. In certain embodiments, the population of cells is bone marrow cells. In some embodiments, the non-progenitor cells in the population of cells differentiate or die.

In certain embodiments of the seventh aspect, the population of cells is removed from a cancer patient prior to treatment of the cancer patient with a therapeutic treatment having a hematopoietic progenitor cell-depleting activity. In some embodiments, the therapeutic treatment is a radiotherapeutic or a chemotherapeutic treatment, including, without limitation, cytarabine (Ara-C), doxorubicin (Dox), or 5-fluorouracil (5-FU), or a combination of a radiotherapeutic and a chemotherapeutic.

In an eighth aspect, the invention provides a method for preserving progenitor cells *in vivo,* comprising administering to a patient an effective amount of a composition of a FRIL family member for an effective period of time, wherein the progenitor cells in the patient are rendered quiescent. Preferably, the patient is a human or a domesticated animal.

In certain embodiments of the eighth aspect of the invention, the patient is a cancer patient. In some embodiments, the effective amount of the composition of a FRIL family member is administered prior to the treatment of the patient with a therapeutic treatment having a hematopoietic progenitor cell-depleting activity. In some embodiments, the therapeutic treatment is a radiotherapeutic or a chemotherapeutic treatment, including, without limitation, cytarabine (Ara-C), doxorubicin (Dox), or 5-fluorouracil (5-FU), or a combination of a radiotherapeutic and a chemotherapeutic.

In a ninth aspect, the invention provides a method for identifying a progenitor cell, comprising contacting a candidate cell with a FRIL family member molecule, wherein binding of the candidate cell to the FRIL family member molecule identifies the candidate cell as a progenitor cell.

In certain embodiments of the ninth aspect, the candidate cell is in a population of cells. In certain embodiments, the candidate cell is from a human.

In a tenth aspect, the invention provides a progenitor cell identified by a method comprising contacting a candidate cell with a FRIL family member molecule, wherein binding of the candidate cell to the FRIL family member identifies the candidate cell as a progenitor cell.

In an eleventh aspect, the invention provides a method for identifying a composition of a member of the FRIL family of progenitor cell preservation factors, comprising contacting a candidate compound with a glycosylated extracellular domain of an FLT3 receptor, wherein the glycosylation pattern of the extracellular domain of the FLT3 receptor is the same as the glycosylation pattern of an extracellular domain of a normally glycosylated FLT3 receptor, wherein a candidate compound that binds the glycosylated extracellular domain of the FLT3 receptor is identified as a composition of a FRIL family member.

In certain embodiments of the eleventh aspect, the candidate compound is a lectin. In certain embodiments, the lectin is synthetic. In certain embodiments, the lectin is from a legume.

In a twelfth aspect, the invention provides an essentially pure composition of a FRIL family member identified by the method comprising contacting a candidate compound with a glycosylated extracellular domain of an FLT3 receptor, wherein the glycosylation pattern of the extracellular domain of the FLT3 receptor is the same as the glycosylation pattern of an extracellular domain of a normally glycosylated FLT3 receptor, wherein a candidate compound that binds the glycosylated extracellular domain of the FLT3 receptor is identified as a member of the FRIL family.

According to the invention, compositions of a FRIL family member may be used as therapeutic agents to preserve progenitor cells in patients, such as cancer patients receiving chemotherapy, who have suffer from a condition that diminishes their progenitor cells. For example, compositions of a FRIL family member may be administered with a pharmaceutically-acceptable carrier (*e*.*g*., physiological sterile saline solution) via any route of administration to a cancer patient receiving chemotherapy in an attempt to reduce the progenitor cell-depleting effects of the chemotherapeutic so that the patient can receive a higher dose of the chemotherapeutic and, preferably, recover from cancer. Pharmaceutically-acceptable carriers and their formulations are well-known and generally described in, for example, Remington's Pharmaceutical Sciences (18th Edition, ed. A. Gennaro, Mack Publishing Co., Easton, PA, 1990).

### BRIEF DESCRIPTION OF THE DRAWINGS

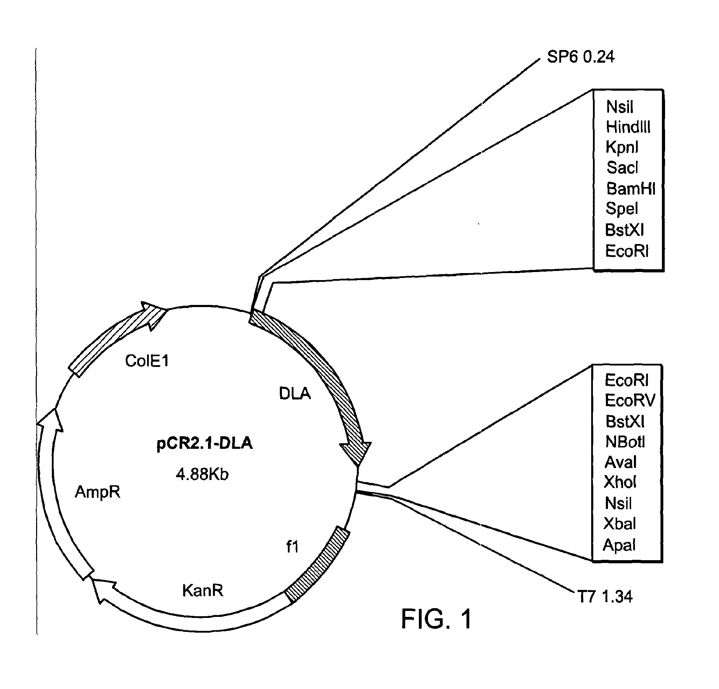

Figure 1 is a map of a cloning vector pCR2.1-DLA manufactured by ligating a cDNA according to the invention in the *Eco*RI site of the cloning vector pCR2.1.

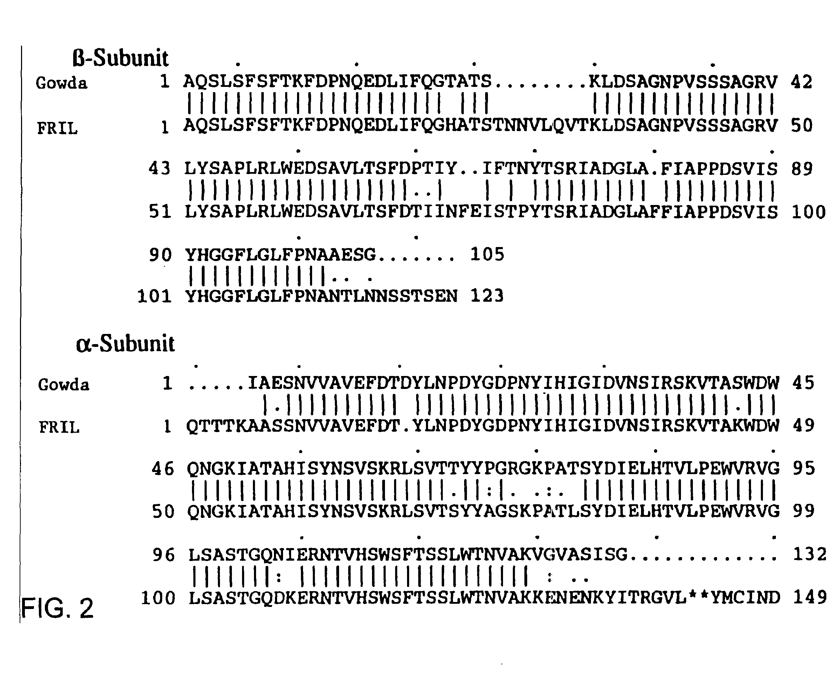

Figure 2 shows a direct amino acid sequence comparison of the mannose lectin described by Gowda et al. (*J Biol Chem* 269:18789-18793,1994) and the derived amino acid sequence of Dl-FRIL, a representative, non-limiting FRIL family member of the invention, encoded by a representative, non-limiting nucleic acid of the invention.

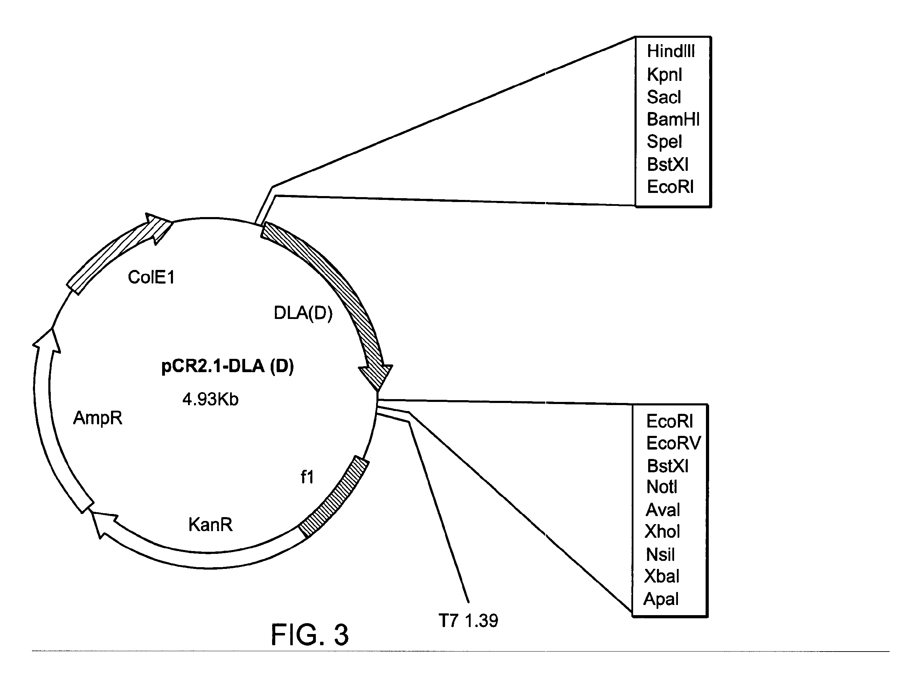

Figure 3 is a map of a cloning vector pCR2.1-DLA(D) manufactured by ligating a mutated cDNA in the EcoRI site of the cloning vector pCR2.1.

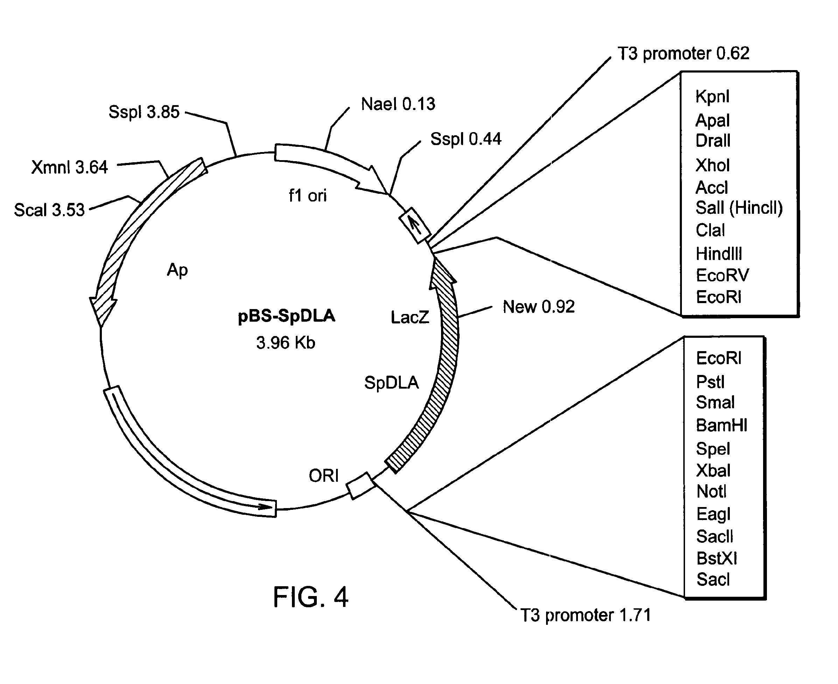

Figure 4 is a map of a cloning vector pBS-SpDLA manufactured by ligating a recombinant fragment in the *Eco*RI site of the cloning vector pBluescript SK+.

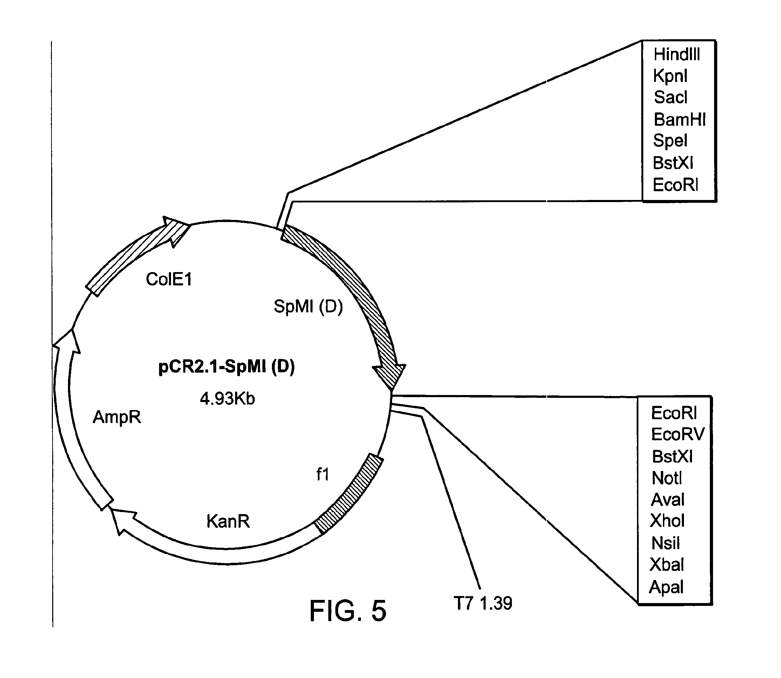

Figure 5 is a map of a cloning vector pCR2.1-SpM1(D) manufactured by ligating a mutated recombinant clone in the *Eco*RI site of the cloning vector pCR2.1.

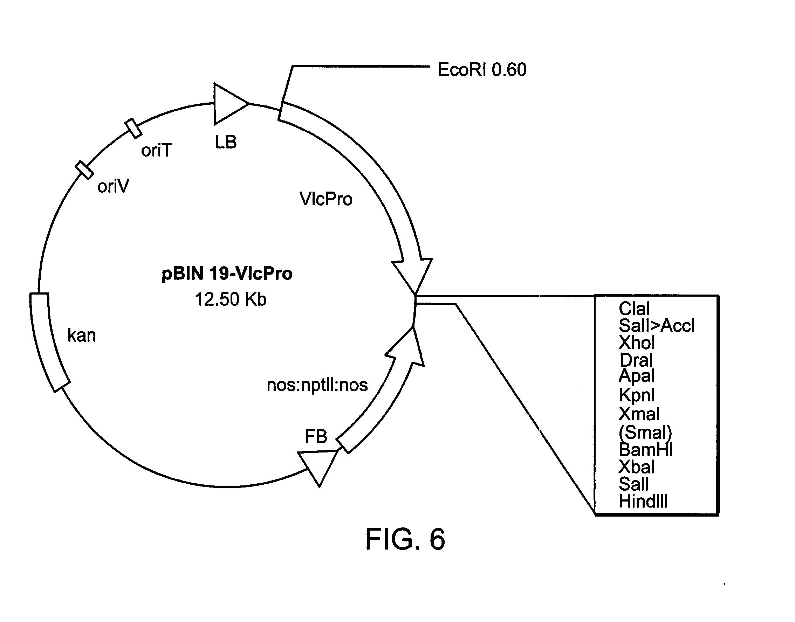

Figure 6 is a map of a recombinant expression vector pBIN-VicPro manufactured by subcloning the vicilin promoter obtained from the pCW66 vector in the *Eco*RI/*Cla*I site of the plant binary vector pBIN19 for *Agrobacterium*-mediated transformation.

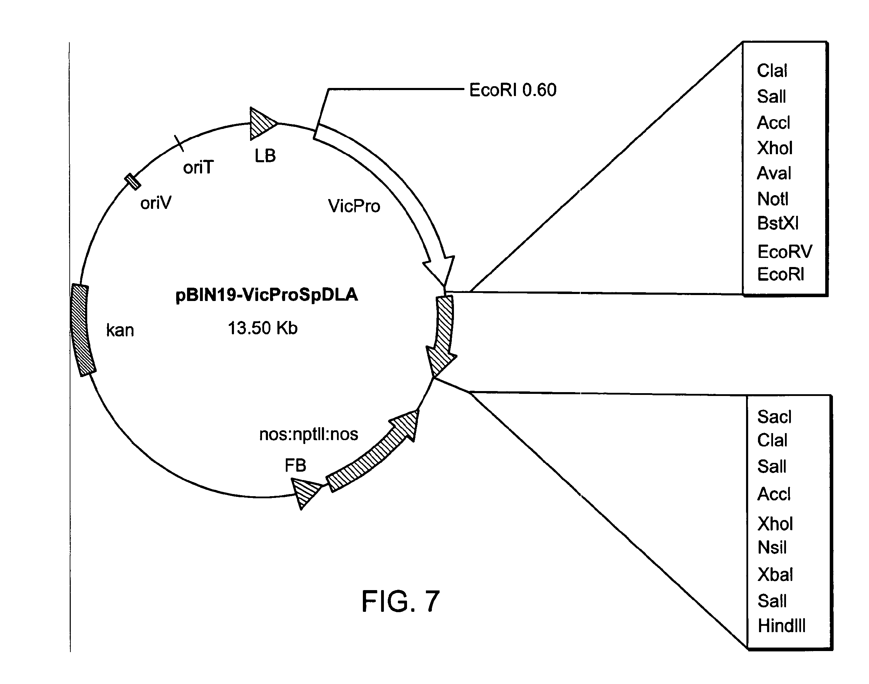

Figure 7 is a map of a recombinant expression vector pBINVicPro-SpDLA manufactured by ligating a recombinant fragment in the *Eco*RI/*Sac*I site of the pBINVicPro vector.

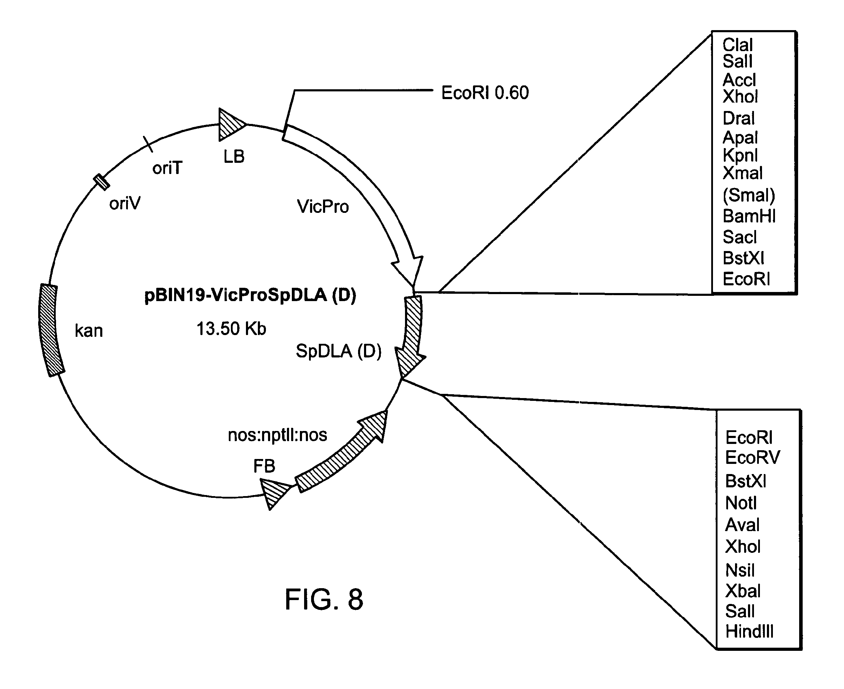

Figure 8 is a map of a recombinant expression vector pBINVicPro-SpDLA(D) manufactured by ligating a mutated recombinant clone in the *Eco*RI site of the pBINVicPro vector.

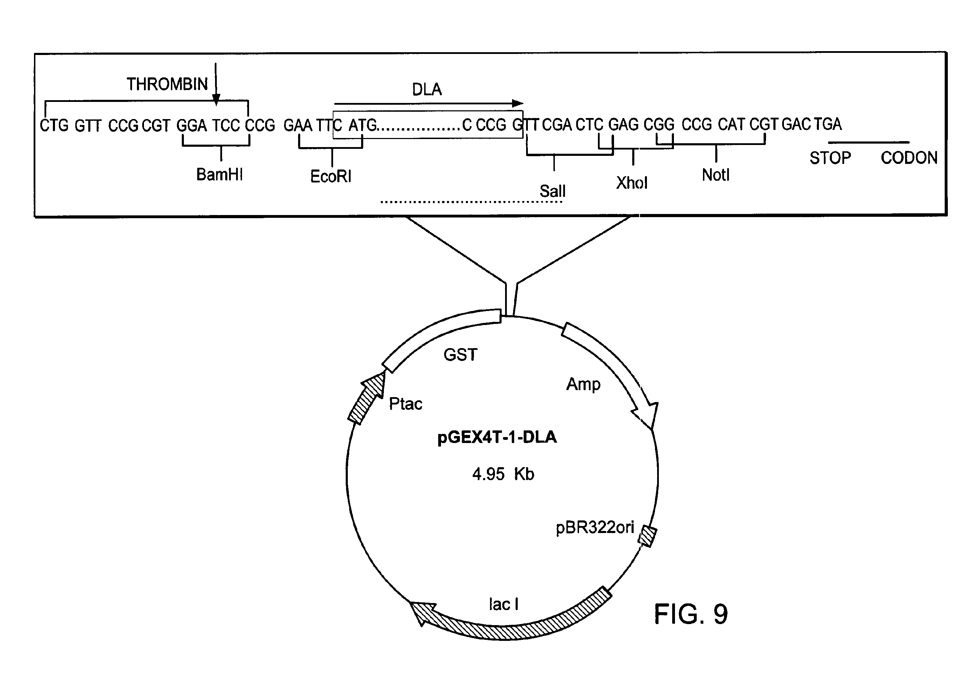

Figure 9 is a map of a recombinant expression vector pGEX4T-1-DLA manufactured by ligating a wild-type cDNA clone in the *Eco*RI/*Sal*I site of the *E. coli* expression vector pGEX4T-1.

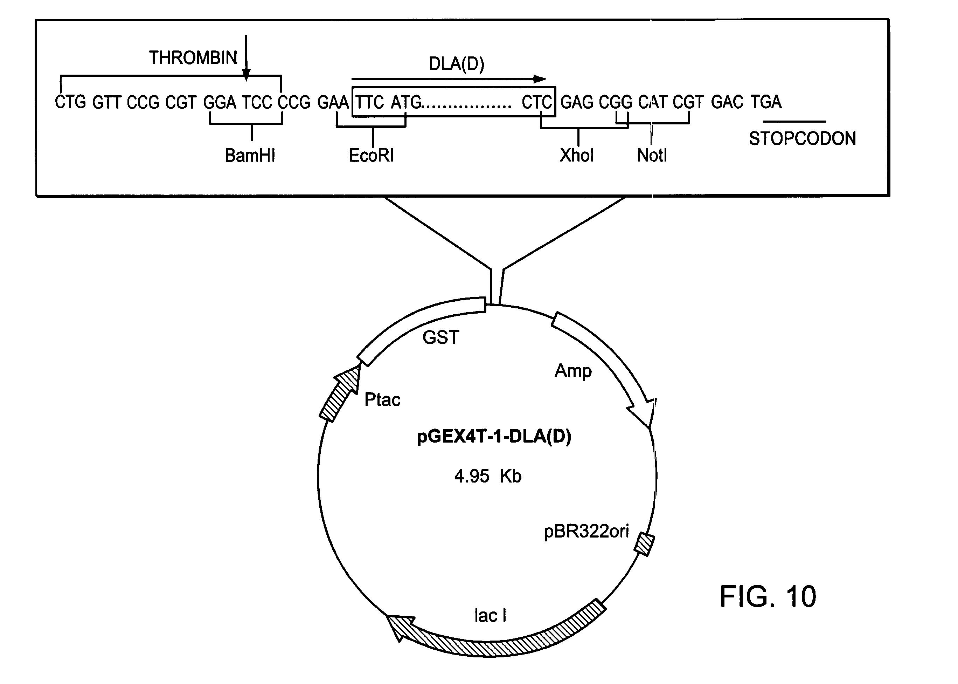

Figure 10 is a map of a recombinant expression vector pGEX4T-1-DLA(D) manufactured by ligating a mutant cDNA clone in the *Eco*RI/*Xho*I site of the *E*. *coli* expression vector pGEX4T-1.

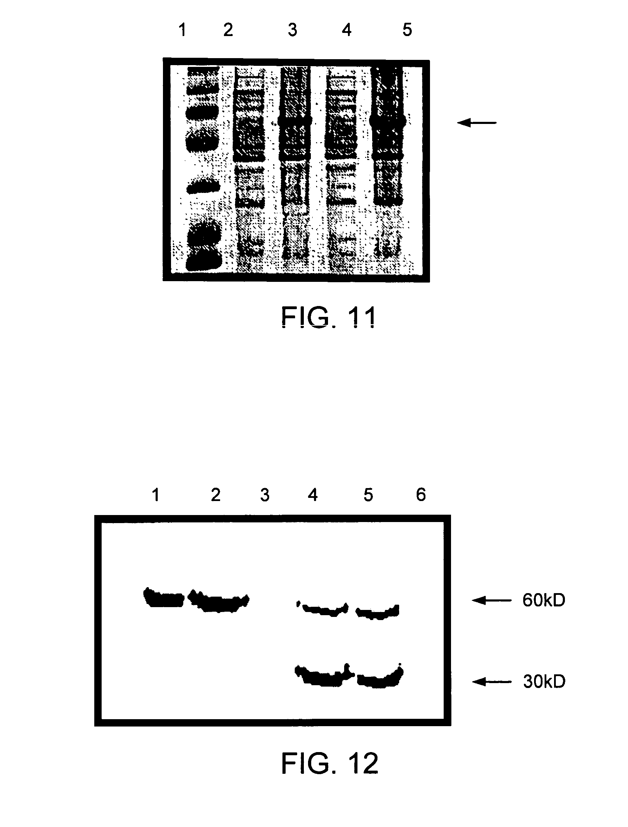

Figure 11 is a representation of an electrophoretogram of a Southern blot of total protein extracts of *E. coli* cells transformed with the recombinant expression vectors pGEX4T-1-DLA and pGEX4T-1-DLA(D).
Figure 12 is a representation of an electrophoretogram of a Western blot of purified GST-fusion proteins with and without cleavage by thrombin.

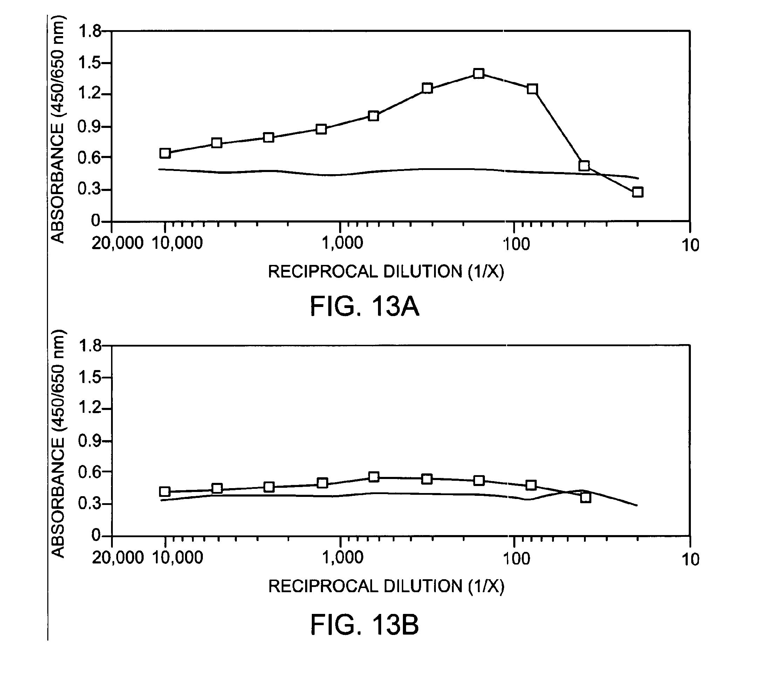

Figure 13A is a representation of a graph showing that a crude extract of an *E. coli* culture containing expressed D1-FRIL, a representative, non-limiting FRIL family member of the invention, specifically stimulates hFLT3 3T3 cells; Figure 13B is a graph showing that the same extract does not stimulate untransfected 3T3 cells.

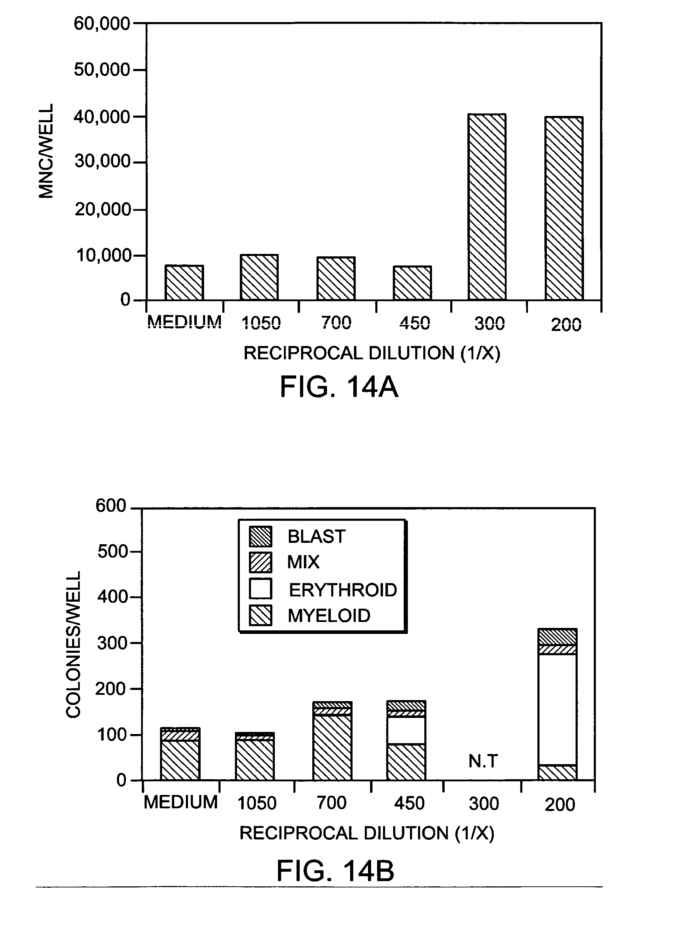

Figure 14A is a representation of a histogram showing that purified Dl-FRIL, a representative, non-limiting FRIL family member of the invention, preserves cord blood mononuclear cells in a dose-responsive manner.
Figure 14B is a representation of a histogram showing that purified Dl-FRIL, a representative, non-limiting FRIL family member of the invention, preserves hematopoietic progenitors in a dose-responsive manner.
Figure 15A is a representation of a photograph of colonies derived from human cord blood mononuclear cells cultured in 40 ng/mL Dl-FRIL, a representative, non-limiting FRIL family member of the invention, for 3 weeks, and then replated in methylcellulose colony assay medium.

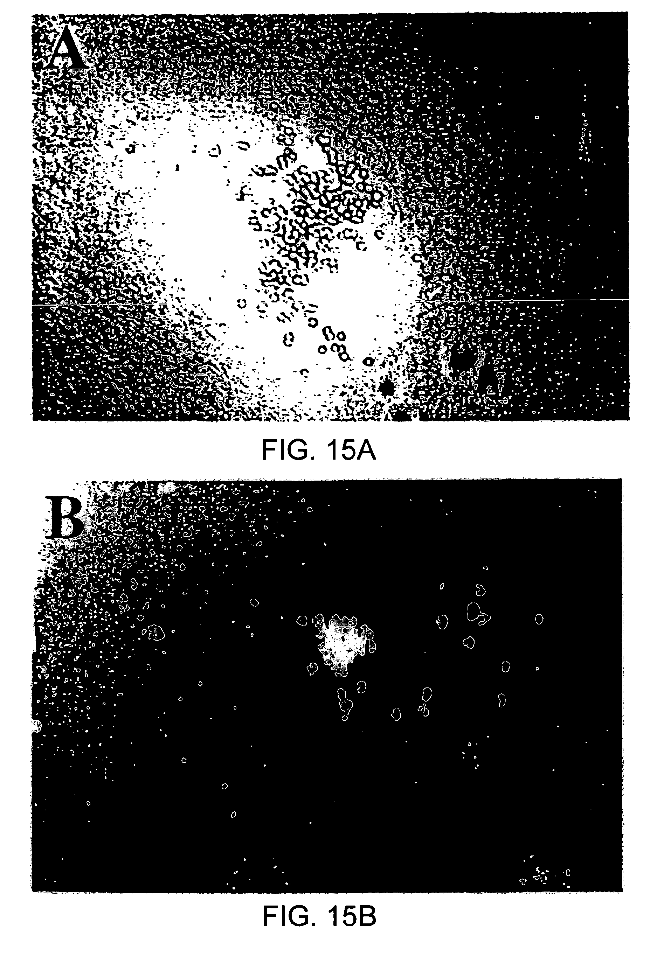

Figure 15B is a representation of a photograph of colonies derived from human cord blood mononuclear cells cultured in 40 ng/mL Dl-FRIL, a representative, non-limiting FRIL family member of the invention, for 4 weeks, and then replated into methylcellulose colony assay medium.

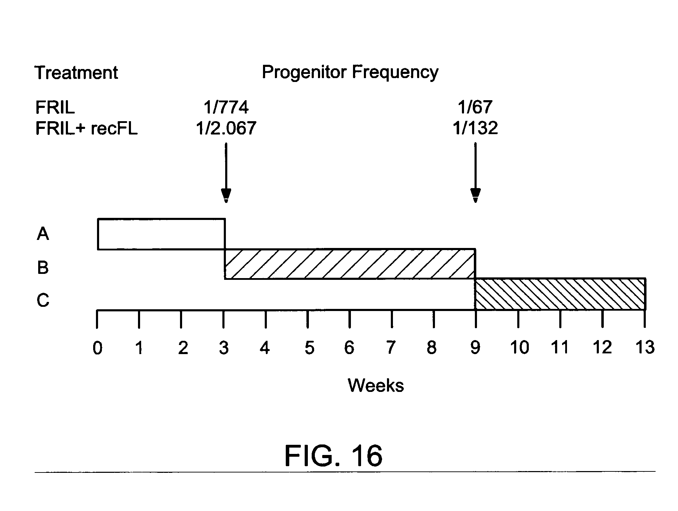

Figure 16 is a schematic diagram showing the serial replating of progenitor cells cultured in Dl-FRIL, a representative, non-limiting FRIL family member of the invention, or Dl-FRIL+recFL *(i.e.*, recombinant FLT3-Ligand). The human cord mononuclear cells were first cultured in supensiion in 40 ng/mL Dl-FRIL of 40 ng/mL Dl-FRIL + 40 ng/mL recFL (solid black box). The cells were then harvested and assessed for progenitor activity by being replated into methylcellulose colony assay medium for 6 weeks (middle striped box). Then the cells were harvested from the colony assay and again replated into methylcellulose colony assay medium for an additional 4 weeks (far right striped box). Progenitor frequencies were determined for cells after 3 weeks of suspension culture in D1-FRIL or Dl-FRIL+recFL, and after an additional 6 weeks of methylcellulose culture (absent Dl-FRIL and/or recFL).

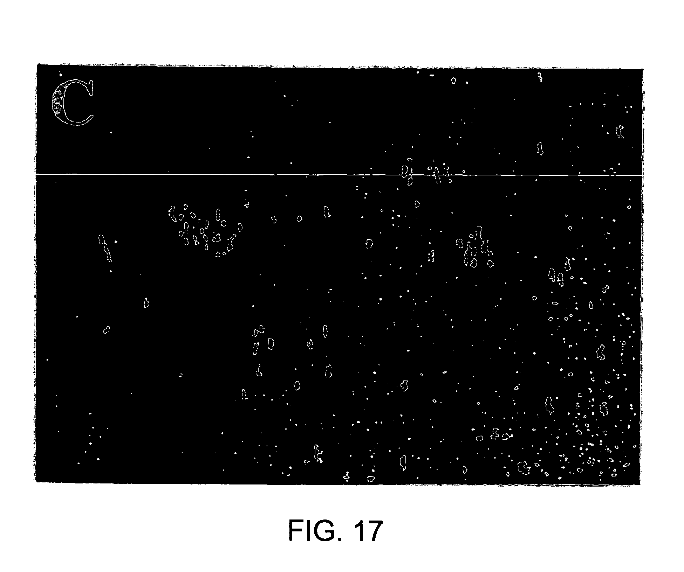

Figure 17 is a representation of a photograph of colonies derived from human cord blood mononuclear cells initially cultured in 40 ng/mL D1-FRIL, a representative, non-limiting FRIL family member of the invention, for 3 weeks, then replated into methylcellulose colony assay medium for 6 weeks, and then replated again into methylcellulose colony assay medium for an additional 4 weeks.

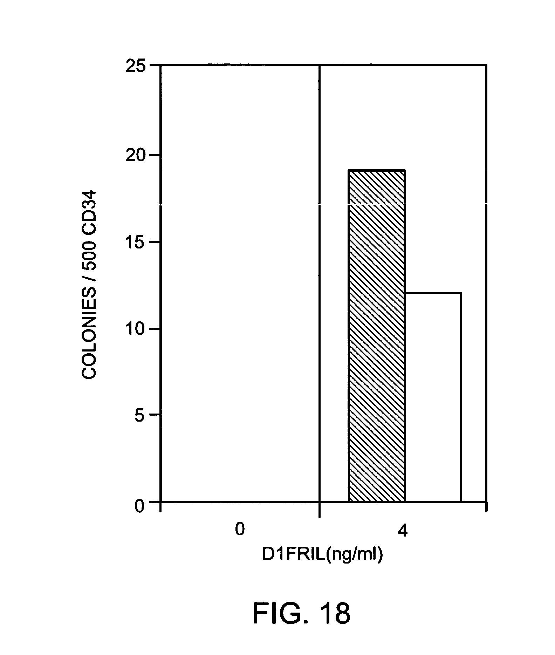

Figure 18 is a representation of a bar graph showing that a representative, non-limiting FRIL family member of the invention, Dl-FRIL, encoded by a representative, non-limiting nucleic acid of the invention is sufficient to preserve progenitor cells *ex vivo,* whereas a cytokine cocktail fails to preserve such cells.

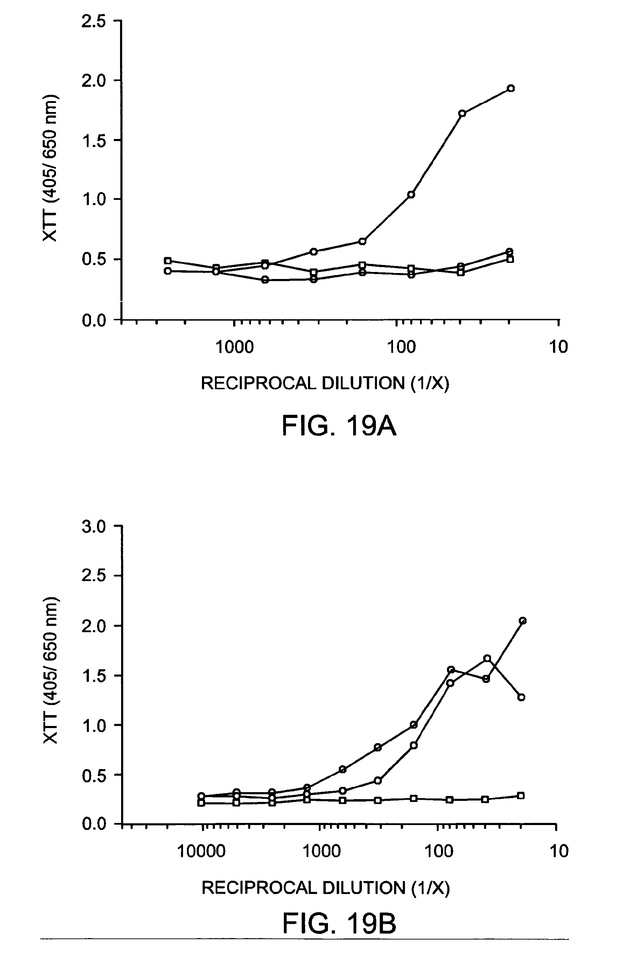

Figures 19A and 19B are representations of line graphs showing the biological specificity of receptor-transfected 3T3 cells. Figure 19A shows that rhM-CSF specifically stimulated Fms 3T3 (solid circles) but not either mFlt3/Fms 3T3 (open circles) or parent 3T3 cells (solid squares) in biological screening assay in a dose-dependent manner. Figure 19B shows that PHA-LCM (reciprocal dilution) stimulated mFlt3/Fms 3T3 (solid circles) and Stk 3T3 (open circles) but not parent untransfected 3T3 cells (solid squares).

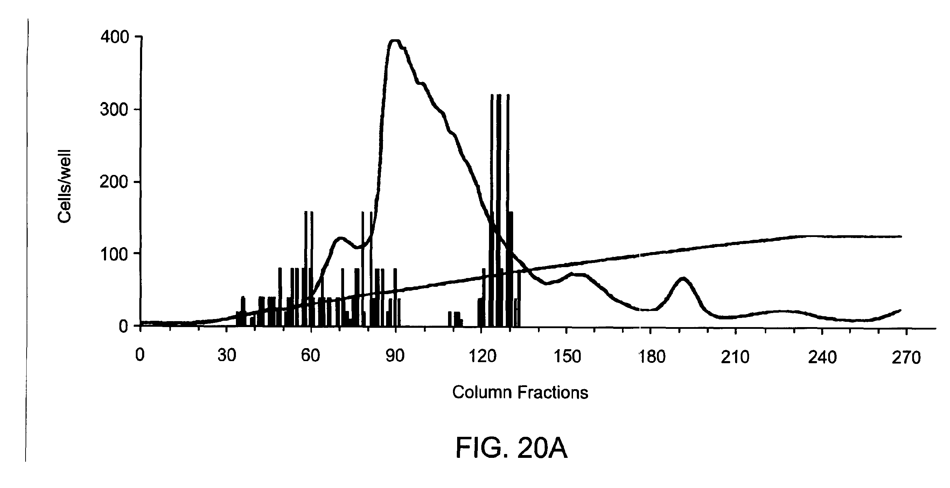

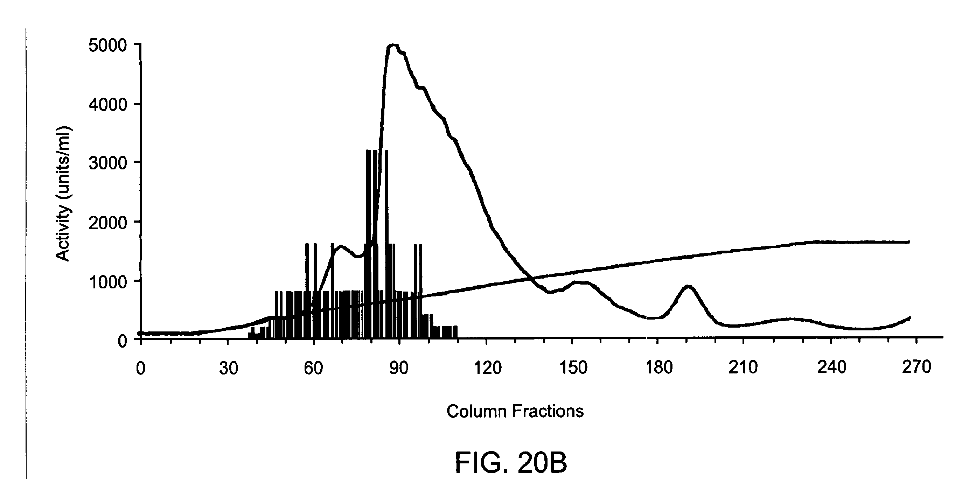

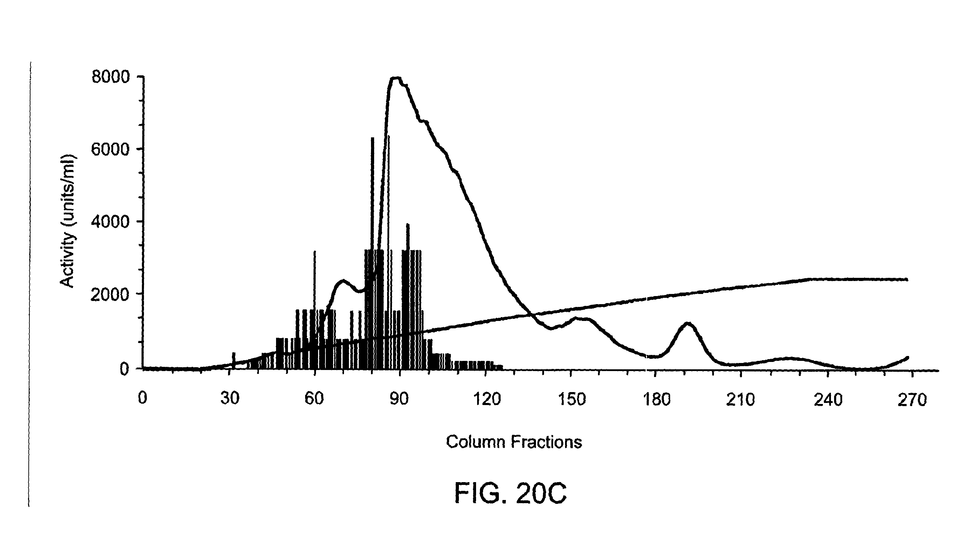

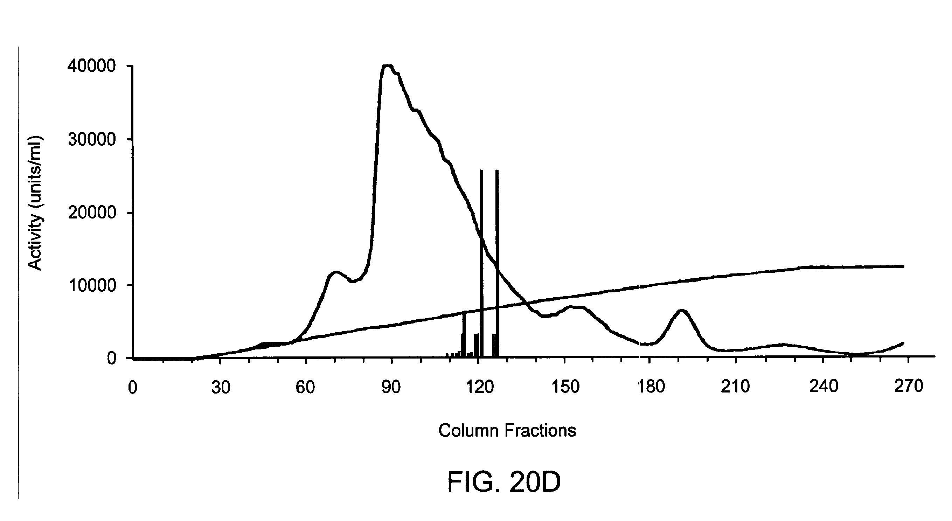

Figures 20A-20D are representations of the detected biological activities of PHA-LCM fractionated by anion exchange chromatography. Figure 20A shows the number of viable cells cord blood cells observed microscopically. Figure 20B shows the stimulation of mFlt3/Fms 3T3 cells by anion exchange column fractions. Figure 20C shows the stimulation of Stk 3T3 cells by anion exchange column fractions. Figure 20D shows the stimulation of Fms 3T3 cells by anion exchange column fractions.

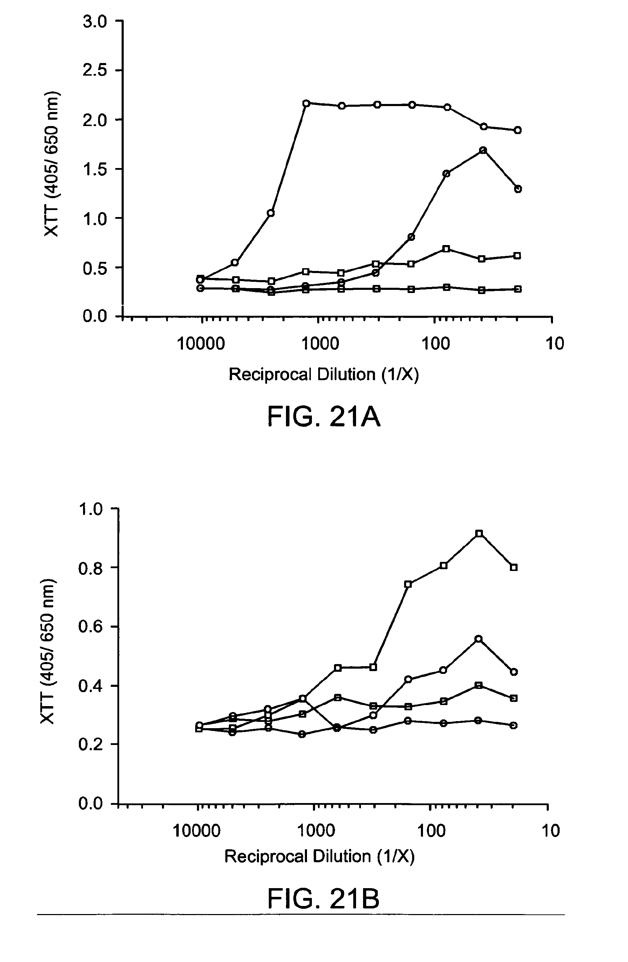

Figures 21A and 21B are representations of line graphs showing the co-factors required in the Flt3 3T3 assay during purification of Pv-FRIL, a representative, non-limiting FRIL family member of the invention. Figure 21A shows that the plateau stimulation of Flt3 3T3 cells decreased during purification from crude, 10-fold concentrated PHA-LCM (solid circles) to partially purified (open circles), and highly purified (solid squares). Medium control is shown is open squares. Figure 21B shows that the decreased plateau stimulation of Flt3 3T3 cells (solid circles) was restored by addition of sub-optimal concentrations (1:200) of crude PHA-LCM (solid squares). Corresponding medium controls are shown in open symbols.

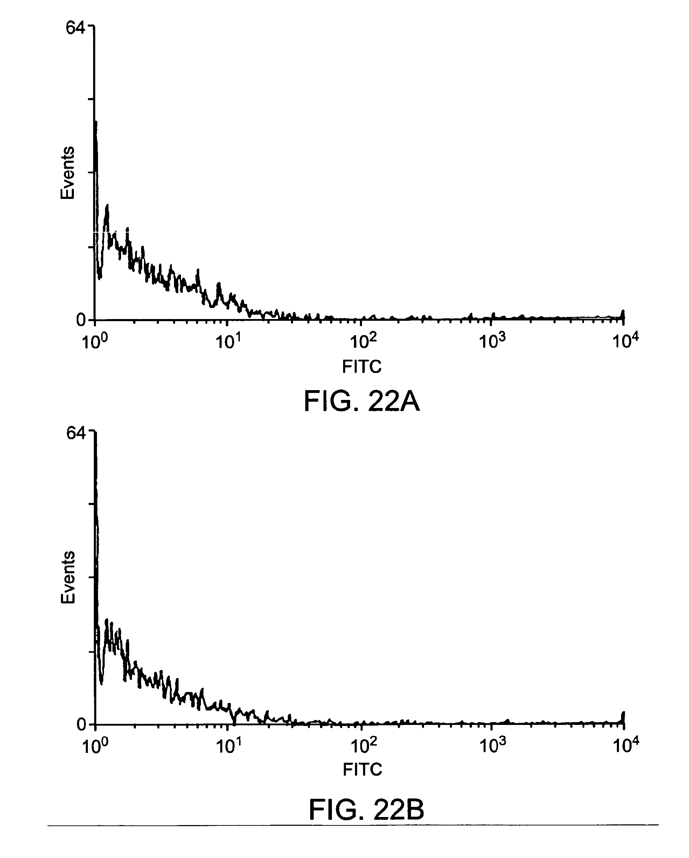

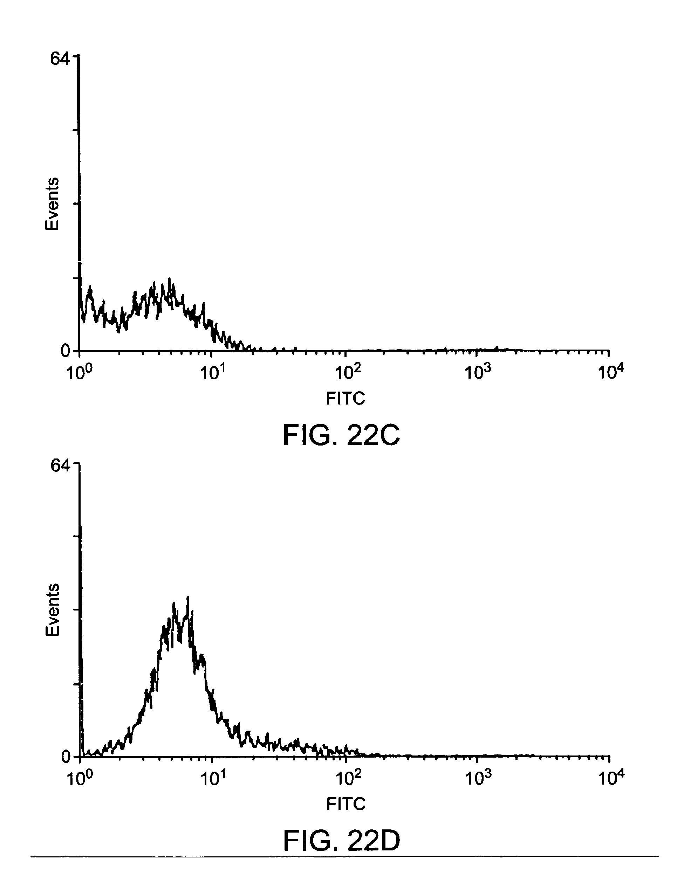

Figures 22A-22D are representations of a series of flow cytometry histograms showing the re-analysis of CD34 expression of cord blood CD34⁺ cells after two weeks in suspension culture with pooled AQS affinity column fractions. CD34 expression was re-analyzed by flow cytometry on pooled AQS affinity column fractions 1-5 (Figure 22A), fractions 6-10 (Figure 22B), fractions 11-15 (Figure 22C), and fractions 16-20 (Figure 22D). Fluorescence intensity on the abcissa and frequency of events on the ordinate in Figures 22A-22D.

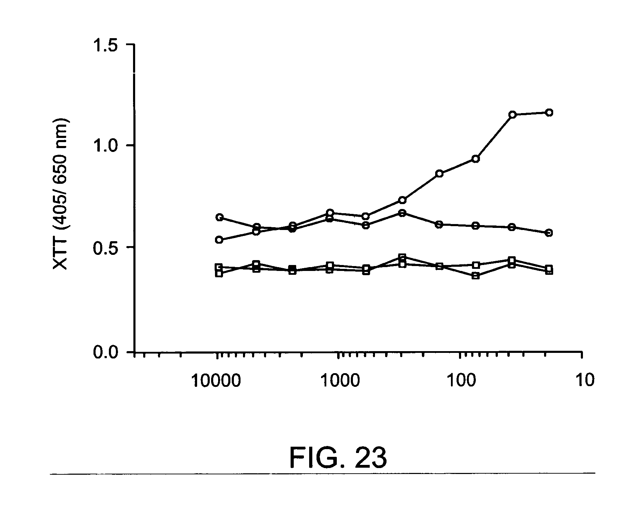

Figure 23 is a representation of a line graph showing the IL1-dependent response of mFlt3/Fms 3T3 cells to FRIL isolated from commercial red kidney bean extract. mFlt3/Fms 3T3 cells respond to FRIL in the presence of rhIL1 (solid circles) but not the absence of IL1 (solid squares). Corresponding medium only controls are shown with open symbols.

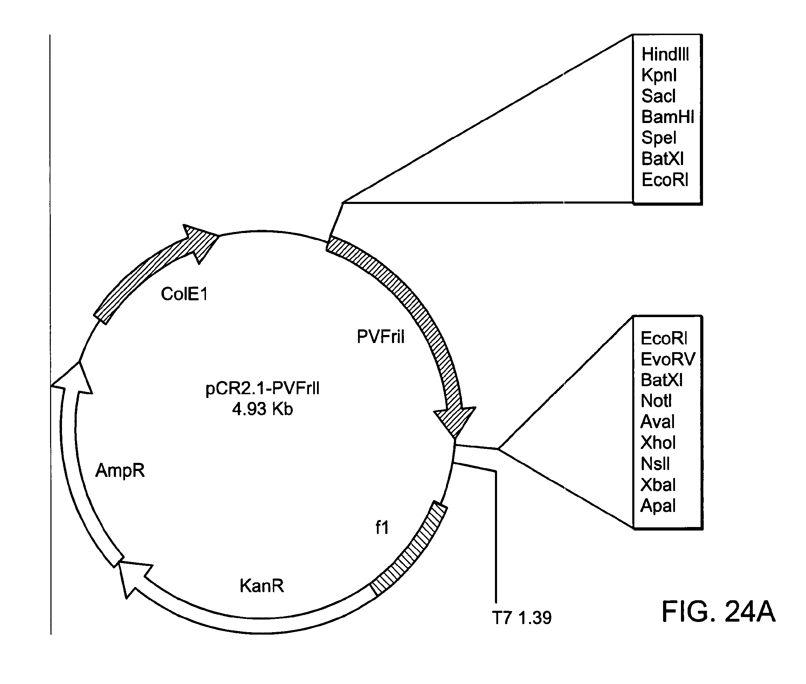

Figure 24A is a map of a cloning vector pCR2.1-Pv-FRIL manufactured by ligating a cDNA according to the invention in the EcoRI site of the cloning vector pCR2.1.

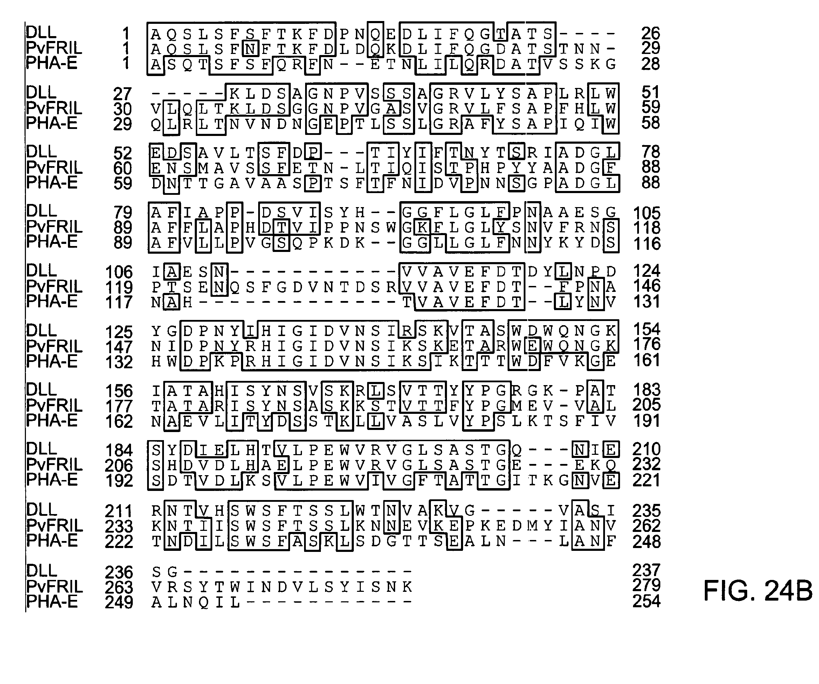

Figure 24B shows a direct amino acid sequence comparison of Pv-FRIL, a representative, non-limiting FRIL family member of the invention, with Dl-FRIL, another representative, non-limiting FRIL family member of the invention, and the PHA lectin, PHA-E.

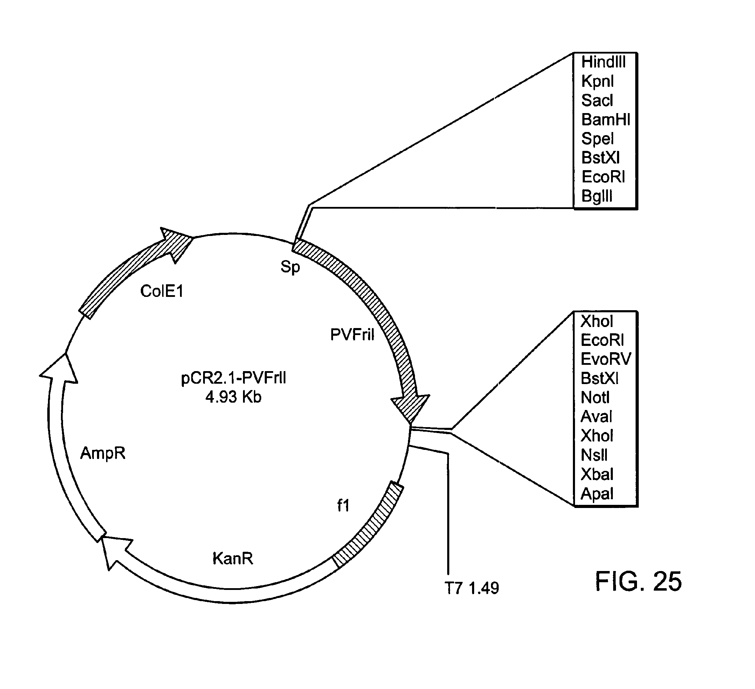

Figure 25 is a map of a cloning vector pCR2.1-SpPv-FRIL manufactured by ligating a cDNA according to the invention in the Xhol site of the cloning vector pCR2.1.

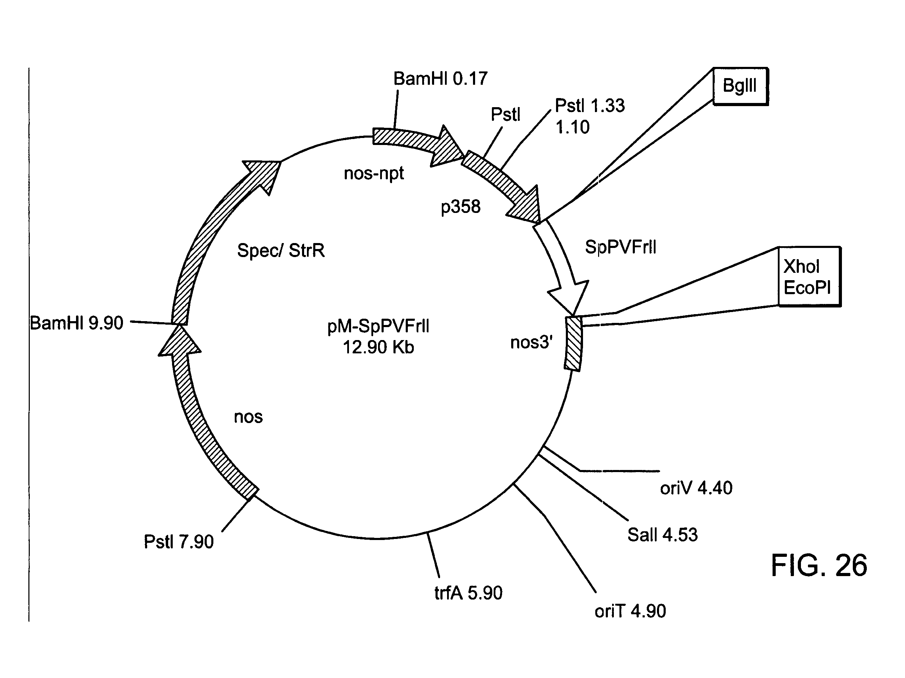

Figure 26 is a map of a cloning vector pM-SpPv-FRIL manufactured by ligating a cDNA according to the invention in the Bg1II/Xhol sites of the cloning vector SpPv-FRIL.

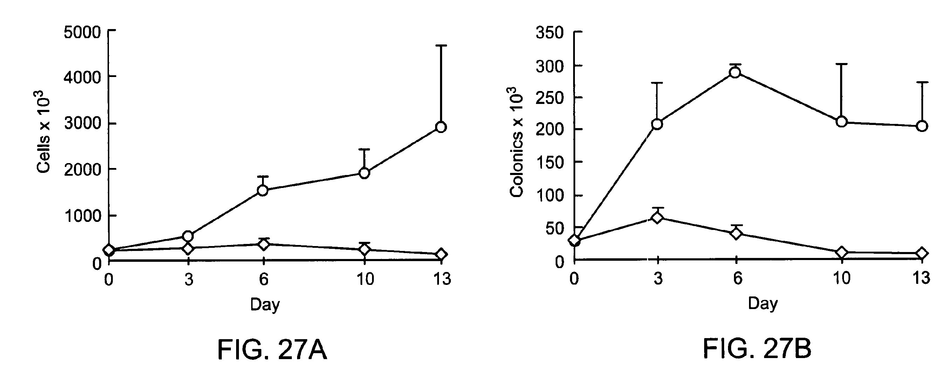

Figures 27A-27B are representations of line graphs showing the total cell numbers and progenitor levels in the presence of D1-FRIL,a representative, non-limiting FRIL family member of the invention, or cytokines. Enriched CB CD34⁺ cells were cultured for 3,6,10, or 13 days in the presence of D1-FRIL (solid symbols) or cytokines (open symbols). Colonies were scored on day 14 and progenitor levels were calculated based on total cell numbers. Values shown represent the mean±SEM of data from up to 10 experiments. Figure 27A shows the total cell numbers over time. Figure 27B shows the progenitor levels in cultures over time.

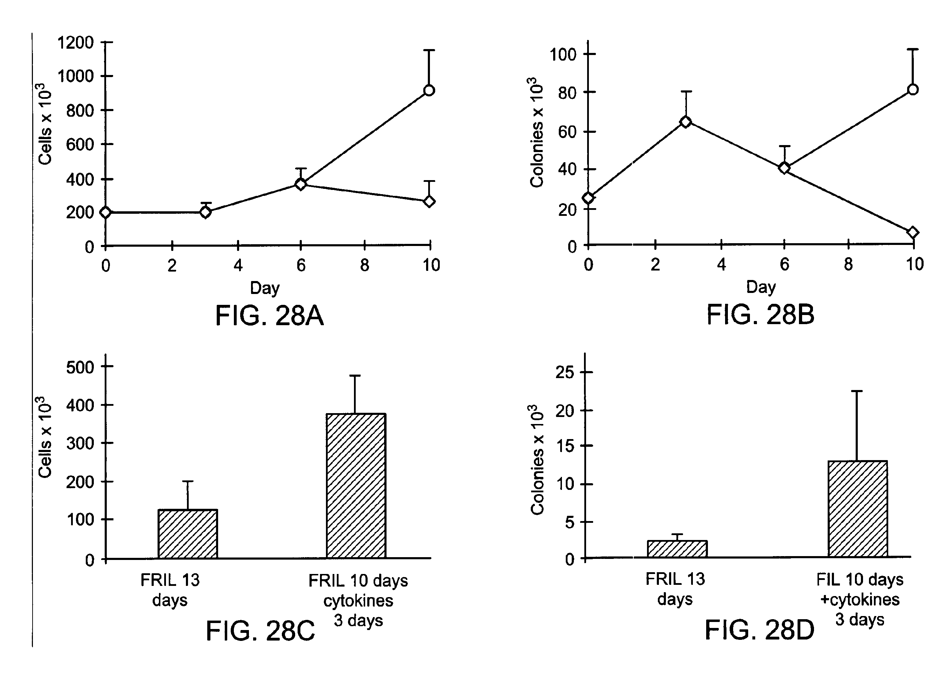

Figures 28A-28D are representations of line and bar graphs showing the total cell numbers and progenitor levels first in the presence of D1-FRIL, a representative, non-limiting FRIL family member of the invention, and second in presence of cytokines. Figure 28A shows the total numbers of cells cultured with D1-FRIL for entire 10 days (solid symbols) or for 6 days followed by 4 days of cytokine stimulation (open symbols). Figure 28B shows the progenitor levels in cells cultured with Dl-FRIL for entire 10 days (solid symbols) or for 6 days followed by 4 days of cytokine stimulation (open symbols). Figure 28C shows the total numbers of cells cultured with Dl-FRIL for 13 days (solid symbols) or for 10 days followed by 3 days of cytokine stimulation (open symbols). Figure 28D shows the progenitor levels in cells cultured with Dl-FRIL for 13 days (solid symbols) or for 10 days followed by 3 days of cytokine stimulation (open symbols).

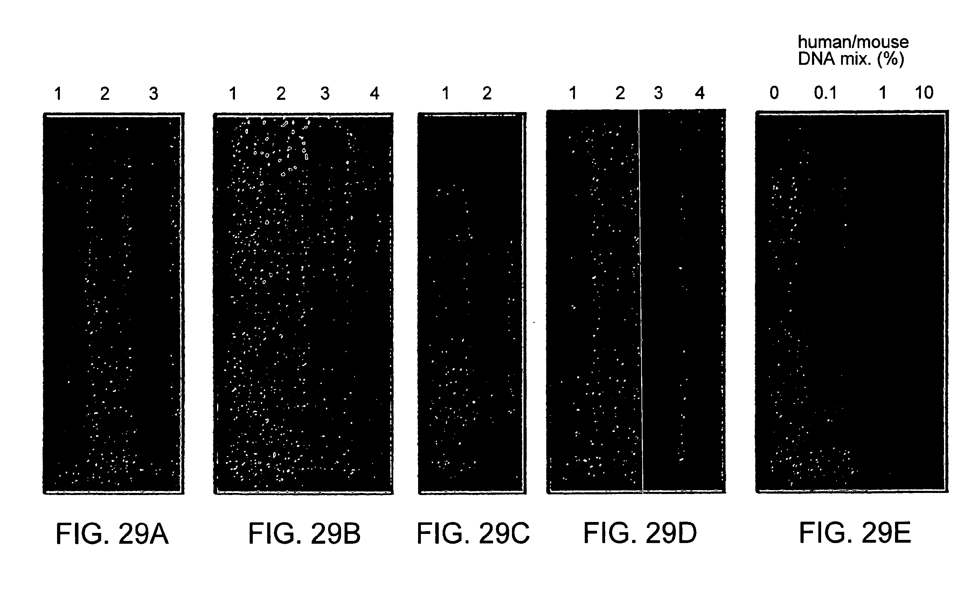

Figures 29A-29D are representations of representative Southern blot analyses showing the quantitative analysis of SRC after *ex vivo* cultures with D1-FRIL,a representative, non-limiting FRIL family member of the invention, or cytokines and after transplantation into mice. Figure 29A is a representation of a representative Southern blot showing human DNA in the marrow of mice transplanted with cells that were cultured with Dl-FRIL for 6 days (lane 1), D1-FRIL for 10 days (lane 2), or with Dl-FRIL for 6 days followed by 4 days with cytokine stimulation (lane 3). Figure 29B is a representation of a representative Southern blot showing human DNA in the marrow of mice transplanted with cells cultured with Dl-FRIL for 10 days (lanes 1-2), or with Dl-FRIL for 6 days followed by 4 days with cytokine stimulation (lanes 3-4). Figure 29C is a representation of a representative Southern blot showing human DNA in the marrow of mice transplanted with the original cells prior to seeding (lane 1), or with cells cultured with Dl-FRIL for 13 days (lane 2). Figure 29D is a representation of a representative Southern blot showing human DNA in the marrow of mice transplanted with cells cultured with FRIL for 10 days (lane 1), Dl-FRIL for 6 days followed by 4 days of cytokine stimulation (lane 2), Dl-FRIL for 13 days (lane 3), or with Dl-FRIL for 10 days followed by 3 days of cytokine stimulation (lane 4).
Figure 29E is a representation of a Southern blot analysis showing the detection of a 0%, 0.1%, 1%, and 10% human DNA per murine DNA.

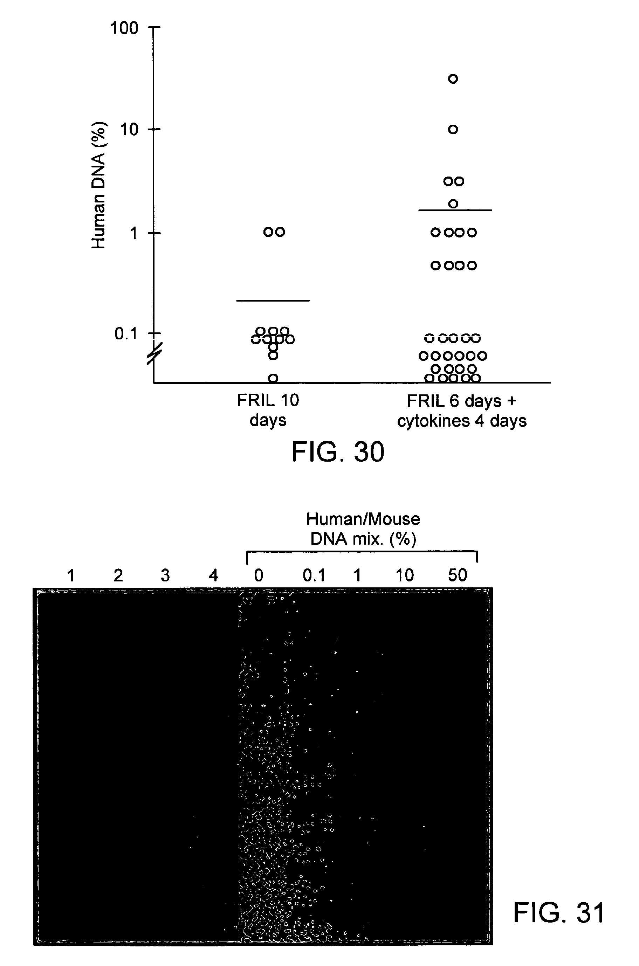

Figure 30 is a representation of a survival chart summarizing the levels of human cell engraftment in the marrow of mice transplanted with CD34⁺ cells cultured in either D1-FRIL, a representative, non-limiting FRIL family member of the invention, for ten days (left panel), or in the presence of D1-FRIL for 6 days followed by culture in the presence of cytokines for four days.
Figure 31 is a representation of a representative Southern blotting analysis showing the levels of levels of human cell engraftment in the BM of NOD/SCID B2M^{*null*} transplanted with CD34⁺CD38^{-/low} cells cultured in the presence of D1-FRIL, a representative, non-limiting FRIL family member of the invention. Sorted cells (2x10⁵ initial cells /treatment) were cultured in the presence of D1-FRIL for 6 days followed by additional 4 days exposure to cytokines, or with Dl-FRIL alone for 10 days. After 10 days, 3.6x10⁵ cells harvested from cytokine culture were divided and transplanted into 3 mice (lanes 1-3), while 3.5x10⁴ cells harvested from Dl-FRIL alone culture were transplanted to one mouse (lane 4). DNA was harvested from the bone marrow of transplanted mice and subjected to Southern blotting analysis with radiolabeled human chromosome 17-specific α-satellite probe (p17H8). A representative experiment out of 4 is shown.

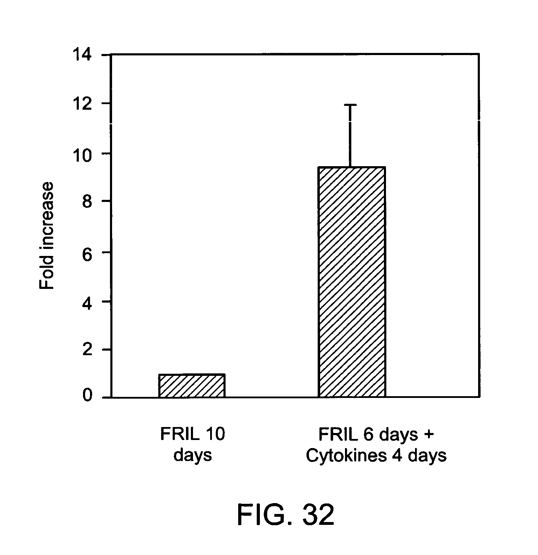

Figure 32 is a representation of a bar graph showing the average fold increase of engraftment levels obtained with CD34⁺CD38^{-/low} cells that were cultured with D1-FRIL, a representative, non-limiting FRIL family member of the invention, for 6 days and with cytokines for 4 days (right bar), as compared to 10 days with D1-FRIL alone (left bar). Data represent mean ± SE from 4 experiments.

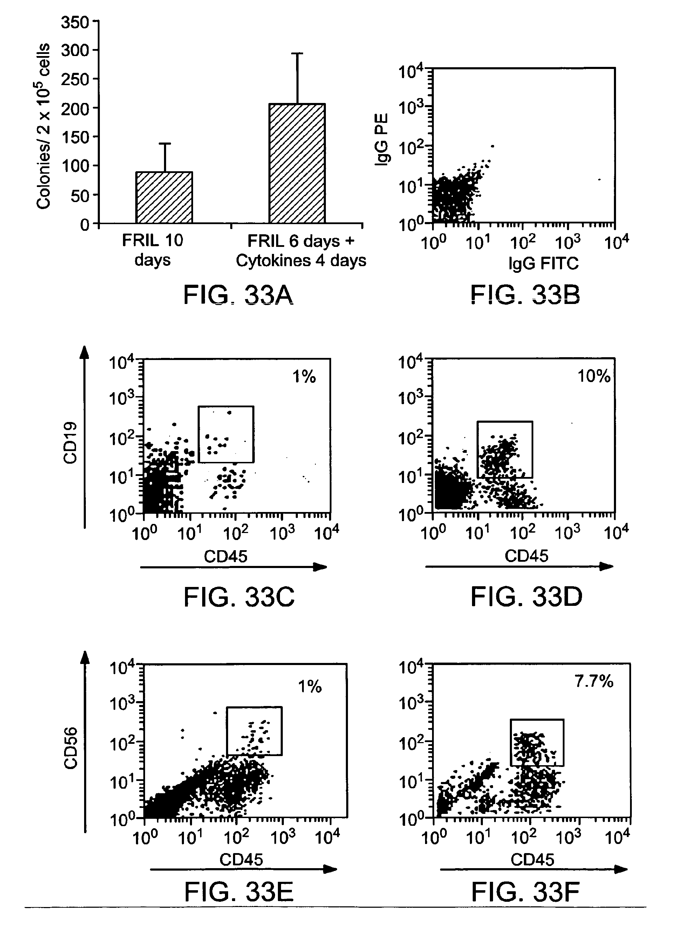

Figures 33A-33F are representations of a bar graph (Figure 33A) and representative flow cytometry histograms (Figure 33B-33F) showing the multilineage differentiation of SRC cultured with Dl-FRIL, a representative, non-limiting FRIL family member of the invention, in the BM of transplanted mice. Figure 33A shows the number of colonies per 2 x 10⁵ cells, where the cells treated as indicated prior to transplantation into mice were recovered from the murine BM, and seeded into semisolid media selective for human colonies. Progenitor levels were calculated based on the total human cell numbers (2 x 10⁵ cells) in the marrow of transplanted mice. Values shown represent the mean±SEM of data from 3 experiments, 9 mice/treatment. Figure 33B shows a representative flow cytometry analysis showing nonspecific labeling where mouse IgG was used as isotype control. Figures 33C and 33D show representative flow cytometry analyses of BM cells from mice that were transplanted with CD34⁺ cells (Figure 33C) or CD34⁺CD38^{-/low} cells (Figure 33D) precultured with Dl-FRIL for 10 days, where the harvested BM cells were stained with anti human CD45 and anti-human CD19. Figures 33E and 33F show representative flow cytometry analyses of BM cells from mice that were engrafted by CD34⁺ cells (Figure 33E) or CD34⁺CD38^{-/low} cells (Figure 33F) precultured with Dl-FRIL for 10 days, where the harvested BM cells were subsequently cultured with SCF + IL-15 for 10 days, and then stained with human specific monoclonal anti-CD45 and anti-CD56.

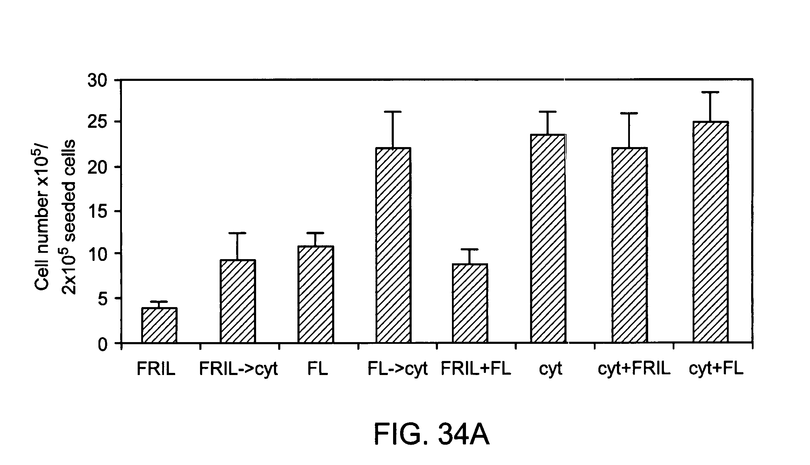

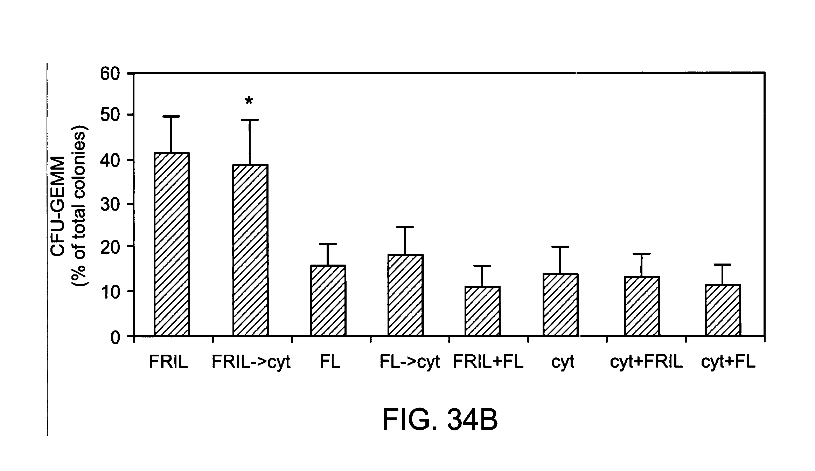

Figures 34A-34B are representations of bar graphs showing the growth effect of Dl-FRIL, a representative, non-limiting FRIL family member of the invention, on CD34⁺ cells and progenitors compared to Flt3 ligand and cytokine combinations. (+) indicates co-culture of factors for the entire 10 days while (->) indicates substitution of the first factor after 6 days with cytokines, as indicated under the x axis. Figure 34A shows the total cell numbers. Figure 34B shows the percentage of CFU-GEMM out of total colonies. Values shown are per 2x10⁵ seeded cells, and represent the mean ± SEM of data from 5 experiments.

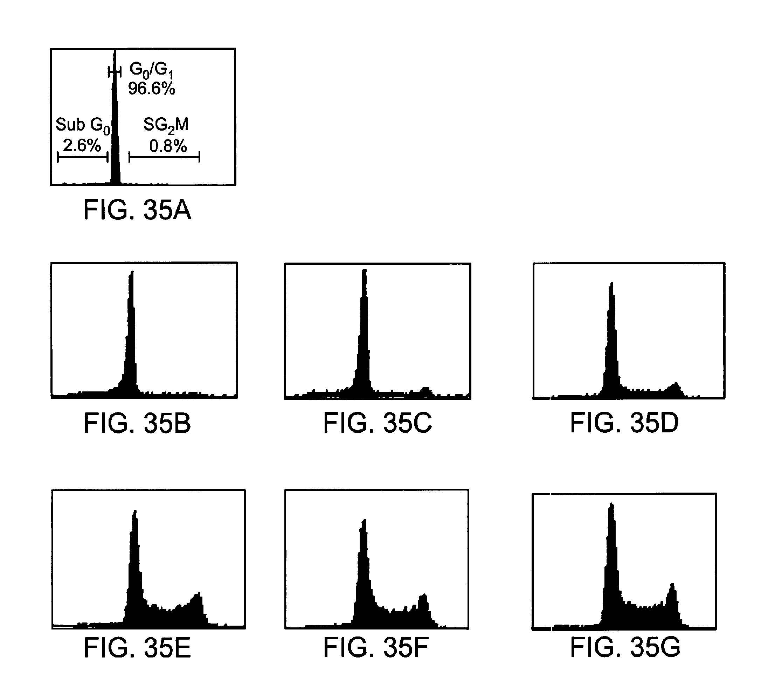

Figures 35A-35G are representatives of flow cytometry histograms showing the cell cycle analyses of CD34⁺ cells cultured with Dl-FRIL, a representative, non-limiting FRIL family member of the invention, or various cytokines, or with combinations thereof. DNA content was determined by flow cytometry with propidium iodide staining. Enriched CD34⁺ cells were cultured for 3 days with no treatment (Fig. 35A), D1-FRIL (Fig. 35B), Flt3-L (Fig. 35C), D1-FRIL+Flt3-L (Fig. 35D), SCF+G-CSF+IL-3+IL-6 (SG36) (Fig. 35E), SG36+D1-FRIL (Fig. 35F), and SG36+Flt3-L (Fig. 35G).

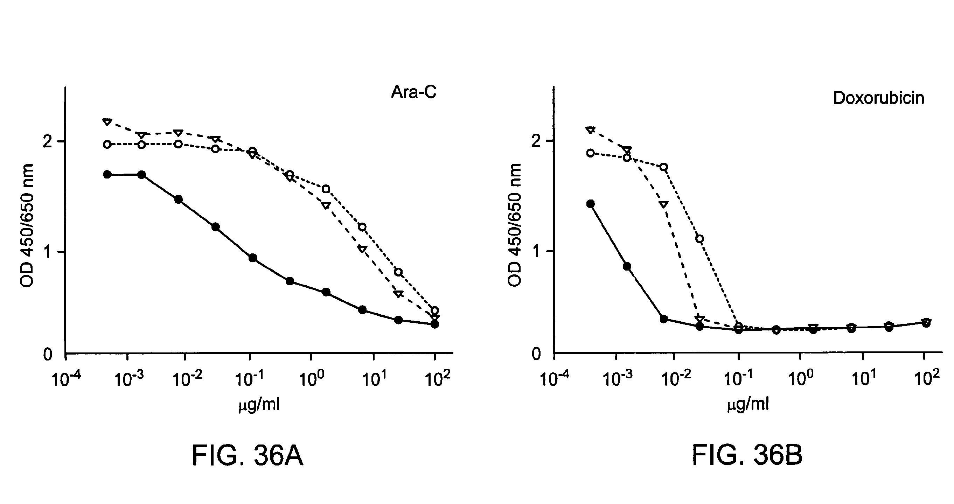

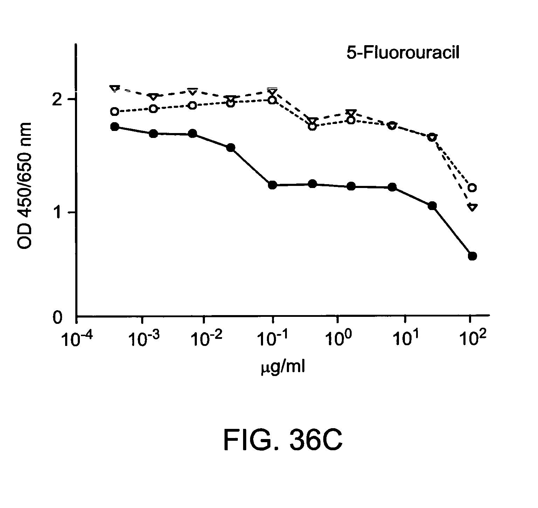

Figures 36A-36C are representations of line graphs showing the dose response of CB mnc chemotherapeutic agents in the presence and absence of Dl-FRIL, a representative, non-limiting FRIL family member of the invention. Chemotherapy agents were assayed over a 5-log dose range on CB MNC (2 x 10⁵ cells /0.1 mL) in AIMV (Life Technologies) containing Agar-SCM (StemCell Technologies). Viable cells were determined after 5 days of culture by XTT. Solid squares indicate chemotherapy drug with no D1-FRIL; solid triangles indicate cultures containing Dl-FRIL at 10 ng/ml in all wells; and open circles indicate Dl-FRIL in all wells at 100 ng/ml. Figure 36A shows the dose response to Ara-C; Figure 36B shows the dose response to doxorubicin, and Figure 36C shows the dose response to 5-FU.

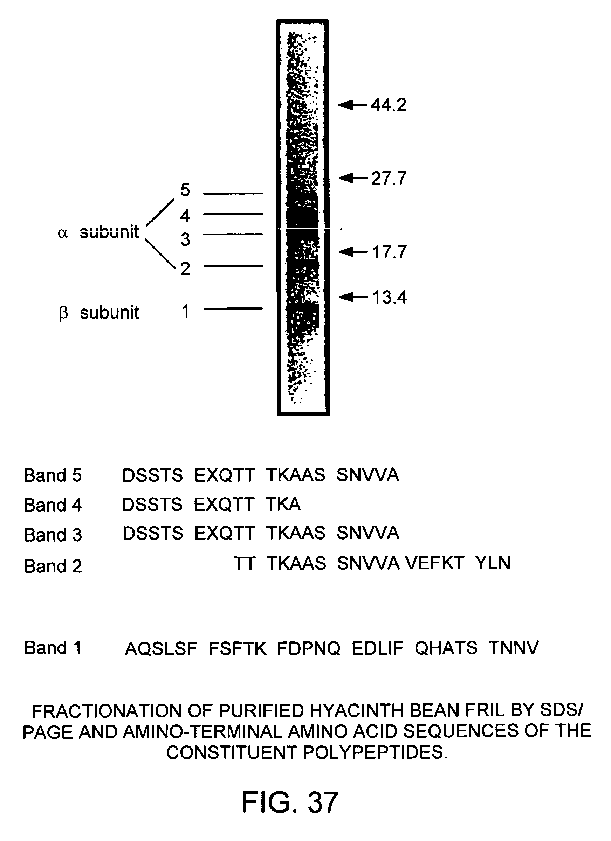

Figure 37 is a representation of a photograph of purified D1-FRIL, a representative, non-limiting FRIL family member of the invention, resolved by SDS-PAGE analysis. Five discrete bands appeared which corresponded to the a and β subunits, each of which was subjected to amino-terminal sequencing. The amino acid sequences of the five bands are as indicated.

### DETAILED DESCRIPTION OF THE PREFERRED EMBODIMENTS

The invention relates the preservation of progenitor cells by members of the FRIL family of progenitor cell preservation factors. More specifically, the invention relates to the *in vivo* or *ex vivo* preservation of progenitor cells, such as hematopoietic progenitor cells, using members of the FRIL family of progenitor cell preservation factors.

The invention provides a family of factors that preserve progenitor cells. Members of this family, the FRIL family, are non-toxic, inexpensively produced reagents that preserve progenitor cells. The invention provides compositions comprising at least one member of the FRIL family, as well as methods for using members of the FRIL family to preserve progenitor cells both *in vivo* and *ex vivo*.

All of the patents and publications cited herein reflect the knowledge in the art and are hereby incorporated by reference in entirety to the same extent as if each were specifically stated to be incorporated by reference. Any inconsistency between these patents and publications and the present disclosure shall be resolved in favor of the present disclosure.

In a first aspect, the invention provides an essentially pure composition of a member of the FRIL family of progenitor cell preservation factors. The term, "FRIL family of progenitor cell preservation factors" is used to mean a family of lectins, wherein each FRIL family member molecule binds to a normally glycosylated FLT3 receptor, wherein each FRIL family member molecule preserves progenitor cells, and wherein one FRIL family member molecule that is isolated from a hyacinth bean *(i.e., Dolichos lab lab)* has an amino acid sequence which comprises the following eight amino acid sequence: TNNVLQXT (SEQ ID NO: 24). By "FRIL family member" or FRIL family member molecule" is meant one or more molecules of the FRIL family of progenitor cell preservation factors.

In accordance with the first aspect of the invention, a composition of a FRIL family member, which includes a mutant of another FRIL family member molecule or a fusion protein comprising a portion derived from a FRIL family member molecule or mutant thereof, wherein each FRIL family member molecule binds to a normally glycosylated FLT3 receptor has at least about 45% amino acid sequence identity with the amino acid sequence of another member of the FRIL family, preferably at least about 50% identity, even more preferably at least about 55% identity, still more preferably at least about 60% identity, and still more preferably at least about 65% identity with the sequence of the second protein. In the case of proteins having high sequence identity, the amino acid sequence of the first protein shares at least about 75% sequence identity, preferably at least about 85% identity, and more preferably at least about 95% identity, with the amino acid sequence of another member of the FRIL family.

Both amino acid sequence identity and nucleic acid sequence identity between two proteins or two nucleic acid molecules can be measured according to standard methods. For example, in order to compare a first amino acid sequence to a second amino acid sequence or a first nucleic acid sequence to a second nucleic acid sequence for the purpose of determining percentage identity between the two sequences, the sequences are aligned so as to maximize the number of identical amino acid or nucleic acid residues. The sequences of proteins sharing at least 50% amino acid sequence identity or the sequences of nucleic acids sharing at least 45% nucleic acid sequence identity can usually be aligned by visual inspection. If visual inspection is insufficient, the proteins or nucleic acids may be aligned in accordance with the FASTA method in accordance with Pearson and Lipman *(Proc. Natl. Acad. Sci. USA* 85:2444-2448, 1988), or, preferably, any of the methods described by George, D.G. et al., in *Macromolecular Sequencing and Synthesis, Selected Methods and Applications,* pages 127-149, Alan R Liss, Inc. (1988), such as formula 4 at page 137 using a match score of 1, a mismatch score of 0, and a gap penalty of -1. From this method, percentage of sequence identity between the first and second amino acid sequences or between the first and second nucleic acid can be determined.

Other methods for determining amino acid or nucleic acid sequence identity are described in Feng and Doolittle (*Journal of Molecular Evolution* 25: 351-360,1987) and Higgins and Sharp (CABIOS 5:151-153,1989).

Another method for determining amino acid or nucleic acid sequence identity between two proteins or nucleic acids is by using sequence analysis software with the default parameters specified therein. Various software packages exist including Sequence Analysis Software Package of the Genetics Computer Group (University of Wisconsin Biotechnology Center, Madison, WI), and the various BLAST programs of the National Center for Biotechnology (National Library of Medicine, Bethesda, MD).

Unless otherwise specified, percentage of amino acid sequence identity or percentage of nucleic acid sequence identity is determined using the basic BLAST program of the National Center for Biotechnology (National Library of Medicine, Bethesda, MD), using the default settings defined therein.

Another test for percentage identity of two nucleic acid sequences is whether they hybridize under normal hybridization conditions, preferably under stringent hybridization conditions. Thus, also included in the invention are proteins that are encoded by nucleic acid molecules that hybridize under high stringent conditions to a sequence complementary to SEQ ID NO: 1, SEQ ID NO: 3, SEQ ID NO: 5, and/or SEQ ID NO: 7. The term "stringent conditions," as used herein, is equivalent to "high stringent conditions" and "high stringency." These terms are used interchangeably in the art.

Stringent conditions are defined in a number of ways. In one definition, stringent conditions are selected to be about 50°C lower than the thermal melting point (Tₘ) for a specific sequence at a defined ionic strength and pH. The Tₘ is the temperature (under defined ionic strength and pH) at which 50% of the target sequence hybridizes to a perfectly matched sequence. Typical stringent conditions are those in which the salt concentration is at least about 0.02 M at pH 7 and the temperature is at least about 60°C. "Stringent conditions," in referring to percentage identity (*e.g.*, homology) or substantial similarity in the hybridization context, can be combined conditions of salt, temperature, organic solvents or other parameters that are typically known to control hybridization reactions. The combination of parameters is more important than the measure of any single parameter. If incompletely complementary sequences recognize each other under high stringency conditions, then these sequences hybridize under conditions of high stringency (see U.S. Pat No. 5,786,210; Wetmur and Davidson, *J. Mol. Biol*. 31, 349-370,1968). Control of hybridization conditions, and the relationships between hybridization conditions and degree of homology are understood by those skilled in the art. See, *e.g.,* Sambrook et al., *Molecular Cloning. A Laboratory Manual,* 2ed., Cold Spring Harbor Laboratory, Cold Spring Harbor, 1989. Further examples of stringent conditions can be found in Goeddel et al., U.S. Patent No. 5,789,550.

In a non-limiting example, "stringent conditions" can be provided in a variety of ways such as overnight incubation at 42°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 µg/ml denatured, sheared salmon sperm DNA. Alternatively, the stringent conditions are characterized by a hybridization buffer comprising 30% formamide in 5 x SSPE (0.18 M NaCl, 0.01 M NaPO₄, pH 7.7, 0.0001 M EDTA) buffer at a temperature of 42°C, and subsequent washing at 42°C with 0.2 x SSPE. Preferably, stringent conditions involve the use of a hybridization buffer comprising 50% formamide in 5 x SSPE at a temperature of 42°C and washing at the same temperature with 0.2 x SSPE.

As used herein, by "preserves progenitor cells" is meant an ability of a FRIL family member molecule (or mutant thereof or fusion protein comprising a FRIL family member molecule or mutant thereof) to retain (*i*.*e*., preserve) progenitor cells in an undifferentiated state, which can be determined using the assays described below *(e.g.,* the SCID mouse reconstituting cell assay and the methylcellulose or other semi-solid medium based hematopoietic progenitor cell assay). In accordance with the invention, "progenitor cell" refers to any normal somatic cell that has the capacity to generate fully differentiated, functional progeny by differentiation and proliferation. Progenitor cells include progenitors from any tissue or organ system, including, but not limited to, blood, mesenchymal, embryonic, nerve, muscle, skin, gut, bone, kidney, liver, pancreas, thymus, brain and the like. Progenitor cells are distinguished from "differentiated cells," the latter being defined as those cells that may or may not have the capacity to proliferate, *i.e.*, self-replicate, but that are unable to undergo further differentiation to a different cell type under normal physiological conditions. Moreover, progenitor cells are further distinguished from abnormal cells such as neoplastic cells, as defined herein. For example, leukemia cells proliferate (self-replicate), but generally do not further differentiate, despite appearing to be immature or undifferentiated.

Progenitor cells include all the cells in a lineage of differentiation and proliferation prior to the most differentiated or the fully mature cell. Thus, for example, progenitors include the skin progenitor in the mature individual. The skin progenitor is capable of differentiation to only one type of cell, but is itself not fully mature or fully differentiated.

By "hematopoiesis" is meant the development of mature, functional blood cells. The progenitor cells that give rise to mature, functional blood cells are called hematopoietic progenitor cells. The most primitive, undifferentiated hematopoietic progenitor cell is called a hematopoietic stem cell. Hematopoietic stem cells typically reside in the bone marrow primarily in a quiescent state, and may form identical daughter cells through a process called "self-renewal."

Production of some mature, functional blood cells results from proliferation and differentiation of "unipotential progenitors," *i.e.,* those progenitors that have the capacity to make only one type of blood cell. For red blood cell (erythrocyte) production, a unipotential progenitor called a "CFU-E" (colony forming unit-erythroid) has the capacity to generate two to 32 mature progeny cells. Various other hematopoietic progenitors have been characterized. For example, hematopoietic progenitor cells include those cells that are capable of successive cycles of differentiating and proliferating to yield up to eight different mature hematopoietic cell lineages.

Uncommitted progenitor cells, such as hematopoietic stem cells, can be described as being "totipotent," *i.e.,* both necessary and sufficient for generating all types of mature cells. Progenitor cells that retain a capacity to generate all cell lineages, but that can not self-renew, are termed "pluripotent." Cells that can produce some but not all blood lineages and can not self-renew are termed "multipotent."

Progenitor cells can be defined by mRNA levels of genes that either specifically regulate progenitors or serve as markers of lineage commitment. For example, genes induced in primitive human hematopoietic progenitor cells include those encoding the shared beta subunits of the IL3, IL5, and/or granulocyte-macrophage colony-stimulating factor (GM-CSF) receptors, termed the beta common chain (McClanahan et al., *Blood* 81:3903-2915,1993); CD34 genes; and/or the receptors for Kit (Turner et al., *Blood* 88:3383-3390, 1996), FLT1, FLT4 (Galland et al., *Oncogene* 8:3233-1240, 1993), FLK1 (Broxmeyer et al., *Int. J. Hematol.* 62:303-215, 1995), and FLT3 (Lyman and Jacobsen, *Blood* 91:3101-1134.,1998). Those genes for intermediate progenitors include the c-Fms, G-CSF receptor, and/or CD34 genes; and the IL-7 receptor gene, a gene induced for B lymphopoiesis.

Murine primitive progenitor populations include receptors for interleukin-1 alpha (IL-1α), IL-3, IL-6, granulocyte colony-stimulating factor (G- CSF), and/or FLK1-1 (the murine homologue of human KDR which binds VEGF) (Broxmeyer, *supra*), but lack receptors for macrophage colony-stimulating factor (M-CSF), granulocyte-macrophage colony stimulating factor (GM-CSF), and leukemia inhibitory factor (LIF). Cells within the intermediate progenitor cell population include receptors for GM-CSF, G-CSF, IL-6, and/or IL-1α.

In accordance with the first aspect of the invention, the terms "bind," "binds," or "bound" are used interchangeably to mean that a FRIL family member molecule of the invention binds to a normally glycosylated FLT3 receptor with an affinity higher than the affinity with which the FLT3-Ligand binds the FLT3 receptor. Preferably, a FRIL family member molecule binds to a normally glycosylated FLT3 receptor with an affinity that is at least as high as the affinity with which an antibody binds its specific ligand. Even more preferably, a FRIL family member molecule of the invention binds to a normally glycosylated FLT3 receptor with an affinity that is higher than the affinity with which an antibody binds its specific ligand. Still more preferably, a FRIL family member molecule of the invention binds to a normally glycosylated FLT3 receptor with a dissociation constant (K_{D}) of at least 10⁻⁷M, more preferably 10⁻⁸ M, even more preferably 10⁻⁹ M, still more preferably, at least 10⁻¹⁰ M, and most preferably, a FRIL family member molecule of the invention binds to a normally glycosylated FLT3 receptor with a dissociation constant (K_{D}) of at least 10⁻¹¹ M. Standard methods for determining binding and binding affinity are known.

In accordance with the invention, by "normally glycosylated FLT3 receptor" is meant an FLT3 receptor that has a glycosylation pattern of an FLT3 cell glycosylated by a normal cell. By "normal cell," as used herein in accordance with all aspects of the present invention, is meant a cell that is not neoplastic. As used herein, by "neoplastic cell" is meant a cell that shows aberrant proliferation, particularly increased proliferation, that is not regulated by such factors as cell-cell contact inhibition and soluble regulators (*e.g.*, cytokines or hormones), and that abnormally glycosylates the FLT3 receptor such that the glycosylation pattern on the FLT3 receptor on the neoplastic cells is abnormal and such that the FLT3 receptor on the neoplastic cell is not bound by a FRIL family member molecule.

In accordance with the first aspect of the invention, by "essentially pure" means a molecule, such as a nucleic acid or protein (*e*.*g*., a FRIL family member molecule), or composition of a molecule that is more free from other organic molecules (*e.g.*, carbohydrates, nucleic acids, proteins, and lipids) that naturally occur with an impure molecule, and is substantially free as well of materials used during the purification process. For example, a protein or nucleic acid molecule is considered to be essentially pure if it is at least approximately 60%, preferably at least approximately 75%, more preferably approximately at least 85%, most preferably approximately at least 90%, and optimally approximately at least 95% pure, *i*.*e*., free from other organic molecules with which it naturally occurs and free from materials used during the purification process. Methods for purifying proteins are known in the art and include, without limitation, HPLC, SDS-PAGE, immunoprecipitation, recombinant protein production, affinity chromatography using specific antibodies, ion-exchange, size-exclusion, and hydrophobic interaction chromatography, or a combination of any of these methods. These and other suitable methods are described, *e*.*g*., in Marston, "The purification of eukaryotic proteins expressed in *E*. *coli,"* in *DNA Cloning,* Glover D.M., ed., Volume III, IRL Press Ltd., Oxford, 1987; Marston and Hartley, "Solubilization of protein aggregates," pp. 266-267 in *Guide to Protein Purification,* Deutscher MP, ed., Academic Press, San Diego, 1990; Laemmli, U.K., *Nature* 227:680-685,1970. A FRIL family member can also be purified by binding to a mannose, which may be coupled on a sold support (*e.g.*, a sepharose bead).

Methods for purifying nucleic acids are known in the art and include, without limitation, Guanidine-HCl extraction, polymerase chain reaction, CsCl gradient fractionation, phenol: chloroform extraction, ethanol precipitation, and standard recombinant DNA methodologies. Standard methods for purifying both proteins and nudeic acid molecules are provided in, *e*.*g*., Ausubel et al., *Current Protocols in Molecular Biology,* John Wiley & Sons, New York, NY,1994; Sambrook et al., *supra.*

In accordance with the first aspect of the invention, a FRIL family member molecule may be purified from a natural source by methods well known in the art. For example, the purification of D1-FRIL from *Dolichos lab lab* is described below in Example 1. The purification of Pv-FRIL from *Phaseolus vulgaris* as described below in Example 5. The purification of YamFRIL from *Sphenostylis stenocarpa* is described below in Example 22.. Such methods also include, for example, those described by Moore in PCT application PCT/US97/22486 and by Gowda et al., *supra.* A suitable natural source from which to purify a FRIL family member molecule includes plants, especially legume plants. Legumes, such as the garden pea or the common bean, are plants ("leguminous plants") from a family (*Leguminosae*) of dicotyledonous herbs, shrubs, and trees bearing (nitrogen-fixing bacteria) nodules on their roots. These plants are commonly associated with their seeds (e.g., the garden pea or the common bean)

More specifically, a FRIL family member molecule according to the first aspect of the invention can be purified from members of the tribe *Phaseoleae.* For example, a FRIL family member molecule can be purified from *Dolichos lab lab (e.g.,* hyacinth beans, which is also known by other common names throughout the world). Alternatively, a FRIL family member molecule can be purified from varieties of the common bean *(Phaseolus vulgaris) (e.g.,* red kidney beans and white kidney beans), from yam bean *(Sphenostylis stenocarpa*) or from *Vigna sinensis,* commonly known as the black-eyed pea.

As demonstrated in the examples below, purification of a FRIL family member molecule from a legume is rapid and inexpensive, and results in a large amount of essentially pure lectin. A native FRIL family member molecule can be easily purified from legumes, such as hyacinth beans (pesticide-free), by mannose-affinity chromatography or ovalbumin affinity chromatography, and is more than 100 times cheaper to produce than recombinant cytokines. In accordance with the first aspect of the invention, by "lectin" is meant a protein that binds sugar residues with high affinity. Most preferably, a FRIL family member molecule is a mannose/glucose-specific legume lectin.

As demonstrated in the examples below, FRIL family member molecules, and compositions of FRIL family member molecules, have many attributes as reagents to either alleviate the progenitor cell-depleting activity of a therapeutic (e.g., a chemotherapeutic) or to alleviate the symptoms of a condition where the patient's progenitor cells are depleted. For example, FRIL family members have unique properties and are the first soluble regulators reported to preserve hematopoietic stem cells and progenitors in a dormant state for extended periods, even in the presence of potent stimulators of proliferation and differentiation. Moreover, because mice tolerate very high levels of compositions of FRIL family members, this may permit more effective protection of stem cells and progenitors by preventing their recruitment during aggressive dose intensification regimens aimed at increasing frequency and dosage levels of chemotherapy. While the biological activity of Dl-FRIL is similar to cytokines (ng/ml range), as demonstrated in the examples below, mice tolerated up to a 1,000-fold more Dl-FRIL than cytokines.

In addition, by preserving hematopoietic stem cells and other progenitor cells in a dormant state, one or more members of the FRIL family allows for the administration of a broad range of cell cycle active chemotherapy drugs with greater frequency and higher dose. Thus, administration of a composition of one or more FRIL family members may permit more aggressive dose-intensification chemotherapy regimens for a broad range of chemotherapy drugs. Administration of a composition of a FRIL family member also provides for a larger reservoir of progenitor cells which could rapidly respond to stimulatory signals after completing chemotherapy.

In certain embodiment of the first aspect of the invention, the FRIL family member of the invention is from a legume *(e.g.,* a bean plant).

In some embodiments, the FRIL family member molecule of the invention is from a hyacinth bean *(i.e., Dolichos lab lab*), and has an amino acid sequence comprising the sequence of SEQ ID NO: 24. Preferably, the FRIL family member molecule of the invention isolated from a hyacinth bean has the amino acid sequence of SEQ ID NO: 2 or comprises a signal sequence having the amino acid sequence of SEQ ID NO: 4, and, even more preferably, is encoded by a nucleic acid having the nucleic acid sequence of SEQ ID NO: 1 or the nucleic acid sequence of SEQ ID NO: 3.

In some embodiments, the FRIL family member molecule of the invention is from a red kidney bean *(i.e., Phaseolus vulgaris*). Preferably, the FRIL family member of the invention isolated from a red kidney bean has the amino acid sequence of SEQ ID NO: 6, and, even more preferably, is encoded by a nucleic acid having the nucleic acid sequence of SEQ ID NO: 5.

In some embodiments, the FRIL family member of the invention is from a yam bean *(i.e., Sphenostylis stenocarpa*). Preferably, the FRIL family member of the invention isolated from a yam bean has the amino acid sequence comprising the amino acid sequence of SEQ ID NO: 8, more preferably has a β subunit having an amino acid sequence which comprises the amino acid sequence of SEQ ID NO: 9, even more preferably has an α subunit having an amino acid sequence which comprises the amino acid sequence of SEQ ID NO: 10, and, even more preferably, is encoded by a nucleic acid having a nucleic acid sequence which comprises the nudeic acid sequence of SEQ ID NO: 7.

In certain embodiments of the first aspect of the invention, the FRIL family member molecule is a mutant derived from a second member of the FRIL family, wherein the mutant is selected from the group consisting of a substitution mutant, a deletion mutant, an addition mutant, or a combination thereof (e.g., a mutant of Dl-FRIL or Pv-FRIL described below). For example, it is preferred to substitute amino acids in a sequence with equivalent amino acids. Groups of amino acids known normally to be equivalent are: (1) Ala(A), Ser(S), Thr(T), Pro(P), and Gly(G); (2) Asn(N), Asp(D), Glu(E), Gln(Q); (3) His(H), Arg(R), Lys(K); (4) Met(M), Leu(L), Ile(I), Val(V); and (5) Phe(F), Tyr(Y), Trp(W). Substitutions, additions, and/or deletions in an amino acid sequence can be made as long as the mutant FRIL family member molecule continues to satisfy the functional criteria described herein. An amino acid sequence that is substantially the same as another sequence, but that differs from the other sequence by means of one or more substitutions, additions, and/or deletions, is considered to be an equivalent sequence. Preferably, less than 50%, more preferably less than 25%, and still more preferably less than 10%, of the number of amino acid residues in a sequence are substituted for, added to, or deleted from the FRIL family member molecule upon which the mutant FRIL family member was derived.

In certain embodiments of the first aspect of the invention, the FRIL family member molecule is a fusion protein comprising a first portion and a second portion, wherein the first portion is derived from a second member of the FRIL family. By "fusion protein" is meant a molecule comprising at least two proteins or polypeptide fragments thereof joined together, wherein the proteins or polypeptide fragments thereof are not joined together in the naturally-occurring organism from which the proteins or polypeptide fragments thereof were derived. The two proteins or polypeptide fragments thereof of a fusion protein may be joined by any means, including, without limitation, a chemical linker, a peptide bond, or a noncovalent bond, such as an ionic bond. By "protein" or "polypeptide" is meant a chain of two or more amino acid residues joined with a peptide bond regardless of length or post-translational modification such as acetylation, glycosylation, lipidation, acetylation, or phosphorylation.

A FRIL family member molecule that is a fusion protein may comprise a first portion derived from a FRIL family member and a second portion derived from a protein or other molecule not related to the FRIL family (e.g., the heavy chain of an antibody).

An additional FRIL family member molecule that is a fusion protein is FRIL family member comprising the a subunit from a first FRIL family member and a β subunit from a second FRIL family member. Such a fusion protein may be generated, for example, by joining a nucleic acid sequence encoding the a subunit of the first FRIL family member in frame with a nucleic acid sequence encoding the β subunit of the second FRIL family member. The nucleic acid encoding such a fusion protein can be engineered to encode an enzyme-specific deavage site between the portion encoding the α subunit of the first FRIL family member and the portion encoding the β subunit of the second FRIL family member.

Where a FRIL family member is a fusion protein, identity of the fusion protein as a FRIL family member is determined by the sequence identity between the FRIL family member-derived portion of the fusion protein and a second FRIL family member, where the FRIL family member-derived portion of the fusion protein and the second FRIL family member share at least about 45% amino acid sequence identity, even more preferably at least about 50% identity, even more preferably at least about 55% identity, still more preferably at least about 60% identity, still more preferably at least about 65% identity yet more preferably at least about 75% sequence identity, still more preferably at least about 85% identity, and most preferably at least about 95% identity, with the amino acid sequence of a second member of the FRIL family.

A FRIL family member in accordance with the first aspect of the invention can also be a recombinant protein made by expressing a recombinant nucleic acid that encodes FRIL in a suitable host. Thus, in a second aspect, the invention features an essentially pure nucleic acid molecule encoding a member of the FRIL family of progenitor cell preservation factors. Exemplary purifications of the nucleic acid molecules of the invention from *Dolichos lab lab* and *Phaseolus vulgaris* are described below. As is well known, if an amino acid sequence (primary structure) is known, a family of nucleic acids can then be constructed, each having a sequence that differs from the others by at least one nucleotide, but where each different nucleic acid still encodes the same protein. For example, if a protein has been sequenced but its corresponding gene has not been identified, the gene can be acquired through amplification of genomic DNA using a set of degenerate primers that specify all possible sequences encoding the protein. Thus, a nucleic acid in accordance to this aspect of the invention need not have a naturally occurring sequence, but need only encode a FRIL family member according to the first aspect of the invention.

In accordance with the second aspect of the invention, a "member of the FRIL family of progenitor cell preservation factors" is a described above the first aspect of the invention. "Essentially pure" is used as described for the first aspect of the invention.

By a "recombinant nucleic acid" is meant a nucleic acid which encodes a FRIL family member molecule, or a portion encoding at least 15 contiguous amino acids thereof, or a mutant thereof, or a fusion protein comprising the molecule, portion thereof or mutant thereof or is capable of expressing an antisense molecule specifically complementary thereto, or a sense molecule that shares nucleic acid sequence identity thereto wherein the recombinant nucleic acid may be in the form of linear DNA or RNA, covalently closed circular DNA or RNA, or as part of a chromosome, provided however that it cannot be the native chromosomal locus for a FRIL family member molecule. Preferred recombinant nucleic acids of the invention are vectors, which may include an origin of replication and are thus replicatable in one or more cell type. Certain preferred recombinant nucleic acids are expression vectors, and further comprise at least a promoter and passive terminator, thereby allowing transcription of the recombinant nucleic acid in a bacterial, fungal, plant, insect or mammalian cell.
By "nucleic acid" or "nucleic acid molecule" as used herein, means any deoxyribonudeic acid (DNA) or ribonucleic acid (RNA), including, without limitation, complementary DNA (cDNA), genomic DNA, RNA, hnRNA, messenger RNA (mRNA), DNA/RNA hybrids, or synthetic nucleic acids *(e.g.,* an oligonucleotide) comprising ribonucleic and/or deoxyribonucleic acids or synthetic variants thereof. The nucleic acid of the invention includes, without limitation, an oligonucleotide or a polynucleotide. The nucleic acid can be single stranded, or partially or completely double stranded (duplex). Duplex nucleic acids can be homoduplex or heteroduplex.

In accordance with the second aspect of the invention, a nucleic acid encoding a FRIL family member has at least about 50% nucleic acid sequence identity with a nucleic acid encoding another member of the FRIL family, preferably at least about 555% nucleic acid sequence identity, more preferably at least about 60% nucleic acid sequence identity, more preferably at least about 65% nucleic acid sequence identity, still more preferably at least about 75% nucleic acid sequence identity, still more preferably at least about 85% nucleic acid sequence identity, and most preferably at least about 95% nucleic acid sequence identity with a nucleic acid encoding another member of the FRIL family. Percentage nucleic acid sequence identity can be determined as described for the first aspect of the invention.

A recombinant nucleic acid according to the second aspect of the invention can also be chemically synthesized by methods known in the art. For example, recombinant DNA can be synthesized chemically from the four nucleotides in whole or in part by methods known in the art. Such methods include those described in Caruthers, M.H., *Science* 230(4723):281-285,1985). DNA can also be synthesized by preparing overlapping double-stranded oligonucleotides, filling in the gaps, and ligating the ends together. See, generally, Sambrook et al., *supra,* and Glover and Hames, eds., *DNA Cloning,* 2d ed., Vols. 1-4, IRL Press, Oxford, 1995.

A recombinant nucleic acid molecule of the invention encoding a mutant FRIL family member can be prepared from wild-type DNA by site-directed mutagenesis (see, for example, Zoller and Smith, *Nucleic. Acids. Res.* 10:6487-6500, 1982; Zoller, M.J., *Methods Enzymol*. 100:468-500,1983; Zoller, M.J., *DNA* 3(6):479-488,1984.; and McPherson, M.J., ed., *Directed Mutagenesis: A Practical Approach*, IRL Press, Oxford, 1991. A recombinant nucleic acid of the second aspect of the invention can be amplified by methods known in the art. One suitable method is the polymerase chain reaction (PCR) method described in Saiki et al., *Science* 239:487,1988, Mullis et al., U.S. Patent No. 4,683,195, and Sambrook et al., *supra.* It is convenient to amplify the clones in the lambda-gt10 or lambda-gt11 vectors using lambda-gt10- or lambda-gt11-specific oligomers as the amplimers (available from Clontech, Palo Alto, CA). Larger synthetic nucleic acid structures can also be manufactured having specific and recognizable utilities according to the invention. For example, vectors (e.g., recombinant expression vectors) are known which permit the incorporation of recombinant nucleic acids of interest for cloning and transformation of other cells. Thus, the invention further includes vectors (e.g., plasmids, phages, and cosmids) which incorporate a nucleotide sequence of the invention, especially vectors which include the recombinant nucleic acid molecule of the invention for expression of a FRIL family member.

A recombinant nucleic acid of the invention can be replicated and used to express a FRIL family member following insertion into a wide variety of host cells in a wide variety of cloning and expression vectors. The host can be prokaryotic or eukaryotic. The nucleic acid can be obtained from natural sources and, optionally, modified. The genes can also be synthesized in whole or in part.

Cloning vectors can comprise segments of chromosomal, non-chromosomal and synthetic DNA sequences. Some suitable prokaryotic cloning vectors include plasmids from *E. coli*, such as colE1, pCR1, pBR322, pMB9, pUC, pKSM, and RP4. Prokaryotic vectors also include derivatives of phage DNA such as M13fd, and other filamentous single-stranded DNA phages.

Vectors for expressing proteins in bacteria, especially *E. coli,* are also known. Such vectors include the pK233 (or any of the *tac* family of plasmids), T7, and lambda P_{L}. Examples of vectors that express fusion proteins are PATH vectors described in Dieckmann and Tzagoloff (*J. Biol. Chem.* 260(3):1513-1520,1985). These vectors contain DNA sequences that encode anthranilate synthetase (TrpE) followed by a polylinker at the carboxy terminus. Other expression vector systems are based on beta-galactosidase (pEX); maltose binding protein (pMAL); glutathione S-transferase (pGST) (see, *e.g.,* Smith, D.B., *Gene* 67:31-40,1988 and Abath, F.G., *Peptide Research* 3(4):167-168,1990).
Vectors useful for cloning and expression in yeast are also available. A suitable example is the 2µ circle plasmid.

Suitable cloning/expression vectors for use in mammalian cells are also known. Such vectors include well-known derivatives of SV-40, adenovirus, cytomegalovirus (CMV) retrovirus-derived DNA sequences. Any such vectors, when coupled with vectors derived from a combination of plasmids and phage DNA, *i.e.*, shuttle vectors, allow for the isolation and identification of protein coding sequences in prokaryotes.

Further eukaryotic expression vectors are known in the art (e.g., Southern and Berg, *J. Mol. Appl. Genet.* 1:327-341,1982; Subramani et al., *Mol. Cell. Biol*. 1:854-864, 1981; Kaufmann and Sharp, *J. Mol. Biol*. 159:601-621,1982; Kaufmann and Sharp, *Mol. Cell. Biol*. 159:601-664,1982; Scahill et al., *Proc. Natl. Acad. Sci. USA* 80:4654-4659,1983; Urlaub and Chasin, *Proc. Natl. Acad. Sci. USA* 77:4216-4220, 1980).

The expression vectors useful in the present invention contain at least one expression control sequence that is operatively linked to the recombinant nucleic acid molecule or fragment thereof to be expressed. The control sequence is inserted in the vector in order to control and to regulate the expression of the recombinant nucleic acid of the invention. Examples of useful expression control sequences are the *lac* system, the *trp* system, the *tac* system, the *trc* system, major operator and promoter regions of phage lambda, the control region of fd coat protein, the glycolytic promoters of yeast, *e.g.,* the promoter for 3-phosphoglycerate kinase, the promoters of yeast acid phosphatase, *e.g.,* Pho5, the promoters of the yeast alpha-mating factors, and promoters derived from polyoma, adenovirus, retrovirus, and simian virus, *e.g.*, the early and late promoters or SV40, and other sequences known to control the expression of genes of prokaryotic or eukaryotic cells and their viruses or combinations thereof.

Useful expression hosts for expressing the recombinant nucleic acids of the invention include well-known prokaryotic and eukaryotic cells. Some suitable prokaryotic hosts include, for example, *E. coli,* such as *E. coli* SG-936, *E. coli* HB101, *E. coli* W3110, *E. coli* X1776, *E. coli* X2282, *E. coli* DHI, and *E. coli* MRC1, *Pseudomonas, Bacillus,* such as *B. subtilis,* and *Streptomyces.* Suitable eukaryotic cells include yeasts and other fungi, insect, animal cells, such as COS cells and CHO cells, human cells and plant cells in tissue culture.

Given the recombinant nucleic acid sequences disclosed herein, the artisan can further design recombinant nucleic acids having particular functions in various types of applications. For example, the artisan can construct oligonucleotides or polynucleotides for use as primers in nucleic acid amplification procedures, such as the polymerase chain reaction (PCR), ligase chain reaction (LCR), Repair Chain Reaction (RCR), PCR oligonucleotide ligation assay (PCR-OLA), and the like. Oligonucleotides useful as probes in hybridization studies, such as *in situ* hybridization, can be constructed. Numerous methods for labeling such probes with radioisotopes, fluorescent tags, enzymes, binding moieties (*e*.*g*., biotin), and the like are known, so that the probes of the invention can be adapted for easy detectability.

Oligonucleotides can also be designed and manufactured for other purposes. For example, the invention enables the artisan to design antisense oligonucleotides, and triplex-forming oligonucleotides, and the like, for use in the study of structure/function relationships. Homologous recombination can be implemented by adaptation of the nucleic acid of the invention for use as targeting means.

Recombinant nucleic acids of the invention produced as described above can further be modified to alter biophysical or biological properties by means of techniques known in the art. For example, the recombinant nucleic acid can be modified to increase its stability against nucleases *(e.g.,* "end-capping"), or to modify its lipophilicity, solubility, or binding affinity to complementary sequences. Methods for modifying nucleic acids to achieve specific purposes are disclosed in the art, for example, in Sambrook et al., *supra.* Moreover, the recombinant nucleic acid of the invention can include one or more portions of nucleotide sequence that are non-coding for a FRIL family member.

In a third aspect, the invention provides a pharmaceutical formulation comprising an essentially pure composition of one or more members of the FRIL family of progenitor cell preservation factors and a pharmaceutically acceptable carrier. By "pharmaceutically acceptable carrier" is meant any inert carrier that is non-toxic to the animal to which it is administered and that retains the therapeutic properties of the compound with which it is administered *(i.e.*, the FRIL family member). Pharmaceutically acceptable carriers and their formulations are well-known and generally described in, for example, Remington's Pharmaceutical Sciences (18th Edition, ed. A. Gennaro, Mack Publishing Co., Easton, PA, 1990). One exemplary pharmaceutically acceptable carrier is physiological saline. Pharmaceutical formulations of the invention may employ any pharmaceutically acceptable carrier, depending upon the route of administration of the composition.

Compositions of FRIL family members may be used safely and efficaciously as a therapeutics. The gastrointestinal tracts of animals come in constant contact with lectins, such as FRIL family members, in raw and/or cooked vegetables and fruits. Many lectins pass through the gastrointestinal tract biologically intact (Pusztai, A., *Eur. J. Clin. Nutr.* 47: 691-699, 1993). Some lectins interact with the gut and are transported into the peripheral blood circulation. For example, a recent study found peanut agglutinin (PNA) in the blood of humans at levels of 1-5 µg/ml an hour after ingesting 200 g of raw peanuts (Wang et al., *Lancet* 352: 1831-1832, 1998). Antibodies to dietary lectins are commonly found in people at levels of ~1 µg/ml (Tchernychev and Wilchek, *FEBS Lett.* 397:139-142,1996). These circulating antibodies do not block carbohydrate binding of the lectins.

In certain embodiments of the third aspect of the invention, administration of a therapeutically effective amount of the pharmaceutical formulation to a patient suffering from a condition whereby the patient's hematopoietic progenitor cells are depleted alleviates and/or reduces the condition in the patient.

In accordance with the third aspect of the invention, by "therapeutically effective amount" is meant a dosage of a composition of a FRIL family member or pharmaceutical formulation comprising a composition of a FRIL family member that is effective to alleviate and/or reduce either a condition whereby the patient's hematopoietic progenitor cells are depleted or a hematopoietic progenitor cell-depleting activity of a therapeutic (e.g., a chemotherapeutic). Preferably, such administration is systematic (e.g., by intravenous injection). When administered systemically, a therapeutically effective amount is an amount of between about 500 ng of the FRIL family member/kg total body weight and about 5 mg/kg total body weight per day. Preferably, a therapeutically effective amount is between about 500 ng/kg and 500 µg/kg total body weight of the FRIL family member per day. Still more preferably, a therapeutically effective amount is between about 5 µg/kg and 50 µg/kg total body weight of the FRIL family member per day. Most preferably, a therapeutically effective amount is an amount that delivers about 50 µg/kg total body weight of the FRIL family member per day.

A composition of a FRIL family member of the invention and pharmaceutical formulation comprising a composition of a FRIL family member of the invention may be administered patients having, or predisposed to developing, a condition whereby the patient's hematopoietic progenitor cells are depleted. Such a condition may be congenital. For example, the patient may have severe combined immunodeficiency or aplastic anemia.

The condition may also be induced by a drug. Thus, in certain embodiments of the third aspect of the invention, administration of a therapeutically effective amount of the pharmaceutical formulation to a patient prior to treatment of the patient with a therapeutic treatment having a hematopoietic progenitor cell-depleting activity alleviates the hematopoietic progenitor cell-depleting activity of the therapeutic in the patient. For example, cancer patients are often treated with radiotherapeutics or chemotherapeutics that have hematopoietic progenitor cell-depleting activity. By "hematopoietic progenitor cell-depleting activity" is meant an activity of a therapeutic treatment whereby the hematopoietic progenitor cells in the patient being treated with the therapeutic treatment are depleted, either by killing the progenitor cells or by inducing the progenitor cells to undergo irreversible differentiation. Non-limiting examples of therapeutic treatments having hematopoietic progenitor cell-depleting activity are the chemotherapeutic agents cytarabine (Ara-C), doxorubicin (Dox), daunorubicin, and 5-fluorouracil (5-FU).

In certain embodiments, administration of the pharmaceutical formulation of the invention to a patient prior to the treatment of the patient with a therapeutic treatment having a hematopoietic progenitor cell-depleting activity enables treatment of the patient with a higher dosage of the therapeutic treatment. The higher dosage of the therapeutic treatment may be accomplished by either an increased dose of the therapeutic treatment and/or an increased duration of treatment with the therapeutic treatment. For example, a child diagnosed with childhood Acute Myelogenous Leukemia (AML) is typically initially treated for the first seven days with daunorubicin at 45 mg/m² on Days 1-3 plus Ara-C at 100 mg/m² for 7 days plus GTG at 100 mg/m² for 7 days. The same child pretreated with a composition in accordance with this aspect of the invention may be able to tolerate a higher dosage (*i.e.*, higher dose and/or prolonged treatment period) of any or all of these chemotherapeutics. Such an increase in dosage tolerance of a therapeutic treatment(e.g., a chemotherapeutic) having a hematopoietic progenitor cell-depleting activity in a cancer patient is desirable since a higher dosage may result in the destruction of more cancerous cells.

The pharmaceutical formulations and/or compositions of the invention may be administered by any appropriate means. For example, the pharmaceutical formulations and/or compositions of the invention may be administered to an mammal within a pharmaceutically-acceptable diluent, carrier, or excipient, in unit dosage form according to conventional pharmaceutical practice. Administration may begin before the mammal is symptomatic for a condition whereby the patient's hematopoietic progenitor cells are depleted. For example, administration of the pharmaceutical formulations of the third aspect of the invention to a cancer patient may begin before the patient receives radiotherapy and/or chemotherapy treatment.

Any appropriate route of administration of a pharmaceutical formulation and/or composition of the invention may be employed, including, without limitation, parenteral intravenous, intra-arterial, subcutaneous, sublingual, transdermal, topical, intrapulmonary, intramuscular, intraperitoneal, by inhalation, intranasal, aerosol, intrarectal, intravaginal, or by oral administration. Pharmaceutical formulations and/or compositions of the invention may be in the form of liquid solutions or suspensions; for oral administration, formulations may be in the form of tablets or capsules; and for intranasal formulations, in the form of powders, nasal drops, or aerosols. The pharmaceutical formulations and/or compositions may be administered locally to the area affected by a condition whereby the patient's hematopoietic progenitor cells are depleted. For example, the pharmaceutical formulations and/or compositions of the invention may be administered directly into the patient's bone marrow. The pharmaceutical formulations and/or compositions of the invention may be administered systemically.

In certain embodiments of the third aspect of the invention, the patient is human or a domesticated animal. By "domesticated animal" is meant an animal domesticated by humans, including, without limitation, a cat, dog, elephants, horse, sheep, cow, pig, and goat. In some embodiments, the patient has cancer.

In some embodiments of the third aspect, the reatment is a radiotherapeutic or a chemotherapeutic treatment, including, without limitation, cytarabine (Ara-C), doxorubicin (Dox), or 5-fluorouracil (5-FU), or a combination of a radiotherapeutic and a chemotherapeutic.

In a fourth aspect, the invention provides a method for a method for alleviating and/or reducing the hematopoietic progenitor cell-depleting activity of a therapeutic treatment in a patient, comprising administering to the animal a therapeutically effective amount of a composition of a FRIL family member prior to administration of the therapeutic treatment to the patient. "Hematopoietic progenitor cell-depleting activity" is as described for the third aspect of the invention. Routes of administration of a composition of a FRIL family member of this aspect of the invention are as described for the administration of the pharmaceutical formulation of the third aspect of the invention. "Therapeutically effective amount" is as described for the third aspect of the invention.

In certain embodiments of the fourth aspect, the patient is a human or a domesticated animal. "Domesticated animal" is as described for the third aspect of the invention. In certain embodiments, the patient has cancer. In some embodiments, the therapeutic treatment is a radiotherapeutic or a chemotherapeutic treatment, including, without limitation, cytarabine (Ara-C), doxorubicin (Dox), or 5-fluorouracil (5-FU), or a combination of a radiotherapeutic and a chemotherapeutic.

In a fifth aspect, the invention provides a method for isolating a population of progenitor cells, comprising contacting a population of cells with a plurality of FRIL family member molecules, and separating the unbound cells, wherein the cells bound to the FRIL family member molecules are an isolated population of progenitor cells. "FRIL family member molecule" and "progenitor cell" are as described for the first aspect of the invention. By "unbound cell" is meant a cell that does not bind to a FRIL family member. "Bind" is a described for the first aspect of the invention.

By "isolated" is meant a population of progenitor cells that is separated from a larger population of cells, wherein the percentage of progenitor cells in the isolated population is at least two fold greater than the percentage of progenitor cells in the larger population. Preferably, the percentage of progenitor cells in the isolated population is at least four fold greater than the percentage of progenitor cells in the larger population. A non-limiting example of the increased percentage of progenitor cells in an isolated population of progenitor cells of the invention is shown below in Table 11. Preferably, the isolated population of progenitor cells of the invention is at most 2% of the total population of umbilical cord blood mononuclear cells (CB mnc). Preferably, the population of progenitor cells of the invention is at most 1% of the total population of umbilical cord blood mononuclear cells (CB mnc). Preferably, an isolated population of progenitor cells binds to a normally glycosylated FLT3 receptor. "Normally glycosylated FLT3 receptor" is as defined above.

In preferred embodiments, the isolated population of progenitor cells is from a human or is from a domesticated animal.

In certain embodiments of the fifth aspect of the invention, the FRIL family member molecules are detectably labeled. By "detectably labeled" is meant that the FRIL family member is attached to a label that is detectable visually or instrumentally. Detectable labels such as enzymes and chromophoric molecules can be conjugated to the FRIL family member molecules by means of coupling agents, such as dialdehydes, carbodiimides, and dimaleimides. Numerous methods of labeling proteins are known in the art. The label can also be directly attached through a functional group on the FRIL family member. Such a functional group may be present on the FRIL family member molecule to be detectably labeled; alternatively, the FRIL family member molecules can be modified using standard techniques to contain a functional group. Some examples of suitable functional groups include, without limitation, amino, carboxyl, sulfhydryl, maleimide, isocyanate, isothiocyanate.

In certain embodiments, the detectable label is radioactive or non-radioactive. Some examples of useful radioactive labels include ³²P, ¹²⁵I, ¹³¹I, and ³H. Use of radioactive labels have been described in U.K. patent document 2,034,323, and U.S. Patent Nos. 4,358,535, and 4,302,204. Some examples of non-radioactive labels include enzymes, chromophores, atoms and molecules detectable by electron microscopy, and metal ions detectable by their magnetic properties.

In certain embodiments of the fifth aspect of the invention, the detectable label is an enzymatic label. Some useful enzymatic labels include enzymes that cause a detectable change in a substrate. Some useful enzymes and their substrates include, for example, horseradish peroxidase (pyrogallol and o-phenylenediamine), beta-galactosidase (fluorescein beta-D-galactopyranoside), and alkaline phosphatase (5-bromo-4-chloro-3-indolyl phosphate/nitro blue tetrazolium). The use of enzymatic labels have been described in U.K. 2,019,404, and EP 63,879, each incorporated herein by reference, and by Rotman, *Proc. Natl. Acad. Sci. USA* 47:1981-1991, 1961.

In certain embodiments of this aspect of the invention, the detectably labeled FRIL family member molecules are labeled by being specifically bound by an antibody that is detectably labeled.

In certain embodiments of this aspect of the invention, the FRIL family member molecules are conjugated to a receptor (or ligand) and is detectably labeled by binding the receptor (or ligand) with the ligand (or receptor), wherein the ligand (or receptor) is detectably labeled. Any of the known ligand-receptor combinations is suitable. Some suitable ligand-receptor pairs include, for example, biotin-avidin or -streptavidin, and antibody-antigen. In certain embodiments, biotin-avidin combination is preferred.

In certain embodiments, the detectable label is a chromophore. Useful chromophores include, for example, fluorescent, chemiluminescent, and bioluminescent molecules, as well as dyes. Some specific chromophores useful in the present invention include, for example, fluorescein, rhodamine, Texas red, phycoerythrin, umbelliferone, luminol.

In certain embodiments of this aspect of the invention, where the FRIL family member molecules are detectably labeled to a chromophore, the unbound cells are separated by using a flow cytometry cell sorter to sort the population of cells contacted with the FRIL family member molecules detectably labeled with a fluorescent marker.

In certain embodiments of the fifth aspect of the invention, the FRIL family member molecules are immobilized on a solid support. "Solid support" includes any surface, including, without limitation, the surface of a sepharose bead, a gel, a matrix, a magnetic bead, and a plastic surface (e.g., the bottom of a tissue culture dish or flask).

In some embodiments, the solid support is a bead, for example, a magnetic bead. In some embodiments, where the solid support is a magnetic bead, the unbound cells are separated by applying a magnet to the population of cells contacted with the FRIL family member molecules immobilized on the magnetic bead. In further embodiments, the population of cells bound to the FRIL family member molecules immobilized on a magnetic bead is rinsed with a physiologically acceptable solution while the magnet is applied. By "physiologically acceptable solution" is meant an inert solution, such as sterile saline solution or tissue culture medium, which is non-toxic to the cells.

Methods for isolating cells that bind FRIL family member molecule-coated magnetic beads are described below in Example 16. Magnetic beads are commerically available (e.g., from Dynabeads Tosylactivated, Lake Success, NY; or from Miltenyi Biotec, Auburn, CA). Since the FRIL family member is protein, it can be conjugated to a magnetic bead via amino- or sulfhydryl-groups of the protein. A FRIL family member molecule can also immobilized on magnetic beads by a biotin-strepavidin interaction.

Preferred magnetic beads are the MACS super-paramagnetic MicroBeads (from Miltenyi Biotec) which are extremely small, approximately 50 nm in diameter (MACS beads are about one million times smaller in volume than eukaryotic cells). Because MACS beads react like magnetic antibodies, magnetic labeling is achieved within minutes. MACS MicroBeads form a stable colloidal suspension and do not precipitate or aggregate in magnetic fields. Because of their size and composition (iron oxide and polysaccharide) make the MACS biodegradable, so labeled cells retain their physiological function. This property of MACS beads is particularly useful for bead-sorted FRIL family member-binding cells, which bind the FRIL family member with such high affinity that it is difficult to remove the beads.

In certain embodiments of the fifth aspect, the solid support is the bottom of a tissue culture plate. In some embodiments, the unbound cells are separated by rinsing the population of cells contacted with the FRIL family member molecules immobilized on the bottom of a tissue culture plate with a physiologically acceptable solution.

FRIL family member molecules may be directly or indirectly attached to the bottom of a tissue culture plate. Following a standard "panning" protocol (see, *e.g.,* Stengelin et al., *EMBO J.* 7(4):1053-1059,1988; Aruffo and Seed, *Proc. Natl. Acad. Sci. USA* 84(23): 8573-8577,1987), a population of cells suspected of containing FRIL family member-binding progenitor cells is incubated on the plate. The plate is then gently rinsed with a physiologically acceptable solution, thereby removing the unbound cells while leaving the FRIL family member-binding population of cells attached to the FRIL family member-coated plate.

In preferred embodiments of the fifth aspect of the invention, the isolated population of progenitor cell is a population of hematopoietic progenitor cells. In various embodiments, the population of cells is whole blood, umbilical cord blood, bone marrow cells, or fetal liver cells.

In certain embodiments of the fifth aspect of the invention, the population of cells is a sorted population of cells, wherein a cell of the sorted population does not express CD11b, CD11c, or CD38. Because more primitive progenitor cell stypically expresses few cell surface molecules, prior to isolating a population of progenitor cells that binds to a FRIL family member, the population of cells is preferably sorted to first remove cells that express one or more of the following cell surface molecules: CD11b, CD11c, and CD38. Following this negative sort (*i.e.*, a sort, wherein the cells retained do not express CD11b, CD11c, and/or CD38), the sorted population is positively sorted for an ability to bind a FRIL family member.

In certain embodiments, the sorted population of cells is sorted by flow cytometry or by magnetic bead selection. Thus, a population of cells *(e.g.,* human umbilical cord blood cells) may be first contacted with chromophore-labeled antibodies (or other molecule such as a ligand) which specifically bind CD11b, CD11c, and/or CD38. Following binding, the population of cells is then negatively sorted by flow cytometry, where the cells which are not bound by the antibodies (and so do not express CD11b, CD11c, and/or CD38) are retained and further sorted for an ability to bind a FRIL family member, wherein the population of cells that binds a FRIL family member is an isolated population of progenitor cells according to the invention.

A population of cells may also be contacted with a molecule, such as an antibody, which specifically binds CD11b, CD11c, and/or CD38, wherein the molecule is attached to a solid support, such as a magnetic bead. The population is then negatively sorted by applying a magnet to the beads, and retaining the cells that do not bind the beads and so are not attracted to the magnet. These sorted cells are then further sorted for an ability to bind a FRIL family member, wherein the population of cells that binds a FRIL family member is an isolated population of progenitor cells according to the invention.

In a sixth aspect, invention provides an isolated population of progenitor cells isolated by a method comprising contacting a population of cells with a plurality of FRIL family member molecules, and separating the unbound cells, wherein the cells bound to the FRIL family member are an isolated population of progenitor cells. "FRIL family member molecule," "progenitor cell," "unbound cell," and "bind" are as described above.

In preferred embodiments, the isolated population of progenitor cells is from a human or a domesticated animal.

In certain embodiments of the sixth aspect, the cells of the isolated population do not express CD34 on their cell surface. In certain embodiments, the cells of the isolated population express the FLK1, FLT1, FLT3, FLT4, or Kit receptor tyrosine kinases. In some embodiments, the cells of the isolated population express the CD11b or CD11c cell surface molecules. In preferred embodiments, the cells of the isolated population express FLT3.

In various embodiments of the sixth aspect of the invention, the cells of the isolated population are hemangioblasts, messenchymal stem cells, bone progenitor cells, hepatic progenitor cells, endothelial progenitor cells, hematopoietic progenitor cells, embryonal stem cells, brain progenitor cells, or dendritic progenitor cells. By "hemangioblast" is meant a cell that is a progenitor cell for both hemaopoietic and endothelial lineages. Preferably, the cells of the isolated population are hematopoietic progenitor cells. By a "messenchymal stem cells" is meant the population of cells that is the progenitor for bone marrow stromal cells, including, without limitation, adipose tissue cells, cartilage-producing cells, muscle cells, and bone cells. Such cells of this aspect of the invention can be used, for example, for tissue repair.

Determination of what type of cells a progenitor cell of the invention will give rise to is made by the various progenitor cell assays described below. For example, if a progenitor cell is suspected of being an endothelial progenitor cell, the cell may be cultured in a methylcellulose progenitor cell assay in the presence of vascular endothelial growth factor. Should cells arising from such a culture have endothelial cell markers, the progenitor cell of the invention is a endothelial progenitor cell or perhaps a hemangioblast.

In certain embodiments of the sixth aspect of the invention, where the cells of the isolated population are hematopoietic progenitor cells, transplantation of the cell into an animal lacking a population of hematopoietic progenitor cells sufficient to enable survival of the animal reconstitutes the animal, wherein the transplanted animal survives. Determination of the ability of a hematopoietic progenitor cell to reconstitute a animal lacking a population of hematopoietic progenitor cells sufficient to enable survival of the animal may be made using the methods described below for the NOD-SCID mouse.

In certain embodiments, the hematopoietic progenitor cells are from a mouse or a human and the animal is a mouse. In some embodiments, the mouse is a severe combined immunodeficient (SCID) mouse (*e*.*g*., the NOD-SCID mouse described below) or a mouse that has been exposed to a sublethal dose of radiation and/or chemotherapy

In certain embodiments of the sixth aspect of the invention, the hematopoietic progenitor cells are from a human and the animal is a human. In some embodiments, the human is a cancer patient receiving a treatment that depletes the patient's hematopoietic progenitor cells. In some embodiments, the treatment is a radiotherapeutic or a chemotherapeutic treatment, including, without limitation, cytarabine (Ara-C), doxorubicin (Dox), or 5-fluorouracil (5-FU), or a combination of a radiotherapeutic and a chemotherapeutic.

In a seventh aspect, the invention provides a method for preserving progenitor cells *ex vivo*, comprising contacting a population of cells comprising at least one progenitor cell with an effective amount of a composition of a FRIL family member for an effective period of time, wherein the progenitor cell in the population are rendered quiescent. "FRIL family member" and "progenitor cell" are as described above for the first aspect of the invention.

In accordance with this aspect of the invention, the terms "effective amount" and "effective period of time" are used to denote known treatments at dosages and for periods of time effective to preserve progenitor cells. Where administered to a patient, preferably, such administration is systemic (*e*.*g*., by intravenous injection). Effective amounts and effective periods of time can be determined using the models and assays described herein. For example, the Examples below describe the preservation of progenitor cells that have SCID-reconstituting ability. In accordance with the invention, an effective amount may range from about 0.1 ng/mL to about 1 µg/mL of a FRIL family member, preferably about 1.0 ng/mL to to about 1.0 µg/mL, more preferably about 1.0 ng/mL to about 100 ng/mL, even more preferably about 10 ng/mL to about 50 ng/mL, and most preferably about 50 ng/mL of a FRIL family member in culture. In accordance with the invention, an effective period of time includes culturing the cells in the presence of a FRIL family member for between would include from about 2 hours to 5 days, more preferably from about 12 hours to about 3 days, and most preferably for about 24 hours..

In certain embodiments of the seventh aspect, the progenitor cells are from a human or from a domesticated animal. In certain embodiments, the population of cells is bone marrow cells.

In some embodiments, the non-progenitor cells in the population of cells differentiate or die. Thus, although the progenitor cells in the culture do not actually expand in number, they are enriched relative to the number of cells in the culture.

In certain embodiments of the seventh aspect, the population of cells is removed from a cancer patient prior to treatment of the cancer patient with a therapeutic treatment having a hematopoietic progenitor cell-depleting activity. In some embodiments, the therapeutic treatment is a radiotherapeutic or a chemotherapeutic treatment, including, without limitation, cytarabine (Ara-C), doxorubicin (Dox), or 5-fluorouracil (5-FU), or a combination of a radiotherapeutic and a chemotherapeutic.

In an eighth aspect, the invention provides a method for preserving progenitor cells *in vivo,* comprising administering to a patient a therapeutically effective amount of a composition of a FRIL family member for a therapeutically effective period of time, wherein the progenitor cells in the patient are rendered quiescent. "FRIL family member" and "progenitor cell" are as described above for the first aspect of the invention. "Therapeutically effective amount" is as described for the third aspect of the invention.

By "therapeutically effective period of time" is meant treatment for a period of time effective to preserve progenitor cells. Where administered to a patient, preferably, such administration is systemic (e.g., by intravenous injection). Effective amounts and effective periods of time can be determined using the models and assays described herein. For example, the examples below describe the preservation of progenitor cells that have SCID-reconstituting ability. In accordance with the invention, a therapeutically effective period of time is injecting a therapeutically effective amount of a composition and/or pharmaceutical formulation of a FRIL family member between about 5 days before the patient receives treatment with a therapeutic treatment (*e*.*g*., a chemotherapeutic) having a progenitor cell-depleting activity to about 2 hours prior to treatment with the therapeutic treatment, wherein the therapeutically effective amount of a composition and/or pharmaceutical formulation of the FRIL family member is administered daily. In accordance with the invention, a preferred therapeutically effective period of time is injecting a patient (e.g., a cancer patient) with a therapeutically effective amount of a composition and/or pharmaceutical formulation of a FRIL family member between about 2 days before the patient receives treatment with a therapeutic treatment (e.g., a chemotherapeutic) having a progenitor cell-depleting activity to about 1 day prior to treatment with the therapeutic treatment, wherein the therapeutically effective amount of a composition and/or pharmaceutical formulation of a FRIL is administered daily. It will be understood that once the patient starts to receive treatment of a therapeutic treatment having a progenitor cell-depleting activity, the therapeutically effective amount of a composition and/or pharmaceutical formulation of a FRIL family member may be different from the therapeutically effective amount of the a composition and/or pharmaceutical formulation of a FRIL family member that the patient received prior to receiving treatment with the therapeutic treatment.

Preferably, the patient is a human or a domesticated animal. "Domesticated animal" is as described for the third aspect of the invention.

In certain embodiments of the eighth aspect of the invention, the patient is a cancer patient. In some embodiments, the effective amount of the composition of the FRIL family member is administered prior to the treatment of the patient with a therapeutic treatment having a hematopoietic progenitor cell-depleting activity. In some embodiments, the therapeutic treatment is a radiotherapeutic or a chemotherapeutic treatment, including, without limitation, cytarabine (Ara-C), doxorubicin (Dox), or 5-fluorouracil (5-FU), or a combination of a radiotherapeutic and a chemotherapeutic.

In a ninth aspect, the invention provides a method for identifying a progenitor cell, comprising contacting a candidate cell with a FRIL family member molecule, wherein binding of the candidate cell to the FRIL family member molecule identifies the candidate cell as a progenitor cell. "FRIL family member molecule" and "progenitor cell" are as described above for the first aspect of the invention.

In certain embodiments of the ninth aspect, the candidate cell is in a population of cells. In certain embodiments, the candidate cell is from a human.

In a tenth aspect, the invention provides a progenitor cell identified by a method comprising contacting a candidate cell with a FRIL family member molecule, wherein binding of the candidate cell to the FRIL family member molecule identifies the candidate cell as a progenitor cell. "FRIL family member" and "progenitor cell" are as described above for the first aspect of the invention.

In an eleventh aspect, the invention provides a method for identifying a composition of a member of the FRIL family of progenitor cell preservation factors, comprising contacting a candidate compound with a glycosylated extracellular domain of an FLT3 receptor, wherein the glycosylation pattern of the extracellular domain of the FLT3 receptor is the same as the glycosylation pattern of an extracellular domain of a normally glycosylated FLT3 receptor, wherein a candidate compound that binds the glycosylated extracellular domain of the FLT3 receptor is identified as a composition of a FRIL family member. "FRIL family member," "progenitor cell," and "normally glycosylated FLT3 receptor" are as described above for the first aspect of the invention.

In accordance with the eleventh aspect of the invention, the normally glycosylated extracellular domain of the FLT3 receptor may expressed on a cell surface (*e.g.*, on the surface of an NIH 3T3 cell, as described in the examples below). Any normal cell may be transfected with a nucleic acid sequence encoding the extracellular domain of the FLT3 receptor (from, *e.g.*, a human or a mouse) provided that the cell normally glycosylates the FLT3 receptor it expresses on its cell surface. It should be noted that the cell need not be transfected with a nucleic acid sequence encoding the entire FLT3 receptor. For example, the cell may be transfected with a nucleic acid molecule encoding a fusion protein comprising the intracellular domain of a non-FLT3 receptor *(e.g.,* the Fms receptor) and the extracellular domain of the FLT3 receptor. A candidate compound according to this aspect of the invention is then incubated with the cell expressing the normally glycosylated extracellular domain of the FLT3 receptor, and binding of the candidate compound to the cell (as can be measured, as described below, by survival of the cell) identifies the candidate compound as a FRIL family member.

Alternatively, the normally glycosylated extracellular domain of the FLT3 receptor need not be expressed by a cell. Instead, the normally glycosylated extracellular domain of the FLT3 receptor may be detectably labeled or immobilized on a solid support (*e.g.*, a magnetic bead). For example, the normally glycosylated extracellular domain of the FLT3 receptor can be bound to the surface of a 96 well microtiter plate. Compositions comprising different candidate compounds (or pools of such compounds) can be detectably labeled (*e.g.*, with biotin), and added to the wells. After incubation for an amount of time required for binding of a positive control (*e*.*g*., a composition of a known FRIL family member) the plate is washed and detectably labeled avidin is added to each well. The plate can then be read on a standard microtiter plate reader to determine binding of a composition of a candidate compound to the plate-bound normally glycosylated extracellular domain of the FLT3 receptor, wherein binding (as measured by detection of the bound detectably labeled avidin) identifies the candidate compound in the composition as a FRIL family member.

In certain embodiments of the eleventh aspect, the candidate compound is a lectin. In certain embodiments, the lectin is synthetic. In certain embodiments, the lectin is from a legume.

In a twelfth aspect, the invention provides an essentially pure composition of a FRIL family member identified by the method comprising contacting a candidate compound with a glycosylated extracellular domain of an FLT3 receptor, wherein the glycosylation pattern of the extracellular domain of the FLT3 receptor is the same as the glycosylation pattern of an extracellular domain of a normally glycosylated FLT3 receptor, wherein a candidate compound that binds the glycosylated extracellular domain of the FLT3 receptor is identified as an essentially pure composition of a member of the FRIL family. "FRIL family member," "progenitor cell," and "normally glycosylated FLT3 receptor," and "essentially pure" are as described above for the first aspect of the invention.

The following examples are intended to further illustrate certain preferred embodiments of the invention and are not limiting in nature. Those skilled in the art will recognize, or be able to ascertain, using no more than routine experimentation, numerous equivalents to the specific substances and procedures described herein. Such equivalents are considered to be within the scope of this invention, and are covered by the following claims.

### EXAMPLE 1

### Purification and Cloning of a FRIL family member, D1-FRIL

### Purification of D1-FRIL from Dolichos lab lab

Seeds from the hyacinth beans *(Dolichos lab lab)* were purchased from Stokes Seeds (Buffalo, NY) and grown in a greenhouse. Dry seeds were ground in a coffee mill and the power was extracted in 5 volumes of 50 mM Tris/HCl containing 1 nM each of MgCl₂ and CaCl₂ for 4 hours at 4°C. Bean solids were pelleted by centrifugation at 10,000 x g for 20 min. The pH of the supernatant was acidified to pH 4.0 with acetic acid, followed by a second centrifugation to clarify the supernatant, and finally the pH was readjusted to 8.0 with sodium hydroxide. This crude extract was stored at -20°C.

Single-step purification of the FRIL family member was achieved by binding to a mannose-Sepharose matrix (Sigma). The gel (*i.e.*, matrix) was tumbled with the thawed crude extract for 4-12 hours at 4°C, carefully washed several times with 50 mM Tris/HCl containing 1 nM each of MgCl₂ and CaCl₂, and then eluted with 20 mM α-methyl α-D-mannoside. Because this FRIL family member was isolated from *Dolichos lab lab,* it is referred to herein as Dl-FRIL.

### RNA Isolation and cDNA Synthesis of Dl-FRIL

Total RNA was prepared from mid-maturation *Dolichos lab lab* seeds stored at -70°C following the procedure of Pawloski et al.Mol. Plant Biol. Manual 5: 1-13, 1994. Poly (A⁺) RNA was obtained from this total RNA using the PolyATract mRNA Isolation System (Promega) according to the manufacturer's instructions. Avian myeloblastosis virus reverse transcriptase (Promega) was used to generate cDNA from 0.5 µg poly(A⁺)RNA, or from 3.0 µg of total RNA, using 1 µg of oligo(dT) in standard reaction conditions (Sambrook et al., *Molecular Cloning. A Laboratory Manual*, 2d ed., Cold Spring Harbor Laboratory, Cold Spring Harbor, 1989).

### Polymerase Chain Reaction and cDNA Cloning of D1-FRIL

Based on the amino acid sequence published by Gowda et al., *J. Biol. Chem*. 269:18789-18793,1994, two degenerate oligonucleotide primers were designed using *Phaseolus* codon usage (Devereux et al., *Nucleic Acids Res.* 12:387-394, 1984):

MLA AA(AG)TT(TC)GA(TC)CC(AT)AA(TC)CA(AG)GA(AG)GA (SEQ ID NO: __)

MLZ TT(AT)CC(AG)TT(TC)TGCCA(AG)TCCCA (SEQ ID NO: __)

A 500+ bp product was amplified from cDNA prepared as described abpve, by 30 cycles of polymerase chain reaction (PCR), each cycle comprising 40 seconds at 94°C, 40 seconds at 50°C, 60 seconds at 72°C, followed by an extension step at 72°C for 10 min. Reactions were performed in 50 µL containing 30 pmol of each primer, 0.2 mM deoxyribonucleotides, and 0.5 unit of AmpliTaq polymerase (Perkin Elmer, Norwalk, CT) in the corresponding buffer.

The 500 bp product obtained by PCR was cloned in the cloning vector, pCR2.1 (Invitrogen, Carlsbad, CA), and sequenced by sequenase dideoxy chain termination (United States Biochemicals) using the following primers:

GTACCGAGCTCGGAT (SEQ ID NO:__)

TCTAGATGCATGCTCGAG (SEQ ID NO:__).

This sequence was designated "D1-FRILa," as relating to the gene encoding the protein of interest, designated "D1-FRIL" as noted above.

Based on the sequence of the D1-FRILa amplified product, a specific primer was prepared:

MLX GTTGGACGTCAATTCCGATGTG (SEQ ID NO: __)

A degenerate primer corresponding to the first five amino acids of the sequence published by Gowda et al., *J. Biol. Chem*. 269:18789-18793, 1994 was also prepared:

MLI GC(TC)CA(AG)TC(TC)CT(TC)TC(TC)TT (SEQ ID NO: __)

The MLX and MLI primers were used in combination to amplify a 480 bp product from cDNA prepared as described above, through 30 PCR cycles using the same conditions described above. This secondary amplified fragment was cloned in the pCR2.1 vector and sequenced as described above, and was designated "Dl-FRILb."

The 3' end of *Dl-FRIL* was obtained through rapid amplification of cDNA ends by polymerase chain reaction (RACE-PCR) (see, *e.g.*, Frohman "RACE: Rapid amplification of cDNA ends," pp. 28-38 in *PCR Protocols: A Guide to Methods and Applications,* Innis MA, Gelfand DH, Sninsky JJ, and White TJ, eds., Academic Press, San Diego, 1990) using the 5'/3' RACEKIT (Boehringer Mannheim, Indianapolis, IN) according to the manufacturer's instructions. In the cDNA synthesis for the 3' RACE, an oligo(dT) anchor primer ("AP") supplied with the kit was used, at a concentration of 32.5 µM, using the standard conditions described earlier in this Example.

AP GACCACGCGTATCGATGTCGAC (SEQ ID NO: __)

Nested PCR amplifications were performed using the AP anchor primer in combination with a specific primer having the following sequence:

MLB AAGTTAGACAGTGCAGGAAAC (SEQ ID NO: __).

The amplification conditions were again 30 cycles of 40 seconds at 94°C, 40 seconds at 55°C, 60 seconds at 72°C each; with an extension step at 72°C for 10 min. A 900+ bp product was obtained, which was subcloned in pCR2.1 and sequenced as described above, and was designated "D1-FRILc" (SEQ ID NO:1).

To obtain the full length cDNA done, the anchor primer AP was used in combination with a specific primer corresponding to the first 5 amino acids encoded at the 5'-terminus:

MLII GCACAGTCATTGTCATTTAG (SEQ ID NO: __).

The full length cDNA was obtained through 30 cycles of PCR, each cycle comprising 60 seconds at 94°C, 60 seconds at 58°C, 90 seconds at 72°C, with an extension step at 72°C for 10 min. The reaction was performed in 100 µL containing 30 pmol of each primer, 0.2 mM deoxyribonucleotide, 1.0 unit of Pfu polymerase (Stratagene, La Jolla, CA). The MLII and AP primers were designed to generate an *Eco*RI site at each end (3' and 5') of the polynucleotide sequence. The full length cDNA was ligated into the EcoRI site of the cloning vector pCR2.1, resulting in the final product "pCR2.1-DLA" illustrated schematically in Fig.1.

### The Nucleotide Sequence of Dl-FRIL

The Dl-FRILc clone was sequenced completely using the dideoxy chain termination method. The nucleotide sequence of the full-length cDNA was determined to be:

1 GCACAGTCAT TGTCATTTAG TTTCACCAAG TTTGATCCTA ACCAAGAGGA
51 TCTTATCTTC CAAGGTCATG CCACTTCTAC AAACAATGTC TTACAAGTCA
101 CCAAGTTAGA CAGTGCAGGA AACCCTGTGA GTTCTAGTGC GGGAAGAGTG
151 TTATATTCTG CACCATTGCG CCTTTGGGAA GACTCTGCGG TATTGACAAG
201 CTTTGACACC ATTATCAACT TTGAAATCTC AACACCTTAC ACTTCTCGTA
251 TAGCTGATGG CTTGGCCTTC TTCATTGCAC CACCTGACTC TGTCATCAGT
301 TATCATGGTG GTTTTCTTGG ACTCTTTCCC AACGCAAACA CTCTCAACAA
351 CTCTTCCACC TCTGAAAACC AAACCACCAC TAAGGCTGCA TCAAGCAACG
401 TTGTTGCTGT TGAATTTGAC ACCTATCTTA ATCCCGATTA TGGTGATCCA
451 AACTACATAC ACATCGGAAT TGACGTCAAC TCTATTAGAT CCAAGGTAAC
501 TGCTAAGTGG GACTGGCAAA ATGGGAAAAT AGCCACTGCA CACATTAGCT
551 ATAACTCTGT CTCTAAAAGA CTATCTGTTA CTAGTTATTA TGCTGGGAGT
601 AAACCTGCGA CTCTCTCCTA TGATATTGAG TTACATACAG TGCTTCCTGA
651 ATGGGTCAGA GTAGGGTTAT CTGCTTCAAC TGGACAAGAT AAAGAAAGAA
701 ATACCGTTCA CTCATGGTCT TTCACTTCAA GCTTGTGGAC CAATGTGGCG
751 AAGAAGGAGA ATGAAAACAA GTATATTACA AGAGGCGTTC TGTGATGATA
801 TATGTGTATC AATGATTTTC TATGTTATAA GCATGTAATG TGCGATGAGT
851 CAATAATCAC AAGTACAGTG TAGTACTTGT ATGTTGTTTG TGTAAGAGTC
901 AGTTTGCTTT TAATAATAAC AAGTGCAGTT AGTACTTGT (SEQ ID NO:1)

The *Dl-FRIL* nucleotide sequence enabled inference of the following derived amino acid sequence for the D1-FRIL protein:

AQSLSFSFTK FDPNQEDLIF QGHATSTNNV LQVTKLDSAG NPVSSSAGRV
LYSAPLRLWE DSAVLTSFDT IINFEISTPY TSRIADGLAF FIAPPDSVIS
YHGGFLGLFP NANTLNNSST SENQTTTKAA SSNVVAVEFD TYLNPDYGDP
NYIHIGIDVN SIRSKVTAKW DWQNGKIATA HISYNSVSKR LSVTSYYAGS
KPATLSYDIE LHTVLPEWVR VGLSASTGQD KERNTVHSWS FTSSLWTNVA

The naturally-occurring signal sequence from the FRIL family member isolated from Dolichos lab lab *(i.e.,* Dl-FRIL) has the following sequence:

MASSNLLTLA LFLVLLTHAN SA (SEQ ID NO: 4)

This sequence is located directly N-terminal to the first amino acid of SEQ ID NO: 2. The nucleic acid sequence of the naturally-occurring Dl-FRIL protein is provided below.

1 ATGGCTTCCT CCAACTTACT CACCCTAGCC CTCTTCCTTG TGCTTCTCAC
51 CCACGCAAAC TCAGCCGCAC AGTCATTGTC ATTTAGTTTC ACCAAGTTTG
101 ATCCTAACCA AGAGGATCTT ATCTTCCAAG GTCATGCCAC TTCTACAAAC
151 AATGTCTTAC AAGTCACCAA GTTAGACAGT GCAGGAAACC CTGTGAGTTC
201 TAGTGCGGGA AGAGTGTTAT ATTCTGCACC ATTGCGCCTT TGGGAAGACT
251 CTGCGGTATT GACAAGCTTT GACACCATTA TCAACTTTGA AATCTCAACA
301 CCTTACACTT CTCGTATAGC TGATGGCTTG GCCTTCTTCA TTGCACCACC
351 TGACTCTGTC ATCAGTTATC ATGGTGGTTT TCTTGGACTC TTTCCCAACG
401 CAAACACTCT CAACAACTCT TCCACCTCTG AAAACCAAAC CACCACTAAG
451 GCTGCATCAA GCAACGTTGT TGCTGTTGAA TTTGACACCT ATCTTAATCC
501 CGATTATGGT GATCCAAACT ACATACACAT CGGAATTGAC GTCAACTCTA
551 TTAGATCCAA GGTAACTGCT AAGTGGGACT GGCAAAATGG GAAAATAGCC
601 ACTGCACACA TTAGCTATAA CTCTGTCTCT AAAAGACTAT CTGTTACTAG
651 TTATTATGCT GGGAGTAAAC CTGCGACTCT CTCCTATGAT ATTGAGTTAC
701 ATACAGTGCT TCCTGAATGG GTCAGAGTAG GGTTATCTGC TTCAACTGGA
751 CAAGATAAAG AAAGAAATAC CGTTCACTCA TGGTCTTTCA CTTCAAGCTT
801 GTGGACCAAT GTGGCGAAGA AGGAGAATGA AAACAAGTAT ATTACAAGAG
851 GCGTTCTGTG ATGATATATG TGTATCAATG ATTTTCTATG TTATAAGCAT
901 GTAATGTGCG ATGAGTCAAT AATCACAAGT ACAGTGTAGT ACTTGTATGT
951 TGTTTGTGTA AGAGTCAGTT TGCTTTTAAT AATAACAAGT GCAGTTAGTA
1001 CTTGT (SEQ ID NO: 3)

A comparative illustration of the derived D1-FRIL amino acid sequence with the reported amino acid sequence of the mannose lectin as determined by Gowda et al. *(J. Biol. Chem*. 269:18789-18793,1994) is shown in Fig. 2. The single sequence derived for D1-FRIL protein comprises domains that correspond directly and with substantial homology to the a subunit (SEQ ID NO: _) and β subunit (SEQ ID NO: _) of the protein described by Gowda et al., *supra.* When the β subunit of the Gowda et al. *(supra)* protein is assigned to the N-terminal domain and is followed linearly by the a subunit, the arrangement of the polypeptides shows homology to other legume lectins. The derived Dl-FRIL amino acid sequence, however, comprises an additional of seven amino acid residues (aa27-34) that does not occur in the amino acid sequence described Gowda et al., *supra.* Several other differences between the amino acid sequences of Dl-FRIL and the amino acid sequence described by Gowda et al., *supra,* are also readily discernible from Fig. 2.

### Site-Specific Mutagenesis

To establish functionality of homologs of the protein encoded by the Dl-FRIL cDNA, a mutation was made in the Dl-FRIL cDNA done. The domains of the derived protein and the pea lectin that include the mutation site are shown below:

Dl-FRIL . Y L N P D Y G . D P N Y I H I G I D V (SEQ ID NO: __)
Pea F Y . N A A W D P S N R D R H I G I D V (SEQ ID NO: __)

It is known that the asparagine residue (the highlighted "N") in the pea lectin is involved in binding to its saccharide ligand. The corresponding asparagine in Dl-FRIL (position 141 of the amino acid sequence of SEQ ID NO: 2) was mutated to aspartic acid ("D"). This mutation was designated "N141D" for convenience.

To introduce the mutation, recombinant PCR was performed (see, *e*.*g*., Higuchi, R., *PCR Protocols: A Guide to Methods and Applications*, Innis M.A., Gelfand D.H., Sninsky J.J., and White TJ, eds., Academic Press, San Diego, 1990). Two PCR reactions were carried out separately on the full length cDNA using two primers that contain the same mutation and produce two products with an overlapping region:

MutI CCATAATCGGGATCAAGATAGGTG (SEQ ID NO: __)
MutII CACCTATCTTGATCCCGATTATGG (SEQ ID NO: __)

The primary PCR products were purified with the QIAquick PCR Purification kit (QIAGEN, Valencia, CA), according to the manufacturer's instructions. The overlapping primary products were then combined and amplified together in a single second reaction using flanking primers:

M1 Forw AACTCAGCCGCACAGTCATTGTCA (SEQ ID NO: __)
APEcoRI GAATTCGACCACGCGTATCGATGTCGAC (SEQ ID NO: __)

Both the primary and the secondary PCR reactions were performed in 100 µL containing 50 pmol of each primer, 0.4 mM deoxyribonucleotide and 1.0 unit Pfu polymerase (Stratagene) in the corresponding buffer. The primary PCR reaction amplified the two separate fragments in 30 cycles, each cycle comprising 40 seconds at 94°C, 40 seconds at 50°C, 60 seconds at 72°C, with an extension step at 72°C for 10 min. The second PCR reaction amplified the recombinant fragment in 12 cycles using the same conditions described above.

The resulting full-length fragment contained the mutation. The recombinant mutated product was cloned in the *Eco*RI site of the cloning vector pCR2.1, as illustrated schematically in Fig. 3, and sequenced as described above. This plasmid is referred to as "pCR2.1-DLA(D)."

### Construction of D1-FRIL-Expressing Plant Expression Vectors and Nicotiana tabacum Transformation

Recombinant PCR was used to modify the 5' ends of both the wild-type and the mutant Dl-FRIL clones, to introduce a signal peptide for entry of the protein into the endoplasmic reticulum. Following the procedure of Higuchi, *supra,* the sequence encoding the signal peptide and the full-length cDNA clones were amplified in two separate primary PCR reactions. The signal peptide sequence was obtained from the amplification of the binary vector pTA4, harboring the complete sequence of the α-amylase inhibitor gene (Hoffman et al., *Nucleic Acids Res*. 10:7819-7828,1982; Moreno et al., *Proc. Natl. Acad. Sci. USA* 86:7885-7889,1989).

The following primers were used for amplification of the signal peptide sequence:

Sigforw GAATTCATGGCTTCCTCCAAC (SEQ ID NO: __)
Sigrev TGACTGTGCGGCTGAGTTTGCGTGGGTG (SEQ ID NO: __)

The primers M1Forw (SEQ ID NO: _) and AP *Eco*RI (SEQ ID NO: _) used for amplification of the Dl-FRIL cDNA described above, were again used to amplify the Dl-FRIL cDNA.

The primers used for the secondary reactions were Sigforw and AP *Eco*RI, which were designed to generate EcoRI sites at the 5' and the 3' ends. Both the primary and the secondary PCR reactions were performed as discussed above for the site-directed mutagenesis.

The wild-type recombinant product SpDLA was cloned in the *Eco*RI site of the pBluescript SK+ cloning vector (Stratagene) to give the vector pBS-SpDLA, as shown in Fig. 4. The mutant SpDLA(D) was cloned in the same site of the cloning vector pCR2.1 to give the vector pCR2.1-SpM1, as shown in Fig. 5. The nucleotide sequence of each PCR product was determined as described above to verify the correct attachment of the signal peptide. The nucleotide sequence of SpDLA is defined by SEQ ID NO:22, and the derived amino acid sequence is defined by SEQ ID NO:23.

The sequences of SEQ ID NO: 22 and SEQ ID NO: 23 are as follows:
(xi) SEQUENCE DESCRIPTION: SEQ ID NO:22:

ATGGCTTCCT CCAACTTACT CACCCTAGCC CTCTTCCTTG TGCTTCTCAC CCACGCAAAC 60

TCAGCCGCAC AGTCATTGTC ATTTAGTTTC ACCAAGTTTG ATCCTAACCA AGAGGATCTT 120

ATCTTCCAAG GTCATGCCAC TTCTACAAAC AATGTCTTAC AAGTCACCAA GTTAGACAGT 180

GCAGGAAACC CTGTGAGTTC TAGTGCGGGA AGAGTGTTAT ATTCTGCACC ATTGCGCCTT 240

TGGGAAGACT CTGCGGTATT GACAAGCTTT GACACCATTA TCAACTTTGA AATCTCAACA 300

CCTTACACTT CTCGTATAGC TGATGGCTTG GCCTTCTTCA TTGCACCACC TGACTCTGTC 360

ATCAGTTATC ATGGTGGTTT TCTTGGACTC TTTCCCAACG CAAACACTCT CAACAACTCT 420

TCCACCTCTG AAAACCAAAC CACCACTAAG GCTGCATCAA GCAACGTTGT TGCTGTTGAA 480

TTTGACACCT ATCTTAATCC CGATTATGGT GATCCAAACT ACATACACAT CGGAATTGAC 540

GTCAACTCTA TTAGATCCAA GGTAACTGCT AAGTGGGACT GGCAAAATGG GAAAATAGCC 600

ACTGCACACA TTAGCTATAA CTCTGTCTCT AAAAGACTAT CTGTTACTAG TTATTATGCT 660

GGGAGTAAAC CTGCGACTCT CTCCTATGAT ATTGAGTTAC ATACAGTGCT TCCTGAATGG 720

GTCAGAGTAG GGTTATCTGC TTCAACTGGA CAAGATAAAG AAAGAAATAC CGTTCACTCA 780

TGGTCTTTCA CTTCAAGCTT GTGGACCAAT GTGGCGAAGA AGGAGAATGA AAACAAGTAT 840

ATTACAAGAG GCGTTCTGTG ATGATATATG TGTATCAATG ATTTTCTATG TTATAAGCAT 900

GTAATGTGCG ATGAGTCAAT AATCACAAGT ACAGTGTAGT ACTTGTATGT TGTTTGTGTA 960

AGAGTCAGTT TGCTTTTAAT AATAACAAGT GCAGTTAGTA CTTGT 1005

(xi) SEQUENCE DESCRIPTION: SEQ ID NO:23:

Met Ala Ser Ser Asn Leu Leu Thr Leu Ala Leu Phe Leu Val Leu Leu
                5                   10                  15
Thr His Ala Asn Ser Ala Ala Gln Ser Leu Ser Phe Ser Phe Thr Lys
            20                  25                  30
Phe Asp Pro Asn Gln Glu Asp Leu Ile Phe Gln Gly His Ala Thr Ser
        35                  40                  45
Thr Asn Asn Val Leu Gln Val Thr Lys Leu Asp Ser Ala Gly Asn Pro
    50                  55                  60
Val Ser Ser Ser Ala Gly Arg Val Leu Tyr Ser Ala Pro Leu Arg Leu
65                  70                  75                  80
Trp Glu Asp Ser Ala Val Leu Thr Ser Phe Asp Thr Ile Ile Asn Phe
                85                  90                  95
Glu Ile Ser Thr Pro Tyr Thr Ser Arg Ile Ala Asp Gly Leu Ala Phe
            100                 105                 110
Phe Ile Ala Pro Pro Asp Ser Val Ile Ser Tyr His Gly Gly Phe Leu
        115                 120                 125
Gly Leu Phe Pro Asn Ala Asn Thr Leu Asn Asn Ser Ser Thr Ser Glu
    130                 135                 140
Asn Gln Thr Thr Thr Lys Ala Ala Ser Ser Asn Val Val Ala Val Glu
145                 150                 155                 160
Phe Asp Thr Tyr Leu Asn Pro Asp Tyr Gly Asp Pro Asn Tyr Ile His
                165                 170                 175
Ile Gly Ile Asp Val Asn Ser Ile Arg Ser Lys Val Thr Ala Lys Trp
            180                 185                 190
Asp Trp Gln Asn Gly Lys Ile Ala Thr Ala His Ile Ser Tyr Asn Ser
        195                 200                 205
Val Ser Lys Arg Leu Ser Val Thr Ser Tyr Tyr Ala Gly Ser Lys Pro
    210                 215                 220
Ala Thr Leu Ser Tyr Asp Ile Glu Leu His Thr Val Leu Pro Glu Trp
225                 230                 235                 240
Val Arg Val Gly Leu Ser Ala Ser Thr Gly Gln Asp Lys Glu Arg Asn
                245                 250                 255
Thr Val His Ser Trp Ser Phe Thr Ser Ser Leu Trp Thr Asn Val Ala
            260                 265                 270
Lys Lys Glu Asn Glu Asn Lys Tyr Ile Thr Arg Gly Val Leu
        275                 280                 285

### A Plant Expression Vector Encoding Recombinant Dl-FRIL

A binary vector was constructed for seed-specific expression of Dl-FRIL. For seed expression, the vicilin promoter obtained from the pCW66 (Higgins et al., *Plant Mol. Biol*. 11:683-695, 1988) was cloned in *Eco*RI/*Kpn*I sites of the plant expression vector pBIN19, to form pBINVicPro, as illustrated in Fig. 6. Downstream of the vicilin promoter, the SpDLA cDNA sequence was ligated into the *Eco*RI/*Sac*I site, giving rise to the pBINVicPro-SpDLA, which is illustrated in Fig. 7. The mutated cDNA done SpDLA(D) was ligated in EcoRI site of the pBINVicPro vector to yield pBINVicPro-SpDLA(D), which is illustrated in Fig. 8. No additional termination sequences were added, relying instead on the stop codons and the polyadenylation site of the DLA and DLA(D) cDNA clones. Both vectors were transferred into *Agrobacterium tumefaciens* strain LBA4404 according to the freeze-thaw procedure reported by An et al., "Binary vectors," in *Plant Molecular Biology Manual,* Vol. A3, Gelvin SB, Schilperoort RA, and Verma DPS, eds., Kluwer Academic Publisher, Dordrecht, The Netherlands, pp. 1-19,1988).

*Agrobacterium-mediated* transformation of *Nicotiana tabacum* leaf disks was carried out and assayed as described (Horsch et al., *Science* 227:1229-1231,1985) using LBA4404 harboring the seed-specific expression vector pBINVicPro-SpDLA (Figure 9). Kanamycin-resistant plants (resistance being conferred by transformation with the pBIN19-based vectors that carry the gene) were scored for their ability to form roots in two consecutive steps of propagation in Murashige-Skoog medium containing 3% of sucrose and kanamycin sulfate (Sigma, St. Louis, MO) at 100 mg/mL.

Recombinant D1-FRIL fusion proteins were cleaved by the transformed plant cells *in vivo*. Thus, the transformed plant cells produced mature Dl-FRIL which, when purified, had a molecular mass of 60 kDa and comprised four subunits, two alpha subunits and two beta subunits *(i.e.,* an α₂β₂ heterodimer), where each subunit is about 15-18 kDa.

### Expression of Recombinant Dl-FRIL in E. coli

The Dl-FRIL wild-type cDNA and mutant clones (without signal peptides), were ligated into the *Eco*RI/*Sal*I and *Eco*RI/*Xho*I of the expression vector pGEX 4T-1 (Pharmacia Biotech, Uppsala, Sweden), to form the expression constructs pGEX-M1 and pGEX M1(D), respectively illustrated in Figs. 9 and 10. The host *E. coli* strain, BL21(D3), was purchased from Novagen (Madison, WI, and transformed with the above construct using the calcium chloride method (see Sambrook et al., *supra;* Gelvin and Schilperoort, *Plant Molecular Biology Manual,* Kluwer Academic Publishers, Dordrecht, The Netherlands, 1988; Altabella et al., *Plant Physiol* 93:805-810, 1990; and Pueyo et al., *Planta* 196:586-596,1995). The induction of the tac promoter (Ptac) was achieved by adding IPTG (isopropyl-β-D-thiogalactopyranoside) (Sigma) at a 1.0 mM final concentration when the cells reached an optical density of 0.4 - 0.6 at 600 nm. The cultures were allowed to grow for 12 hours at 37°C after the addition of IPTG. Control non-induced cultures were maintained under similar conditions. The cells were lysed by treatment with 4 mg/mL lysozyme in phosphate-buffered saline containing 1% TRITON® X-100.

Total cellular protein was extracted from transformed *E. coli* cells and analyzed on SDS-PAGE on a 15% gel using a standard procedure (Sambrook et al., *supra).* The cells from 1 mL of *E. coli* culture were suspended in the same volume of loading buffer (50 mM Tris HCl pH 6.8, 100 mM DTT, 2% SDS, 10% glycerol, 0.1% bromophenol blue) and vortexed. Following transfer to a nitrocellulose membrane, protein was stained with Coomassie Brilliant Blue R250. A representative separation is shown in Fig. 11, with the lanes identified in Table 1, below.

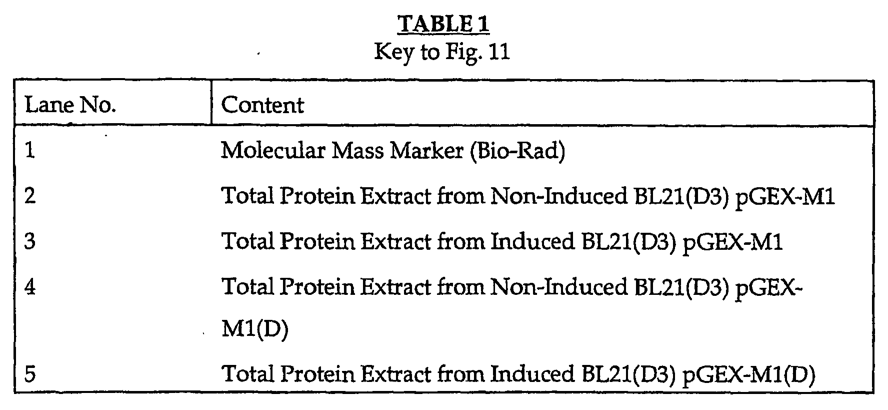

The separation of proteins in Fig.11 shows that the induced cells (lanes 3, 5) both produced an abundant polypeptide having a molecular mass of about 60 kDa (indicated by arrow). The non-induced cells failed to produce any significant amount of this protein (Fig. 11, lanes 2,4).

### Purification of Recombinant D1-FRIL from Transformed E. coli

Induced *E. coli* cells (200 mL) as described above were harvested after 12 hour induction at 37°C by centrifugation at 5000 g for 10 min. The pellet was washed with 50 mM Tris-HCl pH 8.0,2 mM EDTA, and resuspended in 1/10 vol of 1% TRITON surfactant in TBS (20 mM Tris pH 7.5, 500 mM NaCl). The cells were lysed by adding 4 mg/mL of lysozyme and incubating at room temperature for 30-60 min. After centrifugation at 5000 g, the supernatant containing the total soluble proteins was discarded and the resulting pellet, comprising the inclusion bodies and containing the accumulated the recombinant fusion protein, was extracted with 8 M guanidine-HCl (Martson and Hartley, "Solubilization of Protein Aggregates" in *Guide to Protein Purification* vol.182, eds. Deutscher , M.P., pp 266-267,1993).

The recombinant fusion protein solubilized by guanidine-HCl was purified on GST-Sepharose beads (Pharmacia Biotech, Uppsala, Sweden) according the manufacturer's instructions and eluted in 1 mL of reduced glutathione (Sigma). Samples of the purified fusion proteins were cleaved with thrombin (Novagen) using 5 cleavage units /mL purified fusion protein.

For immunoblot analysis (Western blot), the purified proteins were separated by SDS-PAGE in general accordance with the method described above. The gel was equilibrated in transfer buffer (25 mM Tris pH 8.3,192 mM Glycine, 20% MeOH) and blotted onto nitrocellulose (Bio-Rad, Hercules, CA) for 1 hour at 100 V using a Bio-Rad electrotransfer apparatus. Non-specific binding was blocked by incubating the blots for at least 1 hour in 1X TBS (20 mM Tris pH 7.5, 500 mM NaCl) containing 3% gelatin. Blotting was followed by incubation with a primary antibody (a polyclonal rabbit serum raised against the N-terminal peptide of the β-subunit of *Phaseolus vulgaris* FRIL *(i.e.,* Pv-FRIL), 1:100 dilution, 3 hour; described below in Example 5), followed by incubation with a secondary antibody (goat anti-rabbit IgG conjugated to horseradish peroxidase at 1:1000 dilution for 1 hour). The blots were washed and the color developed with the color development reagent (Bio-Rad). A representative result is shown in Fig. 12, with the lanes identified in Table 2, below.

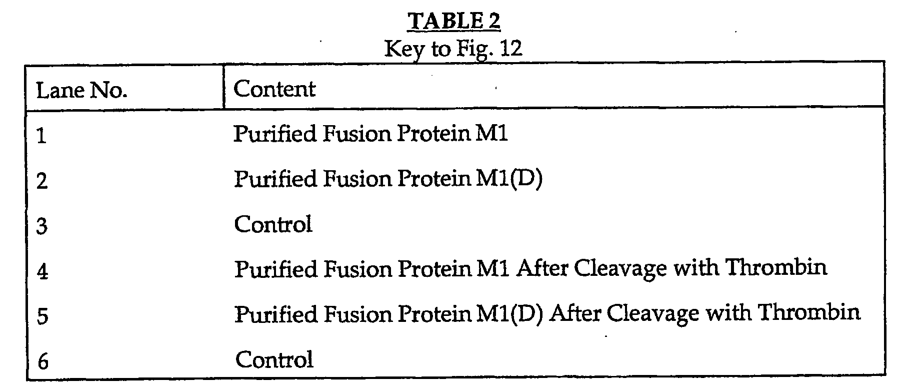

The separation shown in Fig. 12 demonstrates that the two forms of fusion protein have similar molecular masses of about 60 kDa, and that thrombin cleaved both types of fusion protein to produce a new polypeptide of molecular mass 30 kDa.

### EXAMPLE 2

### Recombinant D1-FRIL Specifically Stimulates

### Proliferation of 3T3 Cells Expressing the FLT3 Receptor

D1-FRIL interacts with the mammalian FLK2/FLT3 tyrosine kinase receptor. A specific and quantitative biological assay using NIH 3T3 fibroblasts transfected either with a chimeric receptor having the extracellular portion of the murine FLT3 receptor combined with the intracellular portion of the human Fms receptor (Dosil et al., *Mol. Cell. Biol*. 13(10):6572-65851993) or with the full length human receptor (Small et al., *Proc. Natl. Acad. Sci. USA* 91:459-463, 1994) can be used to evaluate lectin biological activity during purification. Serial two-fold dilutions of lectin samples across rows of a 96 well plate allowed for greater than a thousand-fold range to access FLT3 3T3 biological activity. Either the murine or human FLT3 ligand (FL) (Lyman et al., *Cell* 75:1157-1167,1993; Hannum et al., Nature 368: 643-648,1994) or the FRIL was found to rescue FLT3-transfected cells from death in this assay.

Specifically, 3T3 cells cultured in tissue culture plates (Becton Dickinson Labware, Lincoln Park, NJ) were removed from the plates by washing cells twice in Hank's buffered saline solution (HBSS; Gibco Laboratories, Grand Island, NY). Non-enzymatic cell dissociation buffer (Gibco) was added for 15 minutes at room temperature. The resulting cells wee washed in medium. FLT3 3T3 cells were cultured at a final concentration of 3,000 cells per well in a volume of 100 µL of serum-defined medium containing 10 mg/mL rhIL1-α, 10% AIMV (Gibco, Grand Island, NY) and 90% Dulbecco's modification of Eagle's medium (DMEM; Gibco) in 96 well plates. Under these assay conditions, cells die after two to four days of culture in a humidified incubator at 37°C and 5% CO₂ unless exogenously added ligand rescues cells from death. Each 96 well plate contained wells of cells containing calf serum, which stimulates all 3T3 cells, as a positive controls and wells of cells containing medium only as a negative control ("blank"). Full-length Fms-transfected 3T3 cells (biological response shown in Tessler et al., *J. Biol. Chem.,* 269:12456-12461,1994) served as receptor-transfected control target cells, and parent 3T3 cells served as untransfected control cells. Proliferation and cell survival was quantitated by addition of XTT (2,3-bis[Methoxy-4-nitro-5sulfophenyl]-2H-tetrazolium-5 carboxanilide inner salt) (Diagnostic Chemicals Ltd, Charlottetown, Prince Edward Island, Canada), which is a tetraformazan salt cleaved by actively respiring cells (Roehm et al., *J. Immunol. Methods* 142: 257-265,1991). Proliferation and cell survival was quantitated spectrophotometrically using a Vmax kinetic plate reader (Molecular Devices Corp., Mountain View, CA), and recorded as either relative activity (units/mL) or as specific activity (units/mg). One unit of biological activity was defined as the reciprocal dilution at which half-maximal stimulation of cells is detected.

The crude protein extract from the *E. coli* cultures described in Example 1, above, was tested to determine whether expressed recombinant Dl-FRIL possessed any capacity to stimulate FLT3 3T3 cells using this assay. The data from this experiment are summarized in Figs. 13A and 13B. Specifically, Fig. 13A is a graph showing that the crude extract of the *E. coli* culture containing expressed Dl-FRIL specifically stimulates hFLT3 cells; Figure 13B is a graph showing that the same extract does not stimulate untransfected 3T3 cells. In Figs. 13A and 13B, medium control is represented by a solid line. The ordinate (absorbance) indicates cell viability measured by XTT at three days; the abscissa shows the reciprocal dilution of the extract sample. The apparent inhibition of proliferation observed at higher concentrations (Fig.13A) is not understood, but may relate to toxic components in the crude *E. coli* extract or the consequences of dose-related preservation of the 3T3 fibroblasts.

### EXAMPLE 3

### Recombinant D1-FRIL Preserves Mononuclear Cells and Progenitors in Liquid Culture

The recombinant D1-FRIL protein preserved functional progenitors for at least four weeks in liquid culture. Figs. 14A and 14B, and Table 3 illustrate the results of experiments in which recombinant D1-FRIL was shown to act in a dose-responsive manner to preserve human cord blood progenitors.

To do this, umbilical cord blood from healthy donors was collected in 100 units/ml of heparin. Cord blood mononuclear cells (CB mnc) were isolated within 4 hours of collection by FICOLL-PAQUE® separation (Pharmacia Biotech, Piscataway, NJ) following manufacturer's instruction and washed in X-VIVO 10 medium (BioWhittaker, Walkersville, MD). CB mnc were then cultured in six well tissue culture plates (Corning Inc., Corning, NY) at a concentration of 200,000 cells/mL in a volume of 4 mL of X-VIVO 10 *(i.e.,* 800,000 cells total per well). Dl-FRIL and/or recombinant *E. coli* Flt3-L (recFL; BioSource International, Camarillo, CA) were added at a concentration of 40 ng/ml at the outset (with no addition as a control). Cultures were incubated in humidified chambers without medium changes for up to 29 days.

After incubation, the cultured CB mnc cells were harvested by washing in X-VIVO 10 (*i.e.*, harvested cells were pelleted and resuspended in X-VIVO 10) to remove the Dl-FRIL and/or recFL, and then determining viable cell number by trypan blue (Sigma) exclusion. These results are shown in Fig. 14A. The progenitor number and capacity of harvested cells were assessed by plating the washed cells in triplicate in fetal bovine serum-free, methylcellulose colony assay medium containing IL-2, granulocyte-macrophage CSF, and kit ligand (StemCell Technologies, Vancouver, BC, Canada). After two weeks, the resultant colonies from each of the triplicate wells were scored and the results are shown in Fig. 14B. Thus, Figs. 14A and 14B show that recombinant D1-FRIL preserved cord blood mononuclear cells and progenitors in a dose-responsive manner in liquid culture.

Table 3 shows the resulting colonies after 15, 21, or 29 days of incubation, demonstrating that D1-FRIL, but not recFL, preserved progenitors in suspension culture.

**TABLE 3**

| Day | Medium | Myeloid* | Erythroid* | Mix* | Blast* |
|---|---|---|---|---|---|
| 15 | Dl-FRIL | 1,033 ± 12 | 67 ± 12 | 7 ± 12 | 0 |
| | recFL | 40 ± 69 | 0 | 0 | 0 |
| | Dl-FRIL + recFL | 933 ± 250 | 167 ± 95 | 0 | 0 |
| | No Addition | 0 | 0 | 0 | 0 |
| 21 | Dl-FRIL | 387 ± 83 | 7 ± 12 | 0 | 167 ± 64 |
| | recFL | 0 | 0 | 0 | 0 |
| | Dl-FRIL + recFL | 473 ± 133 | 53 ± 42 | 0 | 300 ± 34 |
| | No Addition | 0 | 0 | 0 | 0 |
| 29 | D1-FRIL | 0 | 0 | 0 | 80 ± 72 |
| | recFL | 0 | 0 | 0 | 0 |
| | D1-FRIL + recFL | 0 | 0 | 0 | 40 ± 20 |
| | No Addition | 0 | 0 | 0 | 0 |

| | | | | | |
|---|---|---|---|---|---|
| *Data reported as ± SD of the three values from the triplicate methylcellulose colony assay. The reported experiment is a representative of four experiments. | | | | | |

In Figs. 14A and 14B and in Table 3, "blast" refers to colonies consisting of primitive, morphologically undifferentiated cells; "mix" refers to colonies consisting of myeloid and erythroid cells; "erythroid" refers to colonies consisting of erythroid cells; and "myeloid" refers to colonies consisting of myeloid cells. In Figs. 14A and 14B, cell number (Fig. 14A) or colony number (Fig. 14B) is shown on the ordinate; the abscissa shows the reciprocal dilution of the sample.

As shown in Table 3, colonies derived from mature myeloid and erythroid progenitors formed from cells cultured for 15 days in either FRIL or FRIL plus recFL; 24-fold fewer mature colonies formed from cells cultured in recFL alone; and no colonies appeared if neither was present. After 21 days in culture, Table 3 shows that myeloid and erythroid colonies formed only from cells exposed to Dl-FRIL. The frequency of myeloid colonies in D1-FRIL-only cultures (based on the initial number of CB mnc) decreased by 2.7 fold from 1 in 774 after 2 weeks in culture to 1 in 2,067 after 3 weeks; erythroid colonies decreased in frequency by 9.6 fold from 1 in 11,940 to 1 in 114,287 (see Table 3).

The colonies from this assay were photographed at various time points. As shown in Fig. 15A, in addition to myeloid and erythroid colonies, day 21 cultures contained small colonies consisting of undifferentiated cells. Fig. 15B shows that only blast-like colonies were observed when the cells were cultured in D1-FRIL for 29 days (see also Table 3).

The progenitor capacity of the blast-like colonies was examined further for cells initially cultured for 3 weeks in either D1-FRIL, recFL, no addition, or D1-FRIL + recFL, and then without these regulators for an additional 6 weeks in a methylcellulose colony assay. No colonies were detected from the cells cultured for 21 days in either recFL alone or medium control. Viable cells were harvested from the cells initially cultured in Dl-FRIL alone and replated in an colony assay (in methylcellulose colony assay medium) for an additional 4 weeks. A schematic diagram of this experiment is shown in Fig.16. As schematically diagramed in Fig. 16, the frequency of blast-like colonies cultured in Dl-FRIL alone decreased by 2.1 fold from day 21 to day 29, from 1 in 4,790 to 1 in 10,000, and 7.5 fold in Dl-FRIL + recFL cultures from 1 in 2,667 to 1 in 20,000 of the initial CB mnc cells cultures. Following the protocol schematically diagrammed in Fig.16, small, diffuse, blast-like colonies were detected at a frequency of 1 in 67 (900 colonies/600,000 CB mnc) exclusively in dishes of cells initially cultured in Dl-FRIL alone, and at a frequency of 1 in 132 (990 colonies/131,000 CB mnc) for cells cultured in Dl-FRIL + recFL (see examplary colonies in Fig.17).

### EXAMPLE 4

### Recombinant Dl-FRIL Acts Directly on Progenitor Cells

To assess whether recombinant Dl-FRIL acts directly or indirectly through accessory cells to preserve progenitor cells, cord blood mononuclear cells were first enriched for progenitors expressing the CD34 antigen by immunomagnetic bead isolation (Dynal Corp., Lake Success, NY). Five hundred CD34⁺ cells were placed into wells containing 100 µL of serum-free medium (BIT9500, StemCell Technologies, Vancouver, BC, Canada) either in the presence of recFL (PeproTech, Princeton, NJ) or a cytokine cocktail of recombinant human interleukin 3 (rhIL3) + recombinant human interleukin 6 (rhIL6) + recombinant human interleukin 11 (rhIL11) + rhTpo Thrombopoietin + FL (FLT3-Ligand) (BioSource International, Camarillo, CA) in 96-well plates and cultured for four weeks without medium changes. The numbers of functional progenitors from these cultures were assessed by plating cells in complete serum-free methylcellulose colony assay medium (StemCell Technologies). After two weeks, the resultant colonies were scored and the results are shown in Fig. 18 (solid bars = recombinant Dl-FRIL; open bars = cytokine cocktail). Clearly, progenitors were preserved only in the recombinant Dl-FRIL-containing cultures (Fig. 18). Thus, purified recombinant Dl-FRIL acts directly on primitive hematopoietic progenitors.

### EXAMPLE 5

### Identification and Cloning of Pv-FRIL, a second FRIL family member

### FRIL activity in PHA-LCM media

A biological screening assay to search for novel stimulators of the Flt3 receptor was developed using NIH 3T3 cells transfected with expression vectors containing cDNA of murine and human Flt3 and the related Fms receptor. The mFlt3/Fms 3T3 cell line is a 3T3 cell line transfected with nucleic acid encoding a fusion protein consisting of the murine extracellular domain of the Flt3 receptor fused to the transmembrane and intracellular domains of the human Fms (provided by Dr. Ihor Lemischka, Princeton University, Princeton, NJ). The Stk 3T3 cell line is a 3T3 cell line transfected with the full-length human Flt3 receptor (provided by Dr. Donald Small, Johns Hopkins University, Baltimore, MD). The human FMS 3T3 cell line is a 3T3 cell line transfected with the full-length human Fms receptor (provided by Dr. Charles Sherr, Saint Jude Children's Research Hospital, Memphis, TN. Parent 3T3 cells were purchased from American Type Culture Collection ("ATCC"; Manassas, VA). Receptor-transfected cells contained Neo resistance genes and were maintained in medium containing 750 g/ml of G418 (Life Technologies, Rockville, MD).

To create factor-dependence for receptor-transfected 3T3 cells, growth conditions were compromised to permit only ligands to rescue cells from death. To do this, 3T3 cell lines were assayed in 96 well plates (Becton Dickinson Labware, Lincoln Park, NJ) containing 3,000 cells in 100 µL of serum-free medium consisting of 10% AIMV (Life Technologies) and 90% DMEM. In each experiment, samples were serially diluted two-fold across rows starting at a 1:10 dilution. Viable cells were quantitated after 3-5 days by XTT (2,3-bis[Methoxy-4-nitro-5sulfophenyl]-2H-tetrazolium-5 carboxanilide inner salt) (Sigma, St. Louis, MO) (Roehm et al., *supra).* Relative activity (units/ml) and specific activity (units/mg) are defined as the reciprocal dilution at which half-maximal stimulation of cells was detected.

The FMS 3T3 cells (transfected with cDNA encoding the human Fms tyrosine kinase receptor) and its ligand, human M-CSF, served as a model system. Recombinant human M-CSF stock of 1 µg/mL was serially diluted (where the first dilution, 1:20, was 50 ng/mL) was used to stimulate Fms3T3 cells. As shown in Fig. 19A, Fms 3T3 cells responded to M-CSF in a dose-responsive manner. Neither the mFlt3/Fms 3T3 cells nor the parent untransfected 3T3 cells responded to M-CSF (Fig.19A).

Various sources of conditioned medium were screened for the presence of Flt3 3T3 stimulatory activity. The most potent source was conditioned medium harvested from human peripheral blood cells activated to secrete high levels and a broad range of cytokines by the mitogenic legume lectin, phytohemagglutinin (PHA), which is derived from impure red kidney bean extracts.. This source, commonly called PHA leukocyte-conditioned medium (PHA-LCM), has been used as a standard positive control in hematopoietic colony assays for over two decades (Sharon and Lis, *Science* 246: 227,1989). To make PHA-LCM, leukopherized blood from normal volunteers was purchased from North American Biologicals Inc., Miami, FL. Mononuclear cells were isolated by FICOLL-PAQUE®, washed in AIMV, and cultured at a concentration of 2 x 10⁶ cells/ml in AIMV containing a 1% volume of crude red kidney bean extract containing PHA from Life Technologies (catalog number 10576-015) in either T150 flasks (Becton Dickinson Labware, Lincoln Park, NJ) or roller bottles (Becton Dickinson Labware) for one week. Cells and debris were removed by centrifugation and conditioned medium was stored at -20°C.

PHA-LCM induced proliferation of mFlt3/Fms 3T3 cells and Stk 3T3 (expressing the human Flt3 receptor) in an indistinguishable manner at approximately 200 units/mL (Fig. 19B). Untransfected 3T3 cells did not respond to PHA-LCM (Figure 19B) and Fms 3T3 cells responded weakly (data not shown).

### Purification of Pv-FRIL from PHA-stimulated Leukocyte Conditioned Media

Each batch of PHA-LCM was generated in serum-free medium with cells from individual normal donors. To start purification, the PHA-LCM with Plt3 3T3 activity was pooled in 30 liter lots with approximately 10⁷ Flt3 3T3 units. Twenty-five liters of PHA-LCM was diafiltered into 50 mM Tris-HCl, pH 7.6, 50 mM NaCl and then concentrated to 2-2.5 liters by tangential flow ultrafiltration on a 10 kDa molecular weight cutoff membrane (Pellicon, Millipore, Bedford, MA). A Blue-sepharose FF (Pharmacia Biotech) column (10 cm x 15 cm) was equilibrated with 50 mM Tris-HCl, pH 7.4, 50 mM NaCl. To remove the human serum albumin, concentrated PHA-LCM was applied to the column at 25 ml/min by pump and the flow through was collected. The flow-through fraction from Blue-sepharose was subjected to anion exchange chromatography by being applied to a Q-sepharose FF (Pharmacia Biotech) column (5 cm x 5 cm) equilibrated with 10 mM Tris-HCl, pH 7.6. The column was washed with equilibration buffer and then eluted at 12 ml/min with a continuous gradient of 0-0.7 M NaCl in 10 mM Tris. Fractions were collected (6 ml/fraction) and an aliquot of each fraction was tested for Flt3 3T3 activity (*i.e.*, an ability to stimulate mFlt3/fms 3T3 cells and/or Stk 3T3 cells) in 96 well plates.

After validation that a fraction had Flt3 T3 activity, a phenyl-sepharose HP (Pharmada Biotech) column (1.6 cm x 10 cm) was equilibrated with 20 mM phosphate, pH 7,1.5 M NH₄SO₄. The pooled sample from Q-sepharose was adjusted to 1.5 M NH₄SO₄ and applied to the phenyl-sepharose column. The column was washed with equilibration buffer and eluted at 1 ml/min with a gradient of 1.5-0.1 M NH₄SO₄ in 20 mM phosphate, pH 7. Fractions (1 ml) were collected and tested for Flt3 3T3 activity. Fractions with Flt3 3T3 activity were pooled and dialyzed against 50 mM Tris-HCl, pH 7.2, 100 mM NaCl and concentrated by vacuum centrifugation.

A Superdex 75 (Pharmacia Biotech) column (1.6 cm x 60 cm) was equilibrated with 50 mM Tris-HCl, pH 7.4, 100 mM NaCl. The pooled sample from phenyl-sepharose was applied to the Superdex 75 column and eluted at 0.6 ml/min. Fractions (1.8 ml) were collected and tested for Flt3 3T3 activity. The active fractions were dialyzed against Tris-HCl, pH 7.2 and concentrated by vacuum centrifugation.

A C4 reverse-phase column (4.6 mm x 100 mm, Vydac, Hesperia, CA) was equilibrated with 0.1% trifluoroacetic acid (TFA) in HPLC grade H₂O. Pooled and concentrated sample from Superdex 75 chromatography was applied to the C4 column and the column was eluted with a gradient of 10-55% acetonitrile, 0.1% TFA over 70 min at 0.5 ml/min. Fractions of 0.5 mL were collected and evaporated by vacuum centrifugation.

Analysis by ELISA for cytokines (IL1-α, IL1-β, IL2, IL3, IL4, IL6, GM-CSF, G-CSF and SCF) during purification of Pv-FRIL were performed using kits purchased from R&D Systems (Minneapolis, MN).

### Pv-FRIL Specific Rabbit Anti-Serum

Throughout purification of Pv-FRIL a New Zealand White rabbit (HRP, Denver, PA) was immunized with crude PHA-LCM, boosted with increasingly purified samples containing Flt3 3T3 activity, and finally immunized with a peptide corresponding to Pv-FRIL (AQSLSF[N, C, S]FTKFDLD), referred to as the AQS-peptide. Samples were glutaraldehyde conjugated to keyhole limpet hemocyanin (KLH, Sigma). The rabbit was immunized with KLH-AQS peptide-containing samples using either Complete Freund's Adjuvant (Sigma) or Hunter's Titermax (Vaxcel, Inc., Norcross, GA). Antiserum demonstrated a 1:5,000 titer to the AQS peptide in an ELISA (data not shown). Since the antiserum contained reactivities to other proteins, further enrichment for AQS peptide-specific antibodies was achieved by either depletion of cross-reactive antibodies or by affinity purification using a AQS peptide covalently linked to an agarose support (AminoLink coupling gel, Pierce).

An anti-AQS affinity column was prepared either by purifying IgG from high titer rabbit antiserum by protein A affinity chromatography (ImmunoPure kit, Pierce) or antibody isolated from the AQS peptide column and then covalently linking antibody to an activated agarose support (AminoLink coupling gel, Pierce).

### Activity of Purified Pv-FRIL

To relate the observations of Pv-FRIL's activity on receptor-transfected 3T3 cells to Flt3 receptor-expressing hematopoietic progenitors, a suspension culture of human cord blood cells enriched for Flt3⁺ progenitors by CD34 immunomagnetic bead selection was adapted to a 96 well plate format. To do this, umbilical cord blood from healthy donors was collected in 100 units/ml of heparin (Fujisawa Healthcare, Deerfield, IL). Mononuclear cells were isolated by FICOLL-PAQUE® (Pharmacia Biotech, Piscataway, NJ), washed in HBSS, and resuspended in serum-defined medium, either AIMV or XVIVO-10 (BioWhittaker, Walkersville, MD). Mononuclear cells were enriched for Flt3⁺ progenitors by CD34 immunomagnetic bead selection (Dynal Corporation, Lake Success, NY). CD34⁺ cells were cultured in 6 well plates (Becton Dickinson Labware) in at a concentration of 10⁵ cells in 1 mL of DMEM containing 10 ng/mL recombinant human IL3 (BioSource International, Camarillo, CA) and 10% fetal calf serum. The number of refractive cells present in culture wells was scored microscopically.

Culture medium always contained IL-3 since early-acting cytokines require additional co-factor(s) for survival and proliferation. Fig. 20A shows that cord blood cells responded to column fractions in two regions of the material eluted from an anion exchange column. The first region of activity corresponded with Flt3 3T3 stimulatory activity (Figs. 20B and 20C); the second associated with an activity detected with Fms 3T3 cells (Fig. 20D); no response was detected in untransfected 3T3 cells (data not shown). The active material, corresponding to Flt3 3T3 activity (peak one in Fig. 20A), was further characterized and purified on different chromatographic matrices including a cation exchange resin, heparin sepharose, hydroxyappatite, ConA-sepharose, phenyl sepharose, and gel filtration (Superdex 75) (data not shown).

The further purified Pv-FRIL was used in the Flt3 3T3 assay described above. Plateau stimulation of Flt3 3T3 cells decreased with sequential purification steps (see Fig. 21A), suggesting removal of essential co-factor(s). Addition of suboptimal levels of crude PHA-LCM to Pv-FRIL obtained in the later stages of purification, restored activity of this partially purified Pv-FRIL to maximal plateau levels (Fig. 21B).

Analysis by ELISA for cytokines that act on hematopoietic progenitors (interleukin 1-α (IL1-α), interleukin 1-β (IL1-β), interleukin 2 (IL2), interleukin 3 (IL3), interleukin 4 (IL4), interleukin 6 (IL6), granulocyte-macrophage colony stimulating factor (GM-CSF), G-CSF (granulocyte colony stimulating factor), and stem cell factor (SCF)) in fractions containing Pv-FRIL purified to near homogeneity revealed that IL1-α had remained with Pv-FRIL through every step of purification (data not shown).

Flt3 3T3 activity was depleted but not eliminated either by adding neutralizing antibodies to IL1 or by removing IL1 by antibody affinity chromatography (data not shown). However, at the levels (<1 ng/mL) found in fractions containing purified Pv-FRIL, exogenous recombinant hIL1- a by itself had no stimulatory activity (data not shown). This observation suggested the possibility that IL1-α may act as a necessary co-factor to obtain maximal stimulation by Pv-FRIL. In subsequent experiments when testing purified Pv-FRIL, the co-factor requirement was met by the addition of either IL1- a or PHA-LCM added at a concentration that did not stimulate Flt3 3T3 cells by itself.

Using this modified Flt3 3T3 assay with the addition of sub-stimulatory amounts of either IL1- a or PHA-LCM, the active protein was purified to near homogeneity in three independent experiments. Table 4 summarizes the results of one such experiment in which a 1% recovery was accompanied by an 80,000-fold purification and resulted in a fraction with a specific activity of 244,500 units/mg.

**TABLE 4**

| Purification Step | Total Protein (mg) | Total Activity (units) | Specific Activity (u/mg) | Fold Purification | Recovery (%) |
|---|---|---|---|---|---|
| PHA-LCM | 231,774 | 7,500,000 | 3 | 1 | 100 |
| Blue Sepharose FF | 1,294 | 2,600,000 | | 670 | 35 |
| Q-Sepharose FF | 347 | 1,400,000 | 4,035 | 1,345 | 19 |
| Phenyl-Sepharose HP | 14 | 1,200,000 | 85,714 | 28,571 | 16 |
| Superdex 75 | 0.12 | 29,340 | 244,500 | 81,500 | 1 |
| Pv-FRIL was purified by its ability to stimulate Flt3-expressing 3T3 cells using four different chromatographic media. This resulted in a 80,000-fold purification with a 1% yield. | | | | | |

### Purified Pv-FRIL

SDS-PAGE showed the purified material to contain a limited number of polypeptides and the molecular size of the active material was determined by eluting the protein from SDS-PAGE gel slices run under non-reducing conditions and assaying the activity of the eluted material. FIt3 3T3 activity was always found in a gel slice that contained 14-22 kDa polypeptides and sometimes in a gel slice containing 32-43 kDa polypeptides (data not shown).

The polypeptide(s) in the active fraction corresponding to the 14-22 kDa range were subjected to aminoterminal sequencing by Edman degradation. To do this, an 18 kDa species from C4 reverse-phase chromatography was resolved by SDS-PAGE, electroblotted onto polyvinylidene difluoride (PVDF) membrane (Immobilon-P, Millipore) and stained with Ponceau S (Bio-Rad Laboratories, Inc., Hercules, CA). The 18 kDa band was cut from the PVDF membrane and N-terminal sequence was determined by automated Edman degradation on an ABI model 477A protein sequencer (PE Applied Biosystems, Foster, CA). The derived peptide sequences were compared against the SwissProt protein sequence database.

In each of three experiments, the sequence AQSLSFXFTKDALD was obtained from a polypeptide of 18 kDa (where X is an unknown amino acid). For the material at the dye front (14 kDa and below) the aminoterminal sequence of TDSRVVAVEFDXFP was found twice. The amino terminus of a smooth muscle protein (SM22-α) was found twice and the amino terminus of myoglobin identified once. Since the sequence starting with AQS was the only sequence identified in each experiment, this polypeptide was concluded to be responsible for Flt3 3T3 activity.

Further purification of Pv-FRIL was obtained by inununoaffinity chromatography using a rabbit antiserum described above that was generated against a synthetic peptide of 13 amino acids corresponding to the N-terminus obtained for the 18 kDa polypeptide (referred to as anti-AQS). Crude PHA-LCM was applied to a rabbit anti-AQS affinity column, and after washing, bound protein was eluted under acidic conditions. Four pools of fractions were assayed for activity in the two different assay systems. The Flt3 3T3 cells responded weakly (<100 u/mL) to the pooled fractions (data not shown). Figs. 22A-22D shows results of an experiment where the pooled fractions were assayed on cord blood cells in the presence of IL3. After two weeks of suspension culture, the number of viable cells and status of CD34 expression was evaluated. A representative of three AQS affinity chromatography experiments is shown in Figs. 22A-22D. Cell cultures supplemented with the two early column fraction pools contained approximately four-fold more cells (426,000 cells and 466,250 cells, respectively) than at the 100,000 cells seeded and no appreciable CD34 staining (Figs. 22A and 22B). The increase in cell number and loss of CD34 expression is attributed to the expected consequences of IL3-induced proliferation and differentiation. In contrast, cell cultures treated with the late-eluting fraction pool (Fig. 22D) contained less than 10,000 cells, or a tenth of input cells, and a uniform population of cells expressing CD34. The late-eluting AQS affinity pool did not show the potent effects of IL3 (high cell numbers and exhausted medium); instead the persistence of viable cells expression CD34 after two weeks in suspension culture suggested that the active component might preserve CD34⁺Flt3⁺ progenitors.

### Pv-FRIL Is Derived From Phaseolus vulgaris

Because PHA is derived form red kidney bean extract and because a FRIL family member, Dl-FRIL, was isolated from another legume, namely *Dolichos lab lab,* mannose-binding lectins were isolated from red kidney bean (*Phaseolus vulgaris*) extract using standard methods, such as the procedure of Rudiger, H., *Isolation of Plant Lectins*, H.-J. Gabius and S. Gabius, eds., pp. 31-46, Berlin, 1993). The kidney bean mannose-binding lectin consisted of polypeptides with molecular weights of 18 kDa and 15 kDa, and the aminotermini of these two polypeptides started with AQSLSFXFKFDPN AND TDSRWAVEDF, respectively (where X is an unknown amino acid).

Pv-FRIL isolated from *Phaseolus vulgaris* was tested for activity in the Flt3 3T3 cell assay. As shown in Fig. 23, Flt3 3T3 cells responded in a dose dependent manner to Pv-FRIL, while parent untransfected 3T3 cells did not.

### Purification of Pv-FRIL from Phaseolus vulgaris

Dry seeds from the red and white kidney beans *(Phaseolus vulgaris)* were purchased from W. Atlee Burpee & Company, Warminster, PA. Lectins were eluted using a standard protocol. Briefly, beans were pulverized in a home coffee grinder and added to buffer of 50 mM Tris/HCl, pH. 8.0, 1 nM each of MgCl₂ and CaCl₂ for 4 hours at 4°C with constant mixing. Bean solids were pelleted by centrifugation at 10,000 x g for 20 min. The pH of the supernatant was modified to pH 4.0 with acetic acid and constant mixing to remove contaminating storage proteins, followed by centrifugation to clarify the supernatant, and finally the pH was readjusted to 8.0 with sodium hydroxide before storing at -20°C.

Specific binding of Pv-FRIL to mannose enabled a single-step purification of the lectin from the supernatant of the bean extract. 50 µL extracts of either red or white kidney beans were incubated in a conical tube with 1 mL of mannose covalently bound to agarose beans (Sigma) at 4°C with constant mixing for 4 hours to overnight. Beans were washed gently by centrifugation (300 g, 5 min) in lectin binding buffer. Mannose binding protein was eluted from the mannose beans after washing by incubation either with 200 mM α-methyl mannoside (Sigma) or 100 mM glycine, pH 2.8.

### DNA Isolation and PCR amplification of Pv-FRIL-Encoding Nucleic Acid

Total genomic DNA was isolated from young *Phaseolus vulgaris* shoots according to the procedure of Dellaporta ("Plant DNA miniprep and microprep: Versions 2.1-2.3,".Freeling, M. and V. Walbot (Ed.). *The Maize Handbook. XXVI+759p.* Springer-Verlag New York, Inc.: New York, New York, USA; Berlin, Germany.), and stored at -20°C. Based on the determined N-terminal amino acid sequences of Pv-FRIL, four degenerate oligonucleotides (PVbeta1, PVbeta2, PValfa1, PValfa2) were designed using *Phaseolus vulgaris* codon usage. The sequences of the primers is as follows:

PVBeta1: TTY ACY AAR TTY GAY YTN GA
PVBeta2: ATY TTY CAR GGW GAY GC
PVAlfa1: TTR ACR TCR ATW CCR ATR TG
PVAlfa2: TAR TTW GGR TCR ATR TTR GCR TT

Two sequential polymerase chain reactions (PCR) were performed. In the first reaction, 10 ng of bean genomic DNA was amplified by 30 cycles of PCR, each cycle comprising 40 seconds at 94 °C, 40 seconds at 50 °C, 60 seconds at 72 °C, and an extension step at 72 °C for 10 min. The reactions were performed in 50 µl containing 30 pmol of each primer, PVbeta 1 and PValfa1, 0.2 mM deoxyribonucleotides and 0.5 unit of Ampli-Taq polymerase (Perkin Elmer) in the corresponding buffer. One microliter of the PCR product was amplified by 30 PCR cycles using the same conditions as described above. The reaction was performed in 50 µL containing 30 pmol of the two primers, PVbeta 2 and PV alfa2, using 0.2 mM deoxyribonucleotides and 0.5 unit of Ampli-Taq polymerase (Perkin Elmer) in the corresponding buffer. The 460 bp fragment obtained was cloned in a T/A plasmid, pCR2.1 (Invitrogen) and sequenced by sequenase dideoxy chain termination (United States Biochemicals).

### RNA Isolation and cDNA synthesis of Pv-FRIL-Encoding Nucleic Acid

Total RNA was prepared from mid-maturation *Phaseolus vulgaris* seeds stored at -70°C following the procedure reported by Pawloski *et al. (Mol. Plant Biol. Manual* 5:1-13, 1994). The 5'/3' RACEKIT (Boehringer Mannheim) was used to generate cDNA from 5.0 µg total RNA used according to the manufacturer's instructions. In the cDNA synthesis for the 3' RACE, the oligo(dT) anchor primer was at the concentration of 32.5 µM, in the standard conditions. For the 5' RACE, a specific primer (SPV1) was used at the concentration of 32.5 µM. The cDNA purification and the subsequent tailing reaction was performed according to the manufacturer's instructions.

### Polymerase Chain Reaction and cDNA cloning of Pv-FRIL-Encoding Nucleic Acid

The 3' end of Pv-FRIL was obtained through rapid amplification of cDNA ends by polymerase chain reaction (RACE-PCR) using the 5'/3' RACEKIT (Boehringer Mannheim) used according to the manufacturer's instructions. Nested PCR amplifications were performed using the PCR-Anchor primer with the specific primers (PV3 and PV4) in two successive amplification reactions. The sequences of these primers is as follows:

PV3: CAA TGT CTT ACA ACT CAC TAA G
PV4: AGT GTG GGA AGA GTG TTA TTC

The amplification conditions were 30 cycles of 40 seconds at 94 °C, 40 seconds at 55 °C, 60 seconds at 72 °C each and an extension step at 72 °C for 10 min. The reactions were performed in 50 µL containing 30 pmol of each primer, PV3 and PCR-Anchor primer in the first, and PV4 and PCR-Anchor primer in the second, 0.2 mM deoxyribonucleotides and 0.5 units of Ampli-Taq polymerase (Perkin Elmer) in the corresponding buffer. The 831 bp product obtained was sub-cloned in pCR2.1 and sequenced as above reported.

For the 5'RACE, again nested PCR reactions were performed using in combination with the Anchor Primer the specific primers SPV2 and SPV3. The sequences of these primers is as follows: 
SPV2: ACC AAA GCT TTG GTT TTC AGA
SPV3: TCT GAA AAC GTT TGA GTA GAG
 The amplification conditions for both reactions were 30 cycles of 40 seconds at 94 °C, 40 seconds at 50 °C, 60 seconds at 72 °C each and an extension step at 72 °C for 10 min. The reactions were performed in 50 µL containing 30 pmol of each primer, SPV2 and PCR-Anchor primer in the first, and SPV3 and PCR-Anchor primer in the second, 0.2 mM deoxyribonucleotides and 0.5 unit of Ampli-Taq polymerase (Perkin Elmer) in the corresponding buffer.

To obtain the full-length cDNA clone, recombinant PCR was performed (Higuchi R., *supra).* Two PCR reactions were carried out separately, one on the 5' fragment and the other on the 3' RACE product using primers with an overlapping region. The overlapping primary products were subsequently re-amplified using the flanking primers resulting in a full-length fragment.

The primary PCR products were purified with the QIAquick PCR Purification kit (QIAGEN) used according to the manufacturer's instructions, and amplified together in a single second reaction. For the second PCR reaction, the primers PVEcoRI and the APEcoRI were used. The sequences of these primers is as follows:

PVEcoRI TAC ATG AAT TCG CTC AGT CAT TAT CTT TTA AC
APEcoRI: GAA TTC GAC CAC GCG TAT CGA TGT CGAC

Both primary and secondary PCR reactions were performed in 100 µL containing 50 pmol of each primer, 0.4 mM deoxyribonucleotide and 1.0 unit Pfu polymerase (Stratagene) in the corresponding buffer. The primary PCR reaction amplified the two separate fragments by 30 cycles, each cycle comprising 40 seconds at 94°C, 40 seconds at 50°C, 60 seconds at 72°C and an extension step at 72°C for 10 min. The second PCR reaction amplified the recombinant fragment in 12 cycles using the same conditions reported above. The full lengthed product was cloned in the EcoRI site of the cloning vector pCR2.1 (Fig. 24A) and sequenced as noted above. This plasmid is referred to as pCR2.1-Pv-FRIL.

The nucleic acid sequence of the Pv-FRIL cDNA is as follows:

1 GCTCAGTCAT TATCTTTTAA CTTTACCAAG TTTGATCTTG ACCAAAAAGA
51 TCTTATCTTC CAAGGTGATG CCACTTCTAC AAACAATGTC TTACAACTCA
101 CTAAGTTAGA CAGTGGAGGA AACCCTGTGG GTGCTAGTGT GGGAAGAGTG
151 TTATTCTCTG CACCATTTCA TCTTTGGGAA AACTCTATGG CAGTGTCAAG
201 CTTTGAAACT AATCTCACCA TTCAAATCTC AACACCTCAC CCTTATTATG
251 CAGCTGATGG CTTTGCCTTC TTCCTTGCAC CACATGACAC TGTCATCCCT

301 CCAAATTCTT GGGGCAAATT CCTTGGACTC TACTCAAACG TTTTCAGAAA
351 CTCCCCCACC TCTGAAAACC AAAGCTTTGG TGATGTCAAT ACTGACTCAA
401 GAGTTGTTGC TGTCGAATTT GACACCTTCC CTAATGCCAA TATTGATCCA
451 AATTACAGAC ACATTGGAAT CGATGTGAAC TCTATTAAGT CCAAGGAAAC
501 TGCTAGGTGG GAGTGGCAAA ATGGGAAAAC GGCCACTGCA CGCATCAGCT
551 ATAACTCTGC CTCTAAAAAA TCAACTGTTA CTACGTTTTA TCCTGGGATG
601 GAAGTTGTGG CTCTCTCCCA TGATGTTGAC TTACATGCAG AGCTTCCTGA
651 ATGGGTTAGA GTAGGGTTAT CTGCTTCAAC TGGAGAGGAG AAACAAAAAA
701 ATACCATTAT CTCATGGTCT TTCACTTCAA GCTTGAAGAA CAACGAGGTG
751 AAGGAGCCGA AAGAAGACAT GTATATTGCA AACGTTGTGC GATCATATAC
801 ATGGATCAAT GACGTTCTAT CTTATATAAG CAATAAATAA ATGTATGATG
851 CACTCAATAA TAATCACAAG TACGTACGGT GTAGTACTTG TATGTTGTTT
901 ATGAAAAAAA AAAA (SEQ ID NO: 5)

The amino acid sequence of Pv-FRIL is as follows:

AQSLSFNFTKFDLDQKDLIFQGDATSTNNVLQLTKLDSGGNPVGASVGRVLFSAPFHLWENS
MAVSSFETNLTIQISTPHPYYAADGFAFFLAPHDTVIPPNSWGKFLGLYSNVFRNSPTSENQ
SFGDVNTDSRVVAVEFDTFPNANIDPNYRHIGIDVNSIKSKETARWEWQNGKTATARISYNS
ASKKSTVTTFYPGMEVVALSHDVDLHAELPEWVRVGLSASTGEEKQKNTIISWSFTSSLKNN
EVKEPKEDMYIANVVRSYTWINDVLSYISNK*MYDALNNNHKYVRCSTCMLFMKKK
(SEQ ID NO: 6)

The amino acid sequence of Pv-FRIL was compared to the amino acid sequences of Dl-FRIL and of the PHA-E lectin. This comparison is shown on Fig. 24B.

### Pv-FRIL-Encoding Plant Expression Vectors and Nicotiana tabacum Transformation

Recombinant PCR was used to introduce a signal peptide for entry of Pv-FRIL into the endoplasmic reticulum at the 5' end of the Pv-FRIL cDNA clone. Following the procedure of Higuchi (*supra*) the signal peptide and the full length cDNA clone were amplified in two separate primary PCR reactions. The signal peptide was obtained from the amplification of the binary vector pTA4, harboring the complete sequence of the bean α-amylase inhibitor gene (Hoffman et al., L.M., Y. Ma and R.F. Barker, *Nucleic Acid Res. 10:* 7819-7828,1982; Moreno and Chrispeels,

### Proc. Natl. Acad. Sci. USA 86: 7885-7889, 1989).

The primers used for the two primary reactions are the following:
Amplification of the Signal Peptide 
Sigforw BglII: AGA TCT ATG GCT TCC TCC AAC
Sigrew: AAA GAT AAT GAC TGA GCG GCT GAG TTT GCG TG
Amplification of the mannose lectin cDNA:

SpMlforw: CAC GCA AAC TCA GCC GCT CAG TCA TTA TCT TT
APXhoI: CTC GAG GAC CAC GCG TAT CGA TGT CGA

The primers used for the secondary reactions, Sigforw and AP, were designed to generate BglII sites at the 5' and XhoI at the 3' ends. Both, primary and secondary PCR reactions were performed as discussed above. The recombinant product SpPv-FRIL was incubated for 10 min. at 72 °C with 0.5 units of Ampli-Taq polymerase (Perkin Eliner) and cloned in the cloning vector pCR2.1 (Fig. 25). The nucleotide sequence of the PCR product was determined as described above to verify the correct attachment of the signal peptide.

A binary vector was constructed for constitutive expression of Pv-FRIL in tobacco plants. The recombinant SpPv-FRIL was cloned in BglII/XhoI sites of a plant expression vector resulting in the formation of pM-SpPv-FRIL (Fig. 26).

The binary vector was transferred in *Agrobacterium tumefaciens* strain C58 according to the freeze-thaw procedure reported of An *et al., supra.* Agrobacterium-mediated transformation of *Nicotiana tabacum* leaf disks was carried out as described by Horsh *et al. (Science* 227:1229 -1231,1985) using C58 harboring the expression vector pM-SpPv-FRIL. Kanamycin resistant plants were scored for their ability to form roots in two consecutive steps of propagation in Murashige-Skoog medium containing 3% sucrose and kanamycin sulfate (Sigma) at 100 mg/L.

Tobacco plants transformed with this construct were grown in a growth room under controlled conditions. The leaves (20 grams) of young plants were harvested and frozen in liquid nitrogen and powdered in a mortar with a pestle. The powder was stirred in a buffer mixture consisting of 1 x phosphate buffered saline containing 1mM CaCl₂ with a cocktail of protease inhibitors (PMSF, Pepstatin and Leupeptin). This slurry was centrifuged at 2000 rpm and the supernatant was centrifuged at 40,000 rpm in a Beckman ultracentrifuge. The clear supernatant was tumbled with 1 ml of ovalbumin-Sepharose for 3 hours. The beads were washed with the same buffer and tumbled overnight with 200 mM trehalose in 1/10 phosphate buffered saline containing the cocktail of protease inhibitors. Coomassie blue stained gel showed this preparation to be pure Pv-FRIL. An immunoblot showed the presence of both alpha and beta subunits, thus, the single polypeptide chain encoding both subunits was cleaved by the transformed cells *in vivo*. Binding to the ovalbumin-sepharose and release by trehalose shows that the product of the transgene is an active lectin.

### EXAMPLE 6

### Dl-FRIL Supports Prolonged ex vivo Maintenance of Quiescent Human CD34+,CD38-/SCID-Repopulating Cells

To further characterize the progenitor cell preservation activity of Dl-FRIL, a functional *in vivo* assay for primitive human hematopoietic cells was used to determine the cells' ability to home to and repopulate the marrow of sublethally irradiated C.B-17 and NOD/LtSz mice homozygous for the severe combined immunodeficiency *Prkdc* ^{*scid*} mutation (Lapidot et al., *Science* 255:1137,1992; Larochelle et al., *Nat. Med*. 2:1329-1337,1996). To do this, the following methods were used:

### Preparation of Human Cells

Human umbilical cord blood (CB) samples were obtained from full term deliveries. The blood samples were diluted 1:1 in phosphate-buffered-saline (PBS) without Mg⁺²/Ca⁺², supplemented with 10% fetal bovine serum (FBS). Low density mononuclear cells were collected after standard separation on Ficoll-Paque (Pharmacia Biotech, Uppsala, Sweden), and washed in RPMI with 10% FBS. Some samples were frozen in 10% DMSO, while the others were used fresh. Enrichment of CD34⁺ cells was performed with mini MACS separation kit (Miltenyi Biotec, Bergisch Gladbach, Germany) according to the manufacturer's instructions. The purity of the enriched CD34⁺ cells was 60-80% using one column. CD34⁺CD38^{-/low} cells were purified by FACS sorting (FACStar⁺, Becton Dickinson, San Jose, CA) after staining CD34⁺ enriched cells with mAb anti human CD34-FITC (Becton Dickinson) and anti human CD38 PE (Coulter, Miami FL USA) (Purity >99%).

### Mice

Eight week old NOD/LtSz-*Prkdc*^{*scid*}/*Prkdc*^{*scid*} (NOD-SCID) mice and NOD/SCID β2 microglobulin knockout mice, hereafter termed NOD/SCID B2M^{*null*} (Christianson et al., *J. Immunol*. 158:3578-3586,1997), bred and maintained under defined flora conditions in sterile micro isolator cages, were irradiated with a sublethal dose of 375 cGy at 67cGy/min. from a cobalt (⁶⁰Co) source prior to transplantation.

Human cells were injected into the tail vein of irradiated mice in 0.5 mL of RPMI with 10% FBS. In some experiments (as indicated) non engrafting irradiated (1500 cGy) CD34⁻ cells served as carrier cells, and were cotransplanted with cultured cells at a final concentration of 0.5x10⁶ cells/mouse. Mice were sacrificed 1 month post transplantation, and bone marrow (BM) cells were flushed from the 8 bones of each mouse (femurs, tibias, humeri, and pelvis).

### Ex vivo cultures

Human CD34⁺ enriched cells were cultured in 24 well plates (2-4x10⁵ cells in 0.5 mL), containing RPMI supplemented with 10% FBS+1% BSA. *Ex vivo* cultures contained the following cytokine combination: Stem cell factor (SCF) -100 ng/mL and Flt3 ligand (Flt3-L) - 100 ng/mL (R&D Systems Inc. Minneapolis, MN, USA), rhIL-6 - 50 ng/mL and sIL6R -1280 ng/mL, (InterPharm Laboratories, Ares-Serono Group, Ness Ziona, Israel) or D1-FRIL -10 ng/mL (ImClone Systems Inc., NY, USA). In some experiments where indicated, the growth factor cocktail included 300 ng/mL SCF, 300 ng/mL Flt3-L, 50 ng/mL G-CSF, 10 ng/mL IL-3 (R&D Systems), and 10 ng/mL IL-6. The cultures were incubated at 37°C in a humidified atmosphere containing 5% CO₂. After 3, 6, 10 and 13 days, the cells were collected, counted, analyzed by FACS, seeded in to semisolid cultures and transplanted into NOD-SCID mice. For CD56⁺ NK cell development, BM cells from engrafted mice were cultured with 100 ng/mL of SCF and IL-15 (R&D) for 10-14 days.

### Preparation of D1-FRIL

D1-FRIL was isolated from the seeds of hyacinth beans (*Dolichos lab lab)* using the protocol described above in Example 1.

### CFU assay.

Semisolid cultures were performed in order to detect the levels of human progenitors in *ex vivo* cultures, and in the marrow of transplanted mice. The cells were plated (4x10³ cells/mL) in 0.9% methylcellulose (Sigma, St. Louis, MO, USA), 30% FBS, 5x10⁻⁵M 2ME, 50 ng/mL SCF, 5 ng/mL IL-3, 5 ng/mL GM-CSF (R&D), and 2 u/mL Erythropoietin (Ortho Bio Tech, Don Mills, ON, Canada). Human cells from the BM of engrafted mice were plated (2 x10⁵ cells/mL) in 15% FBS + 15% human plasma, selective for human colonies only. The cultures were incubated at 37°C in a humidified atmosphere containing 5% CO₂ and scored 14 days later with an inverted microscope for myeloid, erythroid, and mixed colonies by morphologic criteria.

### Cell cycle analysis.

Cells were analyzed for their DNA content by staining with propidium iodide (Sigma). The cells were cultured in *ex vivo* cultures as described, for 3,6,10, and 13 days as indicated. At each time point, the cells were collected, resuspended to a final concentration of 0.1-1x10⁶ cells/mL, and incubated with 0.1% Triton X 100 (Sigma) for 20 minutes on ice. 50 mg/mL propidium iodide (PI) were added before analysis. Flow cytometeric analyses were performed using FACSort (Becton Dickinson, San Jose, CA).

### Flow cytometry analyses.

Human and mouse Fc receptors on BM cells from transplanted mice were blocked by using human plasma (1:50) and anti mouse Fc receptor blockers (anti mouse CD16/CD32 mAb, Pharmingen, San Diego, CA, USA). Isotype control mAb were used in order to exclude false positive cells (Coulter, Miami FL USA). The purity of enriched subpopulations after magnetic bead separation, were analyzed by two color staining, using anti-human CD34 FITC (Becton Dickinson, San Jose, CA, USA) and anti-human CD38 PE (Coulter). The levels of human cells and lymphoid lineages in the marrow of engrafted mice were detected by double staining with anti-human CD45 FITC (Immuno Quality Products, Groningen, The Netherlands) together with anti-human CD19 PE (Coulter) for detection of pre B cells, or with anti-human CD56 PE (Coulter) for detection of NK cells. Cells were washed with PBS supplemented with 1% FBS and 0.02% azide, suspended to a volume of 0.1-1x10⁶ cells/mL, stained with direct-labeled mAb and incubated for 25 minutes on ice. After staining, cells were washed once in the same buffer and analyzed on a FACSort (Becton Dickinson). Analysis was performed using CELLquest software (Becton Dickinson).

### Human cell engraftment analysis.

The levels of human cell engraftment were determined by both flow cytometry for analysis of human myeloid CD45⁺ and lymphoid CD45⁺CD19⁺ pre B cells and quantification of human DNA as previously described (Lapidot et al., *Science* 255:1137,1992; Larochelle et al., *Nat. Med.* 2:1329-1337, 1996; Peled et al., Science 283:845-848,1999). Briefly, high molecular weight DNA was obtained from the BM of transplanted mice by phenol/chloroform extraction. DNA (5 g) was digested with EcoRI, subjected to electrophoresis on 0.6% agarose gel, blotted onto a nylon membrane, and hybridized with a human chromosome 17-specific α-satellite probe (p17H8) labeled with ³²P (Lapidot et al., *supra).* The intensity of the bands in the samples were compared to artificial human/mouse DNA mixtures (0%, 0.1%, 1%, and 10% human DNA) to quantify the human DNA (lanes to the right of lane 4 in Fig. 31). Multiple exposures of the autoradiographs were taken to ensure sensitivity down to 0.01% human DNA. A transplanted mouse was scored positive when both human myeloid and lymphoid cells and human DNA were detected in its BM.

From these experiments, the following results were obtained:

### D1-FRIL maintains but does not expand CB CD34± progenitors in suspension culture

As shown above, Dl-FRIL by itself preserves immature CB progenitor cells up to a month in suspension culture without medium changes (see, e.g., Figs 14A and 14B, and Table 3). To compare D1-FRIL's progenitor-preserving properties to a combination of cytokines (SCF, Flt3-L, IL-6, and sIL6-R) shown to maintain CB progenitor cells in suspension culture (Sui et al., *Proc. Natl. Acad. Sci. USA* 92:2859, 1995; Ebihara et al., *Blood* 90:4363-4368,1997), enriched CB CD34⁺ cells were cultured with either D1-FRIL or cytokines for 3, 6, 10, or 13 days in medium containing 10% FBS and 1% BSA in RPMI. Fresh media and D1-FRIL and/or cytokines were added on day 6. Cells harvested at each time point were counted and assayed for clonogenic progenitor cells by plating *(i.e.,* seeding) in semisolid media.

As shown in Fig. 27A, the total number of cells in Dl-FRIL cultures gradually declined over time from 2x10⁵ cells initially seeded to 1.26x10⁵ cells at day 13, in contrast to the expected 14.3-fold increase to 2.8x10⁶ cells in cytokine cultures at day 13. Similarly, as shown in Fig. 27B, the levels of progenitor cells in Dl-FRIL cultures also remained relatively constant until day 10, after which they declined to 9 % of the starting population (from 24.7x10³ colonies on day 0 to 2.3x10³ colonies on day 13). Progenitor levels increased in cytokine cultures by 10-fold from the outset to day 13 of culture (Fig. 27B).

### D1-FRIL maintains the expansion capacity of CD34± progenitors up to 2 weeks in ex vivo culture

To characterize the expansion capacity of D1-FRIL-preserved progenitor cells in suspension culture, CD34⁺ cells cultured for 6 days with Dl-FRIL were washed and exposed to cytokines (without D1-FRIL) for an additional 4 days. Total cell counts and clonogenic progenitor assays were performed and the results were compared to cells cultured for 10 days with Dl-FRIL. Fresh media and Dl-FRIL were added on day 6. Interestingly, the Dl-FRIL-cultured cells proliferated in response to cytokine stimulation, resulting in a 3.4-fold increase in cell numbers (Fig. 28A) and 13-fold increase in progenitor levels (Fig. 28B).

Further experiments tested D1-FRIL's ability to preserve cytokine-responsive CD34+ progenitor cells cultured with either D1-FRIL for 10 days followed by 3 days of cytokine stimulation or only with D1-FRIL for the entire 13 days. Fresh media and Dl-FRIL were added on day 6. A 2.9-fold increase in total cell numbers (Fig. 28C) and a 5.5-fold increase in progenitor levels (Fig. 28D) were observed for cells cultured with Dl-FRIL for 10 days followed by an additional 3 days of cytokine stimulation compared to cultures with Dl-FRIL alone for 13 days. These results provide evidence that Dl-FRIL alone maintains the proliferative capacity of human progenitor cells up to 13 days in suspension culture and that subsequent stimulation of cells cultured for 10 days with Dl-FRIL still respond to the proliferative signals of the cytokines.

### DI-FRIL maintains SCID repopulating stem cells (SRC) in ex vivo cultures

At each time point during culture, the human CB CD34⁺ cells were collected and the content of each well was assayed for progenitor levels (described above) and 2x10⁵ of the remaining cells were transplanted into one mouse. One month later, mice were sacrificed and their bone marrows were harvested and assayed for the presence of human myeloid and lymphoid cells. Before assaying, human DNA levels in the bone marrow of individual transplanted mice was quantitated by Southern blotting analysis. A representative Southern blot analysis showing the detection of a 0%, 0.1%, 1%, and 10% human DNA per murine DNA is provided in Fig. 29E.

Figs. 29A-29D show representative Southern blot analyses of the BM of mice transplanted with *ex vivo* cultured cells. Fig. 29A is a representative Southern blot showing human DNA in the marrow of mice transplanted with cells cultured with FRIL for 6 days (lane 1), FRIL for 10 days (lane 2), or with FRIL for 6 days followed by 4 days with cytokine stimulation (lane 3). In a typical experiment, the levels of engraftment by cells cultured with Dl-FRIL for 10 days decreased by approximately 10-fold from cells cultured with Dl-FRIL for 6 days (Fig. 29A, lanes 1 and 2). However, when cells cultured with Dl-FRIL for 6 days were subsequently stimulated with cytokines for 4 days, there was an approximate 10-fold increase in the level of engraftment (Fig. 29A, lane 3) compared to Dl-FRIL alone for 10 days (lane 2). Similar results were obtained when cells from other donors were transplanted as shown in Fig. 29B, lanes 3-4 (cells cultured with Dl-FRIL for 6 days followed by 4 days of cytokine stimulation) compared to lanes 1-2 (cells cultured with Dl-FRIL alone for 10 days) prior to transplantation. Dl-FRIL cultures could be prolonged even to 13 days, as shown in Figs. 28C-28D, prior to transplantation into mice. A modest increase in the levels of engraftment was seen comparing the marrow of mice transplanted with the original cells, which were not cultured with Dl-FRIL, prior to seeding (Fig. 29C, lane 1) with cells cultured with Dl-FRIL for 13 days (Fig. 29C, lane 2). Fig. 28D shows the results of another experiment, where the difference between engraftment levels obtained by cells cultured with Dl-FRIL for 10 days (lane 1) or with Dl-FRIL for 6 days followed by 4 days of cytokine stimulation (lane 2) are minor. Nevertheless, a 10-fold increase was observed for cells cultured either with Dl-FRIL for 13 days (Fig. 29D, lane 3) or with Dl-FRIL for 10 days followed by 3 days of cytokine stimulation (Fig. 29D, lane 4) when compared to cells cultured for 10 days (Fig. 29D, lanes 1 and 2).

Levels of human engraftment in the marrow of mice transplanted with CD34⁺ cells cultured with either Dl-FRIL for 10 days (n=12) or Dl-FRIL for 6 days (n=33) followed by cytokine stimulation for 4 days are summarized in Fig. 30. Cells cultured with Dl-FRIL followed by cytokines, engrafted at levels 7.4-fold greater than cells cultured with Dl-FRIL alone for 10 days (Fig. 30, p=0.05).

To test the effect of Dl-FRIL on CB populations highly enriched for primitive human SRC, CD34⁺ cells isolated by immunomagnetic beads were further sorted by flow cytometry based on the absence of CD38 expression. CD34⁺CD38^{-/low} cells (99% purity) cultured with either Dl-FRIL alone or Dl-FRIL followed by cytokines showed patterns of engraftment in NOD/SCID B2M^{*null*} mice (Christianson et al., *supra),* similar to those observed for CD34⁺ cells. These mice have been used successfully for secondary human stem cell transplantation (Peled et al., *supra),* have less innate immunity due to lack of NK activity and thus require fewer (10 fold) human cells for engraftment compared to NOD/SCID mice.

Fig. 31 is a representative Southern blot (1 out of 4 experiments) showing the relative level of engraftment of cells that were cultured with D1-FRIL for 10 days (lane 4) compared to 6 days with Dl-FRIL followed by 4 days cytokine stimulation (lanes 1-3) prior to transplantation into mice. High molecular weight DNA was obtained from the BM of transplanted mice and subjected to Southern blotting analysis for the presence of human DNA.

Based on these results, an increased level of engraftment in cells exposed to cytokines after Dl-FRIL was expected. Consequently, the same initial cell dose transplanted into one mouse in lane 4 was divided into 3 mice in lanes 1-3, indicating an expansion of D1-FRIL cultured SRC when they were subsequently exposed to cytokine stimulation. The level of engraftment in mice transplanted with cells that were treated with Dl-FRIL followed by cytokines (Fig. 31, lanes 1-3) was about 10-fold higher compared to the mouse transplanted with cells cultured with D1-FRIL alone (Fig. 31, lane 4). Since the initial dose of cells for the D1-FRIL + cytokines sample was split into three mice, a 30-fold increase in the level of engraftment is observed for CD34⁺CD38^{-/low} cells stimulated by cytokines after Dl-FRIL compared to the culture with Dl-FRIL alone. In addition, this result represents about a 3-fold greater level of expansion of SRC for CD34⁺CD38^{-/low} cells than for total CD34⁺ cells. Fig. 32 summarizes the fold increase observed in the engraftment levels of CD34⁺CD38^{-/low} cells cultured with Dl-FRIL alone compared to cells subsequently stimulated with cytokines in 6 experiments (3 experiments with NOD/SCID B2M^{*null*} mice and 3 experiments with NOD/SCID mice, gave similar results). These data indicated a 30-fold expansion of SRC for CD34 ⁺CD38^{-/low} cells and a 3-fold greater level than that of total CD34⁺ cells in the corresponding experiments.

Further studies have demonstrated that Dl-FRIL preserves long-term repopulating stem cells. Bone marrows harvested from NOD/SCID mice that were initially transplanted with CD34+ cord blood cells cultured in Dl-FRIL were transplanted into a second set of sublethaly irradiated NOD/SCID mouse recipients. After one month, bone marrows from the second set of recipients were analyzed for the presence of human hematopoiesis, as determined using the assays described above. Multilineage engraftment was observed in the second set sublethaly irradiated NOD/SCID mouse recipients. This observed persistence of repopulating cells in this serial transplantation study indicated the presence of long-term repopulating stem cells.

### D1-FRIL preserves SRC potential of multilineage differentiation in the murine BM

Southern blot analysis detected and quantified the levels of human DNA in the marrow of transplanted mice without specifically indicating the differentiation status of human cells recovered from the murine BM. Since SCID repopulating stem cells (SRC) are defined by multilineage differentiation of myeloid and lymphoid cells in NOD/SCID mice, the *in vivo* differentiation processes of D1-FRIL-cultured CB CD34⁺ cells in the murine BM were further studied. To do this, CD34 ⁺ or sorted CD34⁺CD38^{-/low} cells were cultured either with FRIL for 10 days or with FRIL for 6 days followed by cytokine stimulation for 4 days prior to transplantation. BM of transplanted mice was collected 1 month later and the cells were either seeded in semisolid media selective for human colonies (results shown in Fig. 33A) or were analyzed for lineage specific markers by flow cytometry (results shown in Figs. 33B-33F).

The progenitor capacity of human CB CD34⁺ cells cultured for 10 days with Dl-FRIL and harvested one month after transplantation into NOD/SCID mice was evaluated in semi solid-colony assays that selectively promoted growth of human progenitors. Fig. 33A shows that the levels of human progenitors in the bone marrow of transplanted mice increased 2.3-fold when SRC were stimulated with cytokines for 4 days after 6 days of Dl-FRIL incubation compared to D1-FRIL alone for 10 days prior to transplantation. Moreover, both myeloid and erythroid colonies formed in the colony assays (data not shown) as well as human B cell differentiation that was determined by flow cytometry (Figs. 33C and 33D), indicating that incubation with Dl-FRIL maintained the multilineage differentiation potential of SRC. A representative flow cytometry analysis demonstrated the presence of human CD45⁺CD19⁺ pre-B cells in the marrow of mice transplanted with either CD34⁺ cells (Fig. 33C) or CD34⁺CD38^{-/low} cells (Fig. 33D) cultured with Dl-FRIL for 10 days (1% or 10% CD19⁺ cells, respectively).

To determine whether human lymphoid precursors in the marrow of transplanted mice have the potential to differentiate in addition to myeloid cells also into lymphoid NK cells, cells from transplanted murine bone marrow were also cultured with SCF and IL-15 for 10 days. Cells harvested from cultures were analyzed for human NK cells by flow cytometry using the human-specific monoclonal antibodies to CD45 and the NK cell antigen, CD56. Cells from mice transplanted with CD34⁺ or CD34⁺CD38^{-/low} cells are shown (1% or 7.7% of CD56⁺ cells; Figs. 33E and 33F, respectively).

### ex vivo preservation of early progenitors with D1-FRIL compared to Flt3 ligand

Dl-FRIL was identified by its ability to stimulate proliferation of NIH 3T3 cells transfected with Flt3 and not by untransfected cells or cells transfected with the related Fms tyrosine kinase receptor (Moore et al., *Blood* 90 supp. 1:308,1997). Although D1-FRIL and Flt3-L both stimulate proliferation of Flt3 3T3 cells, they exert different activities on CB progenitor cells. Flt3-L induces quiescent primitive cells into cycle (Lyman and Jacobsen, *Blood* 91:1101-11345,1998). In contrast, Dl-FRIL by itself maintains progenitors in serum-defined medium from 15-29 days in culture without medium changes (Colucci et al., *Proc. Natl. Acad. Sci. USA* 96(2):646-650, 1999). The abilities of Dl-FRIL or Flt3-L to maintain CB CD34⁺ progenitors in suspension cultures consisting of 10% FBS and 1% BSA in RPMI for 10 days were compared in the presence and absence of cytokines. The cells were then were seeded for CFU assay.

As shown in Fig. 34A, the number of total cells harvested after 10 days of suspension culture in Dl-FRIL increased minimally by 1.9-fold, whereas cells cultured in either Flt3-L alone or in combination with Dl-FRIL increased by 4.5-fold and 5.4-fold, respectively (p<0.05). Cultures containing CB CD34⁺ cells with cytokines, either alone or with D1-FRIL and Flt3-L, led to 10.9-12.5 fold increase in cell numbers; however cells cultured with D1-FRIL alone followed by cytokines, increased only by 4.6-fold (Fig. 34A, p<0.05).

Interestingly, cells cultured with D1-FRIL alone or followed by cytokines, selectively maintained a higher number and proportion of the primitive granulocyte-erythroid-macrophage-megakaryocyte colony forming-units (CFU-GEMM) compared to other treatments (Fig. 34B, p<0.05). About 40% of the progenitor cells maintained by Dl-FRIL were CFU-GEMM, a relative 2.6- to 3.7-fold increase with Dl-FRIL cultures compared to other combinations (Fig. 34B, p<0.05). In contrast to Flt3-L, exposure of cells first cultured with Dl-FRIL to cytokines, maintained the high percentage of CFU-GEMM, 18.2% versus 38.6%, respectively (Fig. 34B p<0.05). These results demonstrate that Dl-FRIL and Flt3-L may act differently on primitive progenitor cells in culture.

### Dl-FRIL maintains higher levels of CD34± cells in G₀/G₁ phase of cell cycle compared to cytokine treated cells

*In vivo,* immature CD34⁺ cells are predominantly quiescent, non-cycling cells (Young et al., *Blood* 87:545, 1996; Movassagh et al., *Stem Cells* 15:214-222,1997; Ladd et al., *Blood* 90:658-668,1997). Since Dl-FRIL maintains relatively constant levels of progenitor cells over two weeks in suspension culture that can subsequently respond to cytokine stimulation (see Fig. 27), the cell cycle status of CD34 ⁺ cells incubated with Dl-FRIL was investigated and compared to cultures with cytokine stimulation. DNA content of CB CD34⁺ cells was analyzed by flow cytometry immediately after isolation of cells. A mean value of 96.6% of cells were observed in the G₀/G₁ phase of cell cycle (Fig. 35A).

The cell cycle status of CB CD34⁺ cells was analyzed in 5 indepenent experiments with cells cultured for 3-13 days with either Dl-FRIL or a cytokine combination (SCF, Flt3-L, G-CSF, IL-3, IL-6) (Bhatia et al., *J. Exp. Med*. 186:619-624, 1997; Conneally et al., *Proc. Natl. Acad. Sci. USA* 94:9836-9841, 1997). The percentage of cycling cells in SG₂M phase in both cultures decreased by half from day 3 to day 13, from 17.7% to 9.1% for Dl-FRIL and from 50.9% to 23.2% for cytokines (Table 1) (3.4% of cells initially seeded were in SG₂M).

**TABLE 5**

| Percentage and numbers of cycling CD34± cells (in SG₂M phase) cultured with Dl-FRIL or cytokines for 3,6,10 or 13 days | | | | | |
|---|---|---|---|---|---|
| Days in culture | Dl-FRIL (%) | Cytokines (%) | Dl-FRIL (cells x10³) | Cytokines (cells x10³) | Fold increase induced by cytokines |
| day 3 | 17.7±1.9 | 50.8±1.4 | 35 | 212 | 6 |
| day 6 | 20±2 | 26 | 43.6 | 389.5 | 9 |
| day 10 | 10±1.9 | 29.7±1 | 26.4 | 553 | 21 |
| day 13 | 9.1±4.1 | 23.14 | 11.4 | 664.6 | 60 |
| CD34⁺ cells were cultured (2x10⁵ cells/0.5 ml) with Dl-FRIL or SCF+Flt3 ligand+IL-3+GCSF+IL-6. Cell cycle analysis was performed with propidium iodide staining. Cell numbers were calculated by multiplying percent of cycling cells by total cell numbers. Data represents mean±SE values, from 5 experiments. | | | | | |

More dramatically, the number of cells in SG₂M phase during culture differed from the 6.8x 103 cells in SG₂M phase initially seeded. The average number of cycling cells in Dl-FRIL cultures remained relatively constant from 35x103 cells at day 3 and 43.6x10³ cells at day 6 to a reduced level of 26.4x10³ cells at day 10 and 11.4x10³ cells at day 13. This pattern of cells in cycle explains the low levels of total cell numbers and progenitor cells (see Fig. 27). In contrast, the expected number of cycling cells in cytokine cultures increased dramatically. The average number of cells in SG₂M phase in cytokine cultures increased by 31-fold to 212x10³ cells at day 3, by 57-fold to 389.5x10³ cells at day 6, by 81-fold to 553x10³ cells at day 10, and by 97.7-fold to 664.6x10³ cycling cells at day 13, compared to 6.8 x 103 cells in SG₂M seeded in culture (Table 1). Taken together, these results support the notion that Dl-FRIL preserves more immature progenitor cells in a quiescent state up to 2 weeks in culture compared to cytokine treated cultures.

To test whether Dl-FRIL acts in a dominant manner to prevent cytokines from inducing the quiescent progenitor population into SG₂M phase, the cell cycle status of cells cultured with either Dl-FRIL or Flt3-L, in the presence and absence of cytokines was analyzed, after 3 days of suspension culture as indicated in Figs. 35A-35G and Table 6. Figs. 35A-35G show representative cell cycle histograms of CD34 ⁺ cells cultured with no addition (Fig. 35A), Dl-FRIL alone (Fig. 35B) or various cytokines or combinations thereof (Figs. 35C-35G) for 3 days, and the mean percentage of cells in each cell cycle phase is summarized in Table 6.

**TABLE 6**

| Cell cycle status of CD34+ cells, *ex vivo* cultured for 3 days | | | |
|---|---|---|---|
| Culture conditions | Sub G₀/G₁ | G₀/G₁ | SG₂M |
| Dl-FRIL | 9.4±2.8 | 72.9±2.8 | 17.7±1.9 |
| Flt3-L | 9.4±3.3 | 68.4±2.1* | 22.2±5.4* |
| Dl-FRIL+Flt3-L | 8.6±3.9 | 67.1±1.8* | 24.3±5.65* |
| Cytokines | 2.6±1.7 | 46.5±4.5* | 50.9±1.4* |
| Cytokines+Dl-FRIL | 2.5±0.6 | 47.5±2.2* | 50.0±2.7* |
| Cytokines+Flt3-L | 2.1±0.5 | 47.7±2.4* | 50.2±2.9* |
| Data shown is mean percentage ± SE, from 4 independent experiments. p values = (vs. Dl-FRIL): | | | |

| | | | |
|---|---|---|---|
| * p<0.05. | | | |

As was also shown in Table 5, Dl-FRIL maintains a significantly higher percentage of quiescent cells and lower percentage of cycling cells (Fig. 35B and Table 6) compared to stimulation with cytokines (Figs. 35C-35G and Table 6). Cultures with Flt3-L alone led to a modest, although significant, decrease percentage of cells in G₀/G₁, (p<0.05) and to a corresponding increase in SG₂M phase, compared to cells cultured with DI-FRIL alone (Table 6). Cultures containing Dl-FRIL and Flt3-L together slightly increased the percentage of cells in SG₂M phase, from 17.7% (with Dl-FRIL alone) to 24.3% (p<0.05, Table 6). As shown in Table 5, exposure of CB CD34⁺ cells to cytokines for 3 days induced a substantially greater proportion of cells into SG₂M phase compared to culture with Dl-FRIL alone (p<0.05). The percentage of cells undergoing apoptosis, as determined by subG₀/G₁ population, when cultured in Dl-FRIL was slightly higher than under cytokine conditions. Neither Dl-FRIL nor Flt3-L, under these culture conditions, effected cytokine induction into SG₂M phase of the mostly quiescent CD34⁺ cell population (Table 6).

### EXAMPLE 7

### Dl-FRIL Preserves CB Progenitors in a Dormant State in the Presence of Potent Stimulators

Dl-FRIL was purified from *Dolichos lab lab* as described above in Example 1. Dl-FRIL was assayed over a 5-log dose range (10 ng/mL-1,000 ng/mL) on human C.B MNC cultured in serum-defined medium containing agar-leukocyte conditioned medium, a potent source of a broad range of stimulators. The number of viable MNC and progenitors were evaluated after 5 days in culture. Results from 1 of 3 representative experiments are shown in Table 7 below. The number of viable MNC at the end of culture was reduced by 1.7 - to 5-fold in cultures containing 10-1,000 ng/ml of D1-FRIL. The frequencies of myeloid and erythroid progenitors in these cultures increased from 1.4- to 2.4-fold over the same dose range.

**TABLE 7**

| **Dl-FRIL** (ng/ml) | **MNC** (x 10⁻⁴) | **Progenitor frequency** (Colonies/10⁻⁵ MNC) | |
|---|---|---|---|
| | | Myeloid | Erythroid |
| 1,000 | 20 | 90 +/- 85 | 150+/-42 |
| 100 | 55 | 44 +/-15 | 85 +/-12 |
| 10 | 70 | 77 +/-12 | 103 +/- 6 |
| 1 | 120 | 16+/-2 | 38+/-7 |
| 0.1 | 115 | 21+/-7 | 50+/-7 |
| 0 | 120 | 38+/-7 | 63 +/- 32 |
| **Culture of CB MNC in D1-FRIL results in fewer MNC and a greater frequency of progenitors.** 4 x 10⁶ CB MNC were cultured in 2 mL of AIMV (Life Technologies) containing Agar-SCM (StemCell | | | |
| Technologies) and varying concentrations of D1-FRIL. Cultures were harvested after 5 days and the number of viable MNC and progenitors were assessed. The colony data shown were normalized as the frequency of progenitors per 10⁵ MNC. | | | |

A reduction in cell number and a corresponding increase in the frequency of progenitors in cultures containing Dl-FRIL in the presence of potent stimulators are consistent with our hypothesis that Dl-FRIL can prevent cytokine-induced proliferation and differentiation of progenitors. Table 8 below shows the relative decrease of MNC in Dl-FRIL-containing cultures compared to controls and the relative increase in progenitor frequency of progenitors. The total number of progenitors was reduced as expected (progenitors are at varying stages of differentiation 0 no direct correlation between progenitor number and MNC would be expected).

**TABLE 8**

| | **Fold DECREASE** | **Fold INCREASE** | |
|---|---|---|---|
| **Dl-FRIL (ng/ml)** | **MNC** | **Progenitor frequency** | |
| | | Myeloid | Erythroid |
| 1,000 | 5.0 | 2.4 | 2.4 |
| 100 | 2.0 | 1.2 | 1.4 |
| 10 | 1.7 | 2.1 | 1.6 |
| 1 | 1.0 | 0.4 | 0.6 |
| 0.1 | 1.0 | 0.6 | 0.8 |
| 0 | 1.0 | 1.0 | 1.0 |
| **Reduction in MNC correlates with the relative increase in frequency of progenitors.** The relative reduction in MNC of Dl-FRIL cultures is consistent with increased progenitor frequency in corresponding samples. | | | |

### EXAMPLE 8

### D1-FRIL protects CB MNC from the toxicity of chemotherapy drugs

Experiments showing that D1-FRIL can prevent proliferation and differentiation of CB progenitors in cultures containing potent stimulators indicated that Dl-FRIL protects progenitors from the toxicity of cell cycle-active chemotherapy drugs. The culture system described above was adapted to a 96 well plate format and the widely used chemotherapeutics, cytarabine (Ara-C) (Fig. 36A), doxorubicin (Dox) (Fig. 36B), and 5-fluorouracil (5-FU) (Fig. 36C) over a 5-log dose range were assayed on CB MNC cultured in the presence and absence of Dl-FRIL (see Figs. 36A-36C). D1-FRIL was purified from *Dolichos lab lab* as described above in Example 1. Cultures containing D1-FRIL (either at 10 ng/ml or 100 ng/ml) resulted in a 2-3 log dose shift of CB MNC to Ara-C (Fig. 36A and Dox (Fig. 36B). As shown in Fig. 36C, the presence of Dl-FRIL in 5-FU cultures increased viability over a large dose range. Differences between the dose shift of Ara-C and Dox by D1-FRIL compared to 5-FU may be explained by recent reports demonstrating that 5-FU acts via an RNA mechanism rather than as a DNA-specific drug (Bunz et al., J. *Clin. Invest.* 104:263-269,1999).

### EXAMPLE 9

### Mice tolerate high levels of Dl-FRIL

The *in vivo* toxicity of D1-FRIL was determined in mice. Initially, D1-FRIL was administered intravenously to mice over a 3-log dose range of 0.006-1 mg/kg (0.32-20 µg/mouse). D1-FRIL was well tolerated and these mice have subsequently received 2 monthly challenges of D1-FRIL without any observable short- or long-term adverse effects.

Protocols to test chemoprotective properties of cytokines in mice typically involve daily pre-treatment (bolus or continuous delivery) regimens of 4-10 days before starting chemotherapy. Using this framework as a starting point, Dl-FRIL was injected at 5 mg/kg (100 µg/mouse) intravenously daily for 4 days. No gross adverse effects have been observed in over 150 mice treated with this dose regimen. Dl-FRIL was purified from *Dolichos lab lab* as described above in Example 1.

The upper limits of Dl-FRIL toxicity were next explored by injecting a single bolus intraperitoneal injection of Dl-FRIL (to accommodate 1 mL volume) at 500 mg/kg and monitored the survival of mice for 48 hours. Of the four BALB/c mice receiving this treatment (2 males and 2 females, aged 5 months), only 1 mouse (a male) survived 48 hours. The surviving mouse's weight decreased by approximately 15% in the first 2 day and returned to normal by day 4. The surviving mouse's blood counts were in the normal range 3 days after injection of FRIL. The results demonstrate that even a very large dose of Dl-FRIL is not completely toxic.

Additional dose range finding studies are performed to determine toxicity in mice receiving high doses of Dl-FRIL administered by various routes (intravenous, intraperitoneal, subcutaneous, and oral).

### EXAMPLE 10

### in vivo modulation of progenitors by Dl-FRIL in mice

Using the initial planned dose regimen of Dl-FRIL (5 mg/kg x 4 days) for testing chemoprotection, hematopoietic parameters were examined in mice 3, 5, and 7 days after completing Dl-FRIL treatment. Dl-FRIL was purified from *Dolichos lab* *lab* as described above in Example 1. Weight-matched BALB/c mice (females, aged 8 weeks) were injected intravenously with either 0.2 ml of D1-FRIL (500 mg/ml) or 0.2 ml of HBSS daily for 4 days. Two mice from each group were evaluated at 3 days, 5 days, and 7 days after completing D1-FRIL treatment. Blood was collected in heparinized tubes by eye bleeds prior to sacrificing mice by CO₂. Two femurs and a spleen were harvested from each mouse and the samples were processed within 1 hour. Progenitors wee accessed using standard hematopoietic colony assays (StemCell Technologies). The results from this study are shown below in Table 9.

**TABLE 9**

| **Hematopoietic parameters 3, 5, and 7 days after Dl-FRIL treatment (5 mg/kg x** **4days)** | | | | | | |
|---|---|---|---|---|---|---|
| **Cellularities** | | | | | | |
| **Days** | **RBC** | **WBC** | | **BM** | **Spleen** | |
| 3 | 0.58 | 0.34 | | 0.59 | 1.01 | |
| 5 | 1.26 | 0.98 | | 0.75 | 0.72 | |
| 7 | 0.95 | 0.82 | | 0.91 | 1.12 | |

| **Bone marrow progenitors** | | | | | | |
|---|---|---|---|---|---|---|
| **Days** | **CFU-C** | | **CFU-E** | | **BFU-E + Mix** | |
| | Freq. | Total | Freq | Total | Freq | Total |
| 3 | 1.85 | 1.10 | 1.63 | 1.01 | 1.61 | 1.01 |
| 5 | 1.17 | 0.87 | 0.86 | 0.63 | 1.03 | 0.80 |
| 7 | 1.81 | 1.69 | 0.61 | 0.57 | 2.59 | 2.47 |

| **Spleen progenitors** | | | | | | |
|---|---|---|---|---|---|---|
| **Days** | **CFU-C** | | **CFU-E** | | **BFU-E + Mix** | |
| | Freq. | Total | Freq | Total | Freq | Total |
| 3 | - | - | 2.83 | 3.35 | - | - |
| 5 | 2.41 | 1.68 | 0.95 | 0.63 | 2.12 | 1.42 |
| 7 | - | - | 1.74 | 1.78 | - | - |
| Dl-FRIL data are reported as relative to control values. | | | | | | |

As shown in Table 9, the peripheral blood counts (red blood cell (RBC) and white blood cell (WBC)) of Dl-FRIL-treated mice were found to be reduced by 1.7-and 2.9-fold, respectively, at 3 days, and returned to normal by day 5. Bone marrow (BM) cellularity was also reduced by 2.5-fold at day 3, and returned to normal after 7 days. The spleen cellularity was lower at day 5 but normal at day 3 and day 7.

As shown in Table 9, the frequency of progenitors was slightly increased in bone marrow, by 1.6- to 1.85-fold at day 3, but the total number of progenitors in the bone marrow remained unchanged. In the spleen, a compensatory organ during hematopoiesis stress, a 2.83-fold higher frequency and 3.35-fold higher number of erythroid progenitors were observed in the spleen at day 3 (see Table 9). The frequencies and total number of progenitors in the bone marrow appeared normal at day 5 but fewer mature erythroid progenitors (CFU-E) and more primitive progenitors (BFU-E/mix) were observed at day 7. A similar reduction in CFU-E was observed in spleens at day 5; otherwise, the frequencies and total numbers of progenitors increased from days 3-7.

### EXAMPLE 11

### D1-FRIL protects mice from 5-FU induced death in the critical first week

A dose regimen was established to determine whether FRIL protects mice from death resulting from hematopoietic toxicity of Ara-C and Dox. This murine 5-FU chemoprotection model is based on studies showing that a single dose of 5-FU (150 mg/kg) resulted in >90% reduction of bone marrow cellularity but had limited cytotoxic effect on stem cells (Lerner and Harrison, *Exp. Hematol*. 18:114-118,1990). This finding was consistent with the understanding that stem cells reside in the bone marrow in predominantly a quiescent state. Bone marrow cellularity in these mice was restored after 2 weeks by the recruitment of the dormant progenitors and responsive stem cells that escaped toxicity of 5-FU. Administration of a second dose of 5-FU (also at 150 mg/kg) 3□7 days after the initial dose killed those stem cells and progenitors recruited into S-phase in response to the first treatment of 5-FU (Lerner and Harrison, *supra).*

de Haan et al. (*Blood* 87:4581-4588., 1996) applied this model to test whether prophylactic treatment of mice with hematopoietic regulators, pegylated SCF + IL11, could expand the stem cell and primitive progenitor compartments and better protect mice from death by 5-FU induced hematopoietic toxicity. These experiments demonstrated that although SCF + IL11 pretreatment could accelerate hematopoietic recovery after it was underway by 4 days □ 5 days when compared to controls (from approximately 11 days to 7 days for 40% survival, the SCF + IL11 cytokine pretreatment strategy did not rescue mice from death when the second dose of 5-FU was administered in the critical first week when stem cells are ablated (de Haan et al. *(supra).*

In this study described below, the pretreatment dose regimen for Dl-FRIL described above (5 mg/kg x 4 days) was selected based on the requirement of treating animals with cytokines for several days prior to starting chemotherapy and because Dl-FRIL-treated mice easily tolerated doses (5 mg/kg) that were 10- to 100-fold greater than that used for cytokines. Since FRIL and cytokines act on progenitors in the same concentration range (ng/ml), this pretreatment dose regimen was used to test whether Dl-FRIL can protect mice from 5-FU administered at 2 intervals in the first week: 5-FU (150 mg/kg) was injected at day 0 and then a second dose (also at 150 mg/kg) was injected either on day 3 (d0/3 dose interval) or day 5 (d0/5 dose interval). No survival on the day 3 interval was observed by de Haan et al. *(supra)* at day 3.

D1-FRIL was purified from *Dolichos lab lab* as described above in Example 1.

Weight-matched BALB/c mice (10 mice/group) were injected intravenously with either with 0.2 mL of Dl-FRIL (500 mg/mL) or 0.2 mL of HBSS daily for 4 days. Two hours after the final treatment of Dl-FRIL, mice were injected intraperitoneally with 5-FU (150 mg/kg). Groups of mice received a second dose of 5-FU (150 mg/kg) at either day 3 or day 5. No mice died from a single dose of 5-FU.

As shown in Table 10, Dl-FRIL pretreatment improved survival of mice in two separate experiments.

**TABLE 10**

| | | **5-FU Dose Interval** | | | |
|---|---|---|---|---|---|
| **Exp.** | **Mice** | **D0/3** | | **D0/5** | |
| | | **FRIL** | **HBSS** | **FRIL** | **HBSS** |
| 1 | Males, 16 wk | 3/10 | 0/10 | N.T. | N.T. |
| 2 | Females, 8 wk | 0/10 | 0/10 | 4/10 | 1/20 |
| Improved survival of mice pretreated with FRIL (5mg/kg x 4 days) prior to undergoing 5-FU dose intervals of d0/3 and d0/5. | | | | | |

In the first experiment, 3 of 10 mice survived a d0/3 dose interval of 5-FU compared to no mice in the HBSS pretreatment control. In the second experiment, 4 of 10 mice pretreated with Dl-FRIL survived a d0/5 dose interval of 5-FU compared to 1 of 10 for HBSS pretreated mice.

### EXAMPLE 12

### Optimization of the dose regiment of a FRIL family member to protect mice from 5-Fu induced death

FRIL family members are relatively abundant in legumes. For example, Dl-FRIL accounts for approximately 0.02% of the mass of hyacinth beans.

D1-FRIL is purified by carbohydrate affinity chromatography as described in Example 1, and is evaluated for purity by SDS-PAGE (5 discrete bands are visualized on an overloaded gel; see Fig. 37); is analyzed for mass and composition by amino acid analysis; and is assayed in the cord blood progenitor assay described above.

The murine 5-FU chemoprotection model (Lerner and Harrison, *supra;* de Haan, *supra)* is used to empirically derive the optimal dose regimen of Dl-FRIL to protect mice from death. Dl-FRIL is administered intravenously to mice over a 3-log dose range (5 - 5,000 mg/kg) under various regimens that include Dl-FRIL treatment prior to chemotherapy (daily from 3 days to 2 hour before initiation of chemotherapy) and during chemotherapy.

The mice used in these studies are BALB/c female mice, 8-10 weeks at outset of experiments (Jackson Laboratory, Bar Harbor, ME), weight matched each for experiment, where there are 10 mice per group. Organs from mice receiving a dose of 5,000 mg/kg of FRIL (with no 5-FU treatment) are collected for toxicity studies

The five doses of D1-FRIL are 0, 5, 50, 500, and 5,000 µg/kg. The four dose regimens of D1-FRIL will be -2hour; -1day and -2hour; -2day, -1day, and -2hour; - 3day, -2day, -1day, and -2hour prior to 5-FU treatment. The two maintenance regimens are either daily x 7 day (-2hour to day 7) or every other d (days 0, 2, 4, 6). Thus, one group of mice will receive a dose of Dl-FRIL daily for 7 days; while the second group will receive a dose of D1-FRIL every other day for 7 days.

The 5-FU dose intervals of 150 mg/kg are at dose intervals of d0/3, d0/5, and d0/7.

### EXAMPLE 13

### A FRIL family member has chemoprotective properties with widely used cell cycle-active chemotherapeutics

After establishing the optimal dose regimen of a FRIL family member, the FRIL family member's ability to protect mice from death by cytarabine (Ara-C) and doxorubicin.

Initial dose regimens of cytarabine (Ara-C) and doxorubicin are as follows: Doxorubicin □ 14 mg/kg as single bolus i.p. injection (Grzegorzewski et al., *J.Exp.Med.* 180:1047-1057,1994); Ara-C - 300 mg/kg at time as an i.p. injection at 0 and 12 hours (Paukovits et al., *Blood* 77:1313-1319,1991). Further studies are based on targeted clinical indication

### EXAMPLE 14

### Characterization of the hematopoietic status of mice during optimal dose regimen of a FRIL family member to protect mice from 5-FU induced death

Peripheral blood counts and the status of hematopoietic progenitors (frequency, total number, and cycling status) are characterized in mice during and after receiving the optimal dose regimen of a FRIL family member.

To do this, mice injected with a dose regimen of a FRIL family member with no 5-FU treatment are evaluated daily during and for one week after treatment with the FRIL family member. The mice are evaluated for the following hematopoietic parameters: WBC and RBC counts; bone marrow and spleen cellularities; and progenitor status, which includes hematopoietic colony assays (StemCell Technologies). The progenitors assayed are myeloid (CFU-C), erythroid (CFU-E), and primitive, multipotential (BFU-E/Mix). The frequencies and total numbers are determined, as well as the cycling status of these cells, as measured by ³H-thymidine suicide assay (Moore et al., *Exp. Hematol*. 14:222-229,1986).

### EXAMPLE 15

### Analysis of pharmacology and toxicology of FRIL in mice

The clearance of a FRIL family member from the circulation and its accumulation in the body is determined by preliminary pharmacokinetics.

For these studies, ¹²⁵I-FRIL is injected into mice (dosage of FRIL based on optimization results). Clearance of FRIL from the blood is evaluated at the following timepoints after injection: 5 min., 15 min., 30 min., 1 hour, 2 hours, 4 hours, 8 hours, 12 hours, 36 hours, 48 hours. Two mice are evaluated at each timepoint. Following this study, the animals are sacrificed and the organs collected and evaluated.

Dose-range finding experiments determine the maximal tolerated dose of a FRIL family member by various routes of administration (intravenous, intraperitoneal, subcutaneous, and oral). To do this dose range finding study, four routes at four doses are used. The routes of administration are intravenous, intraperitoneal, subcutaneous, oral. The highest dose of a FRIL family member is 1 g/kg. The dosage of the FRIL family member is reduced by 2-fold until mice survive. Survival is measured at 48 hours after treatment. Other clinical observations are made, including behavorial, lethargy, vocalization, diarrhea. CBC analyses are made. The animals are evaluated for any necropsy *(i.e.,* gross lesions). Following this study, the animals are sacrificed and the organs collected and evaluated.

Acute dose toxicity studies allow identify target organs that may develop lesions after exposure to a FRIL family member. For these acute dose toxicity studies, a dose is selected, and a FRIL family member is injected daily for 7 days. Acute toxicity is evaluated at 7 days, and recovery from acute toxicity is evaluated at 21 days. Blood chemistries, target organs, bone marrow and blood, and other health indicator are evaluated.

Hypersensitivity studies in guinea pigs are performed to test for any adverse immunologic reactions. To do this, fifteen guinea pigs (5 FRIL, 5 DNCB positive control, 5 saline negative control) are used. A FRIL family member is intradermally injected at 0.1 mL. Daily clinical observations at site for redness and edema are compared to the DNCB positive control. The FRIL guinea pigs are challenged at at 2 weeks with 0.05 mL of the FRIL family member, and daily clinical observations are made.

A determination of development of mouse anti-FRIL family member antibodies in mice receiving treatment with a FRIL family member is made to determine the extent and nature of the body's response to a FRIL family member. To do this, a FRIL family member is attached to Dynal's tosylactivated magnetic beads. The FRIL family member-coated beads are incubated with plasma (pooled or individual) from a mouse who has received treatment with the FRIL family member. Using rabbit and rat antiserum to FRIL used as positive control, the presence of FRIL family member-specific antibodies is evaluated by SDS-PAGE and Western blot analysis (horseradish peroxidase or chemiluminesce). Lastly, a determination is made as to whether sugar blocks antibody binding (α-D-mannopyranoside and negative control).

### EXAMPLE 16

### Purification of Progenitor Cells using D1-FRIL-Coated Magnetic Beads

Using magnetic beads coated with a non-limiting FRIL family member, Dl-FRIL, a population of progenitor cells was isolated and characterized. To do this, the following methods were used.

### Preparation of FRIL-beads for cell isolation

D1-FRIL was purified from *Dolichos lab lab* seeds as described in Example 1. D1-FRIL can be immobilized on magnetic beads (M-280 Dynabeads Tosylactivated, Lake Success, NY) via amino- and sulfhydryl-groups of the lectin according to the manufacturer's directions. Dl-FRIL can also immobilized on magnetic beads by a biotin-strepavidin interaction.

In this example, Dl-FRIL was immobilized on magnetic beads by a biotin-strepavidin interaction. Biotinylation of Dl-FRIL via primary amine-groups (EZ-Link Sulfo-NHS-LC-LC-Biotin, Pierce Chemical Company, Rockford, IL) was carried out according to the manufacturer's directions: Biotinylated Dl-FRIL was incubated with strepavidin-labeled magnetic beads (Dynal or Miltenyi Biotec, Auburn, CA) according to the manufacturer's directions.

### Preparation of cells

Human cord blood (CB), peripheral blood, and bone marrow, collected in sterile receptacles containing anticoagulant (e.g., heparin, EDTA), was processed to isolate mononuclear cells (mnc) within six hours of collection by density centrifugation on Ficoll-Paque PLUS (Pharmacia Biotech, Piscataway, NJ) according to the manufacturer's directions. Mononuclear cells harvested at the interface of plasma and Ficoll-Paque were washed and resuspended in serum-defined medium (e.g., XVIVO-10, Biowhittaker, Walkerville, MD or AIM-V, Life Technologies, Rockville, MD).

### Dl-FRIL-bead cell isolation

Dl-FRIL-coated beads specifically bound a minor mnc population found in CB, peripheral blood, and bone marrow. A ten-fold excess of Dl-FRIL-beads was incubated with the cells. For CB, where Dl-FRIL-beads captured approximately 1% of mnc, the ratio of beads to cells was 1:10, or 10-fold greater number of beads for every target cell. The ratio for other FLT3-expressing cell populations, was hematopoietic and non-hematopoietic, was experimentally determined by serial exposure of cells to fresh Dl-FRIL-beads.

Dl-FRIL-beads were washed twice in serum-defined medium prior to use. An aliquot of Dl-FRIL-beads was added to 10 mLof serum-defined medium in a 15 mL conical centrifuge tube (Falcon, Becton-Dickinson, Lincoln, NJ), mixed, and placed in a magnet (Dynal or Miltenyi Biotec, depending on source of magnetic beads) for ten minutes. Medium was aspirated with a 10 mL pipette without disturbing beads bound to walls of centrifuge tube by the magnet charge from the magnet. After washing, 0.5 mL of serum-defined medium was added to the tubes to wash the beads from the walls to the bottom of the conical tube. Medium was added to beads in a small volume (<2 mL) and the centrifuge tube was tumbled on a rocker in a cold room (*i.e.*, at approximately 4°C) for one hour. After incubation, serum-defined medium was added to a final volume of 10 mL and the tube was placed in the magnet for ten minutes. Medium was removed by aspiration without disturbing cells bound to Dl-FRIL-beads on the walls of the centrifuge tube via the magnetic charge. Cells were washed a second time by removing the conical tube from the magnet, adding 10 mL of serum-defined medium, mixing cells, and placing the conical tube back onto the magnet. Following aspiration of the medium, the final volume was adjusted to 2 mL.

### Detachment of Dl-FRILbeads from cells

For some applications, detachment of D1-FRIL-beads from cells is preferred. About half of D1-FRIL-beads detached from CB mnc after overnight incubation on a rocker in the cold room. Although binding studies have demonstrated that excess mannose and α-methyl α-D-mannoside prevent Dl-FRIL from binding to Flt3, neither sugar released tightly bound Dl-FRIL-beads from CB mnc. To remove Dl-FRIL-beads from this subpopulation of cells, the cells were incubated in 100 mM trehalose (Sigma, St. Louis, MO) for one hour on a rocker in the cold room.

It should be noted that since Miltenyi beads are very small (approximately 50 nm) as compared to Dynal beads (approximately 10 µm), when Miltenyi beads were used to purify D1-FRIL-binding progenitor cells, the beads were allowed to remain attached to the purified progenitor cells.

### Receptor tyrosine kinase gene expression

Receptor tyrosine kinase gene expression was characterized by RT-PCR.

Using these methods, the following results were obtained:

### Functional properties of Dl-FRIL bead-selected CB mnc

The progenitor capacity of Dl-FRIL-selected CB mnc was tested in a methylcellulose colony assay under conditions to promote proliferation and differentiation along either the hematopoietic or endothelial lineages (as described above. Table 11 shows the number of hematopoietic colonies (myeloid, erythroid, and mix) and endothelial colonies (other) that formed after culture of unselected cells (CB mnc), Dl-FRIL-selected cells (DI-FRIL⁺) and CB mnc that did not bind to DI-FRIL-beads (DI-FRIL⁻).

**TABLE 11**

| Response of D1-FRIL-selected cells to hematopoietic and endothelial stimuli | | | | | | |
|---|---|---|---|---|---|---|
| Stimulator | | Myeloid | Erythroid | Mix | Other | Total |
| **+ CSFs** | CB mnc | 1 | 3 | 2 | 0 | 6 |
| | D1-FRIL⁺ | 14 | 10 | 4 | 0 | 28 |
| | D1-FRIL⁻ | 1 | 1 | 2 | 0 | 4 |
| **+ VEGF** | CB mnc | 0 | 0 | 0 | 2 | 2 |
| | D1-FRIL⁺ | 0 | 0 | 0 | 19 | 19 |
| | D1-FRIL⁻ | 0 | 0 | 0 | 5 | 5 |
| Cord blood mononudear cells were isolated by Ficoll-Paque, bead selected, and plated in MethoCult□, StemCell Technologies, Vancouver, BC, Canada). | | | | | | |

As shown in Table 11, D1-FRIL-selection increased the number of hematopoietic colonies (stimulated with colony stimulating factors (CSFs) by 14-fold for myeloid colonies, 3.3- to 10-fold for erythroid colonies (CB mnc and Dl-FRIL- cells, respectively), and by 2-fold for mixed colonies. Similar levels of Dl-FRIL-bead enrichment was observed for endothelial colonies (stimulated with vascular endothelial growth factor (VEGF)): 9.5-fold over CB mnc and 3.8-fold for Dl-FRIL⁻ cells.

### Cell surface phenotypic properties of Dl-FRIL bead-selected CB mnc

The cell surface phenotypic properties of Dl-FRIL bead-selected CB mnc was characterized by flow cytometry. Table 12 shows the phenotypes (by percentage of cells expressing the indicated cell surface phenotype marker) of the three CB cell populations: (1) cells not selected by Dl-FRIL-beads (Dl-FRIL'); (2) cells that detached from Dl-FRIL-beads after overnight incubation in the coldroom on a rocker (Dl-FRIL⁺); and (3) cells that retained Dl-FRIL-beads after overnight incubation (Dl-FRIL⁺⁺). The two Dl-FRIL-binding cell populations were analyzed separately to see whether tightness of binding (avidity) related to type of cells selected. Isotype control levels were set at 2%; all values of 2% represent no reactivity with test antibody.

**TABLE 12**

| Flow cytometric analysis of D1-FRIL-selected CB mnc | | | | |
|---|---|---|---|---|
| Antigen | Cell Type | D1-FRIL⁻ (%) | D1-FRIL⁺ (%) | D1-FRIL⁺⁺(%) |
| CD3 | Mature T | 26 | 35 | 6 |
| CD11b | Mac-1, CR3 | 19 | 35 | 67 |
| CD11c | LeuCAMc | 10 | 22 | 32 |
| CD13 | Pan myeloid, CFU-GM | 5 | <2 | <2 |
| CD19 | Pan B | 4 | 5 | 12 |
| CD32 | Low affinity IgG Fcγ-R | 5 | 19 | 26 |
| CD33 | Myeloid progenitors | 3 | 2 | 8 |
| CD34 | Pan progenitors | <2 | <2 | <2 |
| CD38 | Activated T | 88 | 96 | 93 |
| CD42a | Platelet gpIX | 5 | 2 | 7 |
| CD69 | Early activation ag (EA-1) | 6 | 8 | 14 |
| CDw90 | Thy-1, progenitor subset | 8 | 14 | 13 |
| CD117 | c-kit, progenitors | 4 | 2 | 2 |

As shown in Table 12, Dl-FRIL-beads did not capture CB mnc that express CD34, the hallmark marker of hematopoietic stem cells and progenitors. This observation was unexpected because Dl-FRIL-selected cells enrich for progenitors (see Table 11). Although CB CD34⁺ cells uniformly express FLT3, only 70% of Flt3 ⁺ CB mnc also express CD34 (Rappold et al., *Blood* 90:111-125,1997). Consequently, 30% of CB mnc expressed the phenotype of CD34⁻Flt3⁺. Dl-FRIL-beads appeared to capture this latter population of cells.

Cells expressing dendritic cell (DC) markers, CD11b and CD11c, were enriched approximately 2-fold in the D1-FRIL⁺ cell population and over 3-fold in the D1-FRIL⁺⁺ cell population (Table 12). This observation is consistent with reports that Flt3 is involved in dendritic cell proliferation and maturation in mice (Pulendran et al.,*J. Immunol*. 159:2222-2231,1997) and humans (Miller et al., *Blood* 93:96-106., 1999). The rare hematopoietic dendritic cell population is useful in inducing tumor regression and for the treatment of AIDS.

Differences of the cell surface phenotypes were observed between D1-FRIL⁺ and D1-FRIL⁺⁺ CB cells (see Table 11). The percentage of CD3 T cells decreased from 35% for D1-FRIL⁺ cells to 6% for D1-FRIL⁺⁺ cells. Conversely, the percentage of CD11b⁺ cells and CD11c⁺ cells increased from 35% to 67% and from 22% to 32% for D1-FRIL⁺ and D1-FRIL⁺⁺ cell populations, respectively.

Cells that retained D1-FRIL-beads after overnight incubation on a rocker in the cold room (Dl-FRIL⁺⁺ cells) were observed as single cells or as clumps of bead-bound cells. These clumps could not be disrupted either by mechanical means or by elution with competing sugars, mannose or mannose derivatives (data not shown). From studies to characterize the carbohydrate-binding properties of Dl-FRIL, α,α-trehalose demonstrated a 3.6-fold greater potency than mannose and a 1.6- to 2.1-fold greater potency than α-methyl α-D-mannoside derivatives that were tested (Mo et al., Glycobiology 9:173-179,1999). Incubation of dumped Dl-FRIL-bead bound cells with 100 mM Trehalose effectively disrupted the clumped cells and removed most of the Dl-FRIL-beads from cells.

Two populations of trehalose-disrupted Dl-FRIL⁺⁺ cells were analyzed by flow cytometry: cells that no longer bound beads (Dl-FRIL⁺⁺) and cells that still retained beads following incubation with trehalose (Dl-FRIL⁺⁺⁺). The results of one experiment are shown in Table 13.

**TABLE 13**

| Flow cytometric analysis of Dl-FRIL-selected CB mnc after exposure to trehalose | | | |
|---|---|---|---|
| Antigen | Cell Type | D1-FRIL⁺⁺ (%) | D1-FRIL⁺⁺⁺(%) |
| CD3 | Mature T | 6 | 3 |
| CD11b | Mac-1, CR3 | 52 | 76 |
| CD11c | LeuCAMc | 16 | 43 |
| CD13 | Pan myeloid, CFU-GM | 72 | 46 |
| CD19 | Pan B | 5 | 9 |
| CD32 | Low affinity IgG Fc-γ | 43 | 19 |
| CD33 | Myeloid progenitors | 56 | 15 |
| CD34 | Pan progenitors | 2 | 2 |
| CD117 | c-kit, progenitors | 2 | 12 |
| CD135 | Flt3, progenitors | 2 | 5 |

The difference in cell surface phenotypes between D1-FRIL⁺⁺ cells and D1-FRIL⁺⁺⁺ cells in Table 13 was greater than those observed for D1-FRIL⁺ cells and D1-FRIL⁺⁺ cells in Table 12. In Table 13, the percentage of CD3, CD13, CD32, and CD33 cells decreased by 1.6- to 3.7-fold in D1-FRIL⁺⁺⁺ cells compared to D1-FRIL⁺⁺ cells. Conversely, the percentage of CD11b, CD11c, CD19, CD117, and CD135 cells increased by 1.5- to 6-fold in D1-FRIL⁺⁺⁺ cells compared to D1-FRIL⁺⁺ cells. Again no CD34 was observed in either cell population.

The increase in percentage of cells that express CD117 and CD135, two tyrosine kinase receptors central to hematopoietic stem cell and progenitor function (Lyman and Jacobson, *Blood* 91:1101-1134,1998), suggested that avidity of Dl-FRIL-bead binding of cells might correspond to the primitive status of the cells. The studies described herein which characterize the carbohydrate binding properties of Dl-FRIL supported this notion. Dl-FRIL has neither an extending carbohydrate combining-binding site nor a hydrophobic binding site adjacent to it (Mo et al., *Glycobiology* 9:173-179,1999). Dl-FRIL binds most tightly to a trimannoysl structure that is the basis for N-linked glycosylation in mammals. Consequently, Dl-FRIL may bind cells that have undergone less processing of glycosylation, which is consistent with more primitive cells. This property of D1-FRIL-binding may provide a unique method to isolate primitive cells not currently possible by antibodies to CD34. Thus, Dl-FRIL binds the normally glycosylated FLT3 receptor more tightly than the FLT3-Ligand binds to normally glycosylated FLT3. Dl-FRIL binds normally glycosylated FLT3 receptor more tightly than the typical antibody binds its specific ligand.

### Receptor tyrosine kinase gene expression in Dl-FRIL-selected CB cells

The number of cell surface receptors and markers increases as pluripotent hematopoietic stem cells proliferate and differentiate. The number of functional receptors on the most primitive cells is probably less than ten. The levels of detection for flow cytometry are probably in the range of several hundred cell surface molecules. Consequently, analysis of primitive cell populations cannot be analyzed by flow cytometry.

The presence or absence of functional tyrosine kinase receptors on primitive cells was further characterized by RT-PCR. Expression of Flt3, Kit, Fms, Flk1, Flt1, and Flt4 mRNA was determined for CB mnc, CD34-selected cells, and Dl-FRIL-selected cells. For CB mnc, the PCR products for the receptors were either faint or not detectable. The pattern of gene expression for cells selected by CD34-beads or Dl-FRIL-beads was the same; all tyrosine kinase receptors showed stronger PCR signals. These data suggest that receptors associated with stem cells (Flt3 and Kit) and primitive endothelial cells (Flk1, Flt1, and Flt4) are also detected in Dl-FRIL selected cells.

### EXAMPLE 17

### Use of beads coated with a FRIL family member to isolate CD34⁻ primitive stem cells

A rare human stem cell population with the phenotype of CD34⁻CD38⁻Lin has been identified by its ability to establish multilineage engraftment in NOD/SCID mice (Bhatia et al., *Nat. Med.* 4:1038-1045,1998). These repopulating cells give rise to stem cells that express the hallmark CD34 marker. Isolating CD34 CD38⁻Lin⁻ cells is labor-intensive methods of negative selection that includes the use of immunomagnetic beads and flow cytometry to deplete cells that express CD34, CD38, and lineage markers. Rapid, efficient, positive selection of CD34⁻CD38⁻Lin⁻ cells would be preferable for clinical uses. However, the absence of the highly expressed CD34 marker and low number of functional receptors on this rare population of cells will prevent use of antibodies for cell isolation.

FRIL attached to magnetic beads are used in a unique method to isolate the rare CD34⁻CD38⁻Lin⁻ cell population by binding primitive cells that express this phenotype. Isolation of CD34⁻CD38⁻Lin⁻ is achieved by a single-step cell isolation. However, since FRIL-beads also recognize cells that express CD11b, CD11c, and CD38, optimal isolation of CD34⁻CD38⁻Lin⁻ cells is improved by first negatively selecting unwanted cells by immunomagnetic beads that bind to CD11b, CD11c, and/or CD38.

### EXAMPLE 18

### Use of beads coated with a FRIL family member to isolate normal stem cells from patients with leukemia

A majority of leukemias express the phenotype of CD34⁺Flt3⁺ (Carow et al., *Blood* 87:1089-1096, 1996). Consequently, methods that rely on CD34 expression cannot distinguish normal stem cells and progenitors from leukemic cells in the bone marrow and peripheral blood of patients.

FRIL does not interact with two cell leukemic lines tested with the CD34⁺Flt3⁺ phenotype (KG1-A and ML-1). FRIL neither effects growth of these leukemic cell lines nor do FRIL-beads capture appreciable numbers of cells (data not shown). Since FRIL-beads select normal progenitors with the phenotype of CD34⁻Flt3⁺, FRIL-beads provide a unique method that distinguishes between normal and leukemic cells.

A FRIL family member attached to magnetic beads is used to isolate normal hematopoietic stem cells and progenitors from the bone marrow and peripheral blood of leukemia patients. This is accomplished using a method similar to leukopheresis, where blood is passed through a device that retains cells of interest. In this example, FRIL-beads binds CD34⁻Flt3⁺ normal cells. Since FRIL also interacts with Flt3-expressing CD11b and CD11c cells, prior exposure and removal of the cells that immunomagnetic beads that bind to CD11b and/or CD11c (*i*.*e*., negative selection) may permit enrichment of primitive cells.

### EXAMPLE 19

### Use of FRIL-beads to isolate dendritic progenitors and mature cells from normal individuals

Dendritic cells (DC) are immune cells that capture antigens and initiate T cell-mediated immune responses (Banchercau and Steinman, *Nature* 392:245-252, 1998). DC act as first lines of defense in the skin, gut, and lymphoid organs. Antigens on DC can activate naive and quiescent T cells and small numbers of DC pulsed with lose dosages of antigens stimulate strong T cell responses. Under certain circumstances, DC also induce T cell tolerance. Consequently, the unique properties of DC has generated significant interest to use these cells to treat cancer and AIDS. DC are derived from CD34⁺progenitors in the bone marrow of humans. The cytokines GM-CSF, TNF-α, and Flt3 ligand (FL) influence DC development (Banchereau and Steinman, *supra;* Pulendran et al., *J. Immunol.* 159:2222-2231, 1997). Injection of FLT3-Ligand in mice dramatically increases the number of DC (Pulendran et al., *supra*).
FRIL interacts with the Flt3 receptor on DC, and FRIL-beads capture cells with the dendritic phenotype of CD11b and CD11c. Selecting dendritic cells with FRIL-beads from human bone marrow, peripheral blood, or cord blood allows the efficient and effective isolation of DC for clinical use.

### EXAMPLE 20

### Use of FRIL-beads to isolate endothelial stem cells and progenitors

Endothelial stem cells and progenitors give rise to cells that form blood vessels in a process called angiogenesis. During strokes and heart attacks, new blood vessels are needed to repair damage. Activation of endothelial stem cells and progenitors to produce more mature cells is mediated by the cytokines that activate the Flk1/KDR, Flt1, and Flt4 tyrosine kinase receptors. Flt3 is expressed on very primitive endothelial progenitors. FRIL-beads are used to capture a population of cells from cord blood that express all of these receptors.

### EXAMPLE 21

### Use of FRIL family members and Non-FRIL family member lectins to alter signal transduction and other cellular pathways

Drugs designed to alter signal transduction pathways need to specifically distinguish target cells. FRIL is used as a targeting vehicle to deliver small molecules to Flt3-expressing cells such as stem cells, progenitors, and dendritic cells. FRIL has several advantages for drug delivery: 1) FRIL specific for Flt3; 2) FRIL is stable in the cytoplasm; 3) FRIL is capable of undergoing conjugation with small molecules; 4) FRIL can be delivered in dose-responsive manner; and 5) FRIL provides specificity for overlapping pathways of signal transduction.

Other legume- or bulb-derived lectins can also delivery small molecule drugs to specific cell populations. For example, the lectins PHA and ConA both bind to CD3-T cell receptor complex and the FC-gamma receptor (CD32) (Leca et al. *Scand. J. Immunol*. 23:535-544,1986); UDA binds the Vβ domain of the T cell receptor (Galelli et al., *J. Immunol.* 151:1821-1831,1993).

Standard methods of conjugation are used to attach small molecules, oligos, or enzymes to plant lectins.

### EXAMPLE 22

### Purification and Cloning of YamFRIL from Sphenostylis stenocarpa

Dry seeds of Yam bean *(Sphenostylis stenocarpa)* were ground in a coffee mill and the powder was extracted with 5 volumes of 10 mM Na Acetate buffer, pH 5.2, containing 1 mM CaCl₂ for 1 hour at 4°C. After centrifugation, the clear supernatant was neutalized with Tris-HCl pH 8.0.

YamFril purification was achieved through and absorption on a ovalbumin gel affinity column (Sigma) and was eluted with 200 mM trehalose.

The resulting protein was fractionated into 2 polypeptides that were submitted to N-terminal amino acid sequences.

Beta band:          AQSVSFTFTKFDSDQ  (SEQ ID NO: 9)

Alpha band:     AASNNVVAVEFDTXPN  (SEQ ID NO: 10)

Reverse-transcriptase PCR was performed on total RNA obtained from International Institute of Tropical Agriculture (Ibadan, Oyo State, Nigeria), using degenerate primers based on the alpha and beta N-terminus sequences (*i.e.*, SEQ ID NO: 10 and SEQ ID NO: 9, respectively).

3' RACE PCR was performed on total and polyA+ RNA using gene specific primers with an Anchor Primer.

Partial cDNA clones were obtained and the following sequences deduced.

SEQUENCE LISTING

<110> IMCLONE SYSTEMS INCORPORATED

THE REGENTS OF THE UNIVERSITY OF CALIFORNIA

<120> PROGENITOR CELL PRESERVATION FACTORS AND RELATED
METHODS AND PRODUCTS

<130> 115-85 EPT2

<150> EP 99 967 798.2

<151> 1999-12-30

<150> PCT/US99/31307

<151> 1999-12-30

<160> 61

<170> PatentIn version 3.0

<210> 1

<211> 939

<212> DNA

<213> Artificial Sequence

<220>

<223> D1-FRIL.

<400> 1

gcacagtcat tgtcatttag tttcaccaag tttgatccta accaagagga tcttatcttc 60

caaggtcatg ccacttctac aaacaatgtc ttacaagtca ccaagttaga cagtgcagga 120

aaccctgtga gttctagtgc gggaagagtg ttatattctg caccattgcg cctttgggaa 180

gactctgcgg tattgacaag ctttgacacc attatcaact ttgaaatctc aacaccttac 240

acttctcgta tagctgatgg cttggccttc ttcattgcac cacctgactc tgtcatcagt 300

tatcatggtg gttttcttgg actctttccc aacgcaaaca ctctcaacaa ctcttccacc 360

tctgaaaacc aaaccaccac taaggctgca tcaagcaacg ttgttgctgt tgaatttgac 420

acctatctta atcccgatta tggtgatcca aactacatac acatcggaat tgacgtcaac 480

tctattagat ccaaggtaac tgctaagtgg gactggcaaa atgggaaaat agccactgca 540

cacattagct ataactctgt ctctaaaaga ctatctgtta ctagttatta tgctgggagt 600

aaacctgcga ctctctccta tgatattgag ttacatacag tgcttcctga atgggtcaga 660

gtagggttat ctgcttcaac tggacaagat aaagaaagaa ataccgttca ctcatggtct 720

ttcacttcaa gcttgtggac caatgtggcg aagaaggaga atgaaaacaa gtatattaca 780

agaggcgttc tgtgatgata tatgtgtatc aatgattttc tatgttataa gcatgtaatg 840

tgcgatgagt caataatcac aagtacagtg tagtacttgt atgttgtttg tgtaagagtc 900

agtttgcttt taataataac aagtgcagtt agtacttgt 939

<210> 2

<211> 264

<212> PRT

<213> Artificial Sequence

<220>

<223> D1-FRIL.

<400> 2

Ala Gln Ser Leu Ser Phe Ser Phe Thr Lys Phe Asp Pro Asn Gln Glu

1               5                   10                  15

Asp Leu Ile Phe Gln Gly His Ala Thr Ser Thr Asn Asn Val Leu Gln

            20                  25                  30

Val Thr Lys Leu Asp Ser Ala Gly Asn Pro Val Ser Ser Ser Ala Gly

        35                  40                  45

Arg Val Leu Tyr Ser Ala Pro Leu Arg Leu Trp Glu Asp Ser Ala Val

    50                  55                  60

Leu Thr Ser Phe Asp Thr Ile Ile Asn Phe Glu Ile Ser Thr Pro Tyr

65                  70                  75                  80

Thr Ser Arg Ile Ala Asp Gly Leu Ala Phe Phe Ile Ala Pro Pro Asp

                85                  90                  95

Ser Val Ile Ser Tyr His Gly Gly Phe Leu Gly Leu Phe Pro Asn Ala

            100                 105                 110

Asn Thr Leu Asn Asn Ser Ser Thr Ser Glu Asn Gln Thr Thr Thr Lys

        115                 120                 125

Ala Ala Ser Ser Asn Val Val Ala Val Glu Phe Asp Thr Tyr Leu Asn

    130                 135                 140

Pro Asp Tyr Gly Asp Pro Asn Tyr Ile His Ile Gly Ile Asp Val Asn

145                 150                 155                 160

Ser Ile Arg Ser Lys Val Thr Ala Lys Trp Asp Trp Gln Asn Gly Lys

                165                 170                 175

Ile Ala Thr Ala His Ile Ser Tyr Asn Ser Val Ser Lys Arg Leu Ser

            180                 185                 190

Val Thr Ser Tyr Tyr Ala Gly Ser Lys Pro Ala Thr Leu Ser Tyr Asp

        195                 200                 205

Ile Glu Leu His Thr Val Leu Pro Glu Trp Val Arg Val Gly Leu Ser

    210                 215                 220

Ala Ser Thr Gly Gln Asp Lys Glu Arg Asn Thr Val His Ser Trp Ser
225                 230                 235                 240

Phe Thr Ser Ser Leu Trp Thr Asn Val Ala Lys Lys Glu Asn Glu Asn
                245                 250                 255

Lys Tyr Ile Thr Arg Gly Val Leu
            260

<210> 3
<211> 1005
<212> DNA
<213> Artificial Sequence

<220>
<223> Nucleic acid sequence of the naturally-occurring
D1-FRIL protein.

<400> 3
atggcttcct ccaacttact caccctagcc ctcttccttg tgcttctcac ccacgcaaac 60

tcagccgcac agtcattgtc atttagtttc accaagtttg atcctaacca agaggatctt 120

atcttccaag gtcatgccac ttctacaaac aatgtcttac aagtcaccaa gttagacagt 180

gcaggaaacc ctgtgagttc tagtgcggga agagtgttat attctgcacc attgcgcctt 240

tgggaagact ctgcggtatt gacaagcttt gacaccatta tcaactttga aatctcaaca 300

ccttacactt ctcgtatagc tgatggcttg gccttcttca ttgcaccacc tgactctgtc 360

atcagttatc atggtggttt tcttggactc tttcccaacg caaacactct caacaactct 420

tccacctctg aaaaccaaac caccactaag gctgcatcaa gcaacgttgt tgctgttgaa 480

tttgacacct atcttaatcc cgattatggt gatccaaact acatacacat cggaattgac 540

gtcaactcta ttagatccaa ggtaactgct aagtgggact ggcaaaatgg gaaaatagcc 600

actgcacaca ttagctataa ctctgtctct aaaagactat ctgttactag ttattatgct 660

gggagtaaac ctgcgactct ctcctatgat attgagttac atacagtgct tcctgaatgg 720

gtcagagtag ggttatctgc ttcaactgga caagataaag aaagaaatac cgttcactca 780

tggtctttca cttcaagctt gtggaccaat gtggcgaaga aggagaatga aaacaagtat 840

attacaagag gcgttctgtg atgatatatg tgtatcaatg attttctatg ttataagcat 900

gtaatgtgcg atgagtcaat aatcacaagt acagtgtagt acttgtatgt tgtttgtgta 960

agagtcagtt tgcttttaat aataacaagt gcagttagta cttgt 1005

<210> 4

<211> 22

<212> PRT

<213> Artificial Sequence

<220>

<223> Signal sequence from the FRIL family isolated from

Dolichos lab lab

<400> 4

Met Ala Ser Ser Asn Leu Leu Thr Leu Ala Leu Phe Leu Val Leu Leu

1               5                   10                  15

Thr His Ala Asn Ser Ala

            20

<210> 5

<211> 914

<212> DNA

<213> Artificial Sequence

<220>

<223> Pv-FRIL.

<400> 5

gctcagtcat tatcttttaa ctttaccaag tttgatcttg accaaaaaga tcttatcttc 60

caaggtgatg ccacttctac aaacaatgtc ttacaactca ctaagttaga cagtggagga 120

aaccctgtgg gtgctagtgt gggaagagtg ttattctctg caccatttca tctttgggaa 180

aactctatgg cagtgtcaag ctttgaaact aatctcacca ttcaaatctc aacacctcac 240

ccttattatg cagctgatgg ctttgccttc ttccttgcac cacatgacac tgtcatccct 300

ccaaattctt ggggcaaatt ccttggactc tactcaaacg ttttcagaaa ctcccccacc 360

tctgaaaacc aaagctttgg tgatgtcaat actgactcaa gagttgttgc tgtcgaattt 420

gacaccttcc ctaatgccaa tattgatcca aattacagac acattggaat cgatgtgaac 480

tctattaagt ccaaggaaac tgctaggtgg gagtggcaaa atgggaaaac ggccactgca 540

cgcatcagct ataactctgc ctctaaaaaa tcaactgtta ctacgtttta tcctgggatg 600

gaagttgtgg ctctctccca tgatgttgac ttacatgcag agcttcctga atgggttaga 660

gtagggttat ctgcttcaac tggagaggag aaacaaaaaa ataccattat ctcatggtct 720

ttcacttcaa gcttgaagaa caacgaggtg aaggagccga aagaagacat gtatattgca 780

aacgttgtgc gatcatatac atggatcaat gacgttctat cttatataag caataaataa 840

atgtatgatg cactcaataa taatcacaag tacgtacggt gtagtacttg tatgttgttt 900

atgaaaaaaa aaaa 914

<210> 6

<211> 279

<212> PRT

<213> Artificial Sequence

<220>

<223> Pv-FRIL.

<400> 6

Ala Gln Ser Leu Ser Phe Asn Phe Thr Lys Phe Asp Leu Asp Gln Lys

1               5                   10                  15

Asp Leu Ile Phe Gln Gly Asp Ala Thr Ser Thr Asn Asn Val Leu Gln

            20                  25                  30

Leu Thr Lys Leu Asp Ser Gly Gly Asn Pro Val Gly Ala Ser Val Gly

        35                  40                  45

Arg Val Leu Phe Ser Ala Pro Phe His Leu Trp Glu Asn Ser Met Ala

    50                  55                  60

Val Ser Ser Phe Glu Thr Asn Leu Thr Ile Gln Ile Ser Thr Pro His

65                  70                  75                  80

Pro Tyr Tyr Ala Ala Asp Gly Phe Ala Phe Phe Leu Ala Pro His Asp

                85                  90                  95

Thr Val Ile Pro Pro Asn Ser Trp Gly Lys Phe Leu Gly Leu Tyr Ser

            100                 105                 110

Asn Val Phe Arg Asn Ser Pro Thr Ser Glu Asn Gln Ser Phe Gly Asp

        115                 120                 125

Val Asn Thr Asp Ser Arg Val Val Ala Val Glu Phe Asp Thr Phe Pro

    130                 135                 140

Asn Ala Asn Ile Asp Pro Asn Tyr Arg His Ile Gly Ile Asp Val Asn

145                 150                 155                 160

Ser Ile Lys Ser Lys Glu Thr Ala Arg Trp Glu Trp Gln Asn Gly Lys

                165                 170                 175

Thr Ala Thr Ala Arg Ile Ser Tyr Asn Ser Ala Ser Lys Lys Ser Thr

            180                 185                 190

Val Thr Thr Phe Tyr Pro Gly Met Glu Val Val Ala Leu Ser His Asp

        195                 200                 205

Val Asp Leu His Ala Glu Leu Pro Glu Trp Val Arg Val Gly Leu Ser

    210                 215                 220

Ala Ser Thr Gly Glu Glu Lys Gln Lys Asn Thr Ile Ile Ser Trp Ser

225                 230                 235                 240

Phe Thr Ser Ser Leu Lys Asn Asn Glu Val Lys Glu Pro Lys Glu Asp

                245                 250                 255

Met Tyr Ile Ala Asn Val Val Arg Ser Tyr Thr Trp Ile Asn Asp Val

            260                 265                 270

Leu Ser Tyr Ile Ser Asn Lys

        275

<210> 7

<211> 678

<212> DNA

<213> Artificial Sequence

<220>

<223> YamFril partial mRNA sequence.

<400> 7

acgaagttcg acagcgacca aaaggatctt atgttccaag gtcataccat ttctagcagc 60

aatgtcatac aactcaccaa gttagacagt aatggaaacc ctgtgagtac cagtgtggga 120

agagtgttat actctgcacc attgcgcctt tgggaaagct ctacagtagt gtcaaccttt 180

gagaccactt tcacctttca aatctcaaca ccttacacta gtcctcctgg tgatgggctc 240

gccttcttcc ttgcaccata tgacactgtc atccctccaa attctgctgg caatcttctt 300

ggactctttc ctaacttaaa tgctttaaga aactccacca ccagtaaaga aaccactatt 360

gatgtcaatg ctgcatctaa caacgttgtt gccgttgaat ttgacaccta ccctaacgac 420

aatattggtg atccaagata caaacacatt ggaatcgatg tcaactctat caggtccaag 480

gcaactgttg cgtgggactg gcaaaatggg aaaacagcca ctgcacacat cagctataac 540

tctgcctcta aaagactatc tgttactact ttttatcctg ggggtaaagc tgtgagtctt 600

tcccatgacg ttgagctcac tcaagtgctt cctcaatgga ttagagtagg gttctctgct 660

tcaacaggat tagagaaa 678

<210> 8

<211> 234

<212> PRT

<213> Artificial Sequence

<220>

<223> YamFril deduced amino acid squence.

<400> 8

Ala Gln Ser Val Ser Phe Thr Phe Thr Lys Phe Asp Ser Asp Gln Lys

1               5                   10                  15

Asp Leu Met Phe Gln Gly His Thr Ile Ser Ser Ser Asn Val Ile Gln

            20                  25                  30

Leu Thr Lys Leu Asp Ser Asn Gly Asn Pro Val Ser Thr Ser Val Gly

        35                  40                  45

Arg Val Leu Tyr Ser Ala Pro Leu Arg Leu Trp Glu Ser Ser Thr Val

    50                  55                  60

Val Ser Thr Phe Glu Thr Thr Phe Thr Phe Gln Ile Ser Thr Pro Tyr

65                  70                  75                  80

Thr Ser Pro Pro Gly Asp Gly Leu Ala Phe Phe Leu Ala Pro Tyr Asp

                85                  90                  95

Thr Val Ile Pro Pro Asn Ser Ala Gly Asn Leu Leu Gly Leu Phe Pro

            100                 105                 110

Asn Leu Asn Ala Leu Arg Asn Ser Thr Thr Ser Lys Glu Thr Thr Ile

        115                 120                 125

Asp Val Asn Ala Ala Ser Asn Asn Val Val Ala Val Glu Phe Asp Thr

    130                 135                 140

Tyr Pro Asn Asp Asn Ile Gly Asp Pro Arg Tyr Lys His Ile Gly Ile

145                 150                 155                 160

Asp Val Asn Ser Ile Arg Ser Lys Ala Thr Val Ala Trp Asp Trp Gln

                165                 170                 175

Asn Gly Lys Thr Ala Thr Ala His Ile Ser Tyr Asn Ser Ala Ser Lys

            180                 185                 190

Arg Leu Ser Val Thr Thr Phe Tyr Pro Gly Gly Lys Ala Val Ser Leu

        195                 200                 205

Ser His Asp Val Glu Leu Thr Gln Val Leu Pro Gln Trp Ile Arg Val

    210                 215                 220

Gly Phe Ser Ala Ser Thr Gly Leu Glu Lys

225                 230

<210> 9

<211> 15

<212> PRT

<213> Artificial Sequence

<220>

<223> Beta band polypeptide.

<400> 9

Ala Gln Ser Val Ser Phe Thr Phe Thr Lys Phe Asp Ser Asp Gln

1               5                   10                  15

<210> 10

<211> 16

<212> PRT

<213> Artificial Sequence

<220>

<223> Alpha band polypeptide.

<220>

<221> MOD_RES

<222> (14)

<223> any amino acid.

<400> 10

Ala Ala Ser Asn Asn Val Val Ala Val Glu Phe Asp Thr Xaa Pro Asn

1               5                   10                  15

<210> 11

<211> 23

<212> DNA

<213> Artificial Sequence

<220>

<223> MLA degenerate oligonucleotide primer.

<220>

<221> misc_feature

<222> (3)

<223> a or g.

<220>

<221> misc_feature

<222> (6)

<223> t or c.

<220>

<221> misc_feature

<222> (9)

<223> t or c.

<220>

<221> misc_feature

<222> (12)

<223> a or t.

<220>

<221> misc_feature

<222> (15)

<223> t or c.

<220>

<221> misc_feature

<222> (18)

<223> a or g.

<220>

<221> misc_feature

<222> (21)

<223> a or g.

<400> 11

aanttnganc cnaancanga nga                                23

<210> 12

<211> 20

<212> DNA

<213> Artificial Sequence

<220>

<223> MLZ degenerate oligonucleotide primer.

<220>

<221> misc_feature

<222> (3)

<223> a or t.

<220>

<221> misc_feature

<222> (6)

<223> a or g.

<220>

<221> misc_feature

<222> (9)

<223> t or c.

<220>

<221> misc_feature

<222> (15)

<223> a or g.

<400> 12

ttnccnttnt gccantccca 20

<210> 13

<211> 15

<212> DNA

<213> Artificial Sequence

<220>

<223> primer.

<400> 13

gtaccgagct cggat                                        15

<210> 14

<211> 18

<212> DNA

<213> Artificial Sequence

<220>

<223> primer.

<400> 14

tctagatgca tgctcgag 18

<210> 15

<211> 22

<212> DNA

<213> Artificial Sequence

<220>

<223> MLX primer.

<400> 15

gttggacgtc aattccgatg tg 22

<210> 16

<211> 17

<212> DNA

<213> Artificial Sequence

<220>

<223> MLI degenerate primer.

<220>

<221> misc_feature

<222> (3)

<223> t or c.

<220>

<221> misc_feature

<222> (6)

<223> a or g.

<220>

<221> misc_feature

<222> (9)

<223> t or c.

<220>

<221> misc_feature

<222> (12)

<223> t or c.

<220>

<221> misc_feature

<222> (15)

<223> t or c.

<400> 16

gcncantcnc tntcntt 17

<210> 17

<211> 22

<212> DNA

<213> Artificial Sequence

<220>

<223> Oligo(dT) anchor primer.

<400> 17

gaccacgcgt atcgatgtcg ac 22

<210> 18

<211> 21

<212> DNA

<213> Artificial Sequence

<220>

<223> MLB primer.

<400> 18

aagttagaca gtgcaggaaa c 21

<210> 19

<211> 20

<212> DNA

<213> Artificial Sequence

<220>

<223> MLII primer.

<400> 19

gcacagtcat tgtcatttag                                        20

<210> 20

<211> 18

<212> PRT

<213> Artificial Sequence

<220>

<223> D1-FRIL.

<400> 20

Tyr Leu Asn Pro Asp Tyr Gly Asp Pro Asn Tyr Ile His Ile Gly Ile

1               5                   10                  15

Asp Val

<210> 21

<211> 19

<212> PRT

<213> Artificial Sequence

<220>

<223> Pea.

<400> 21

Phe Tyr Asn Ala Ala Trp Asp Pro Ser Asn Arg Asp Arg His Ile Gly

1               5                   10                  15

Ile Asp Val

<210> 22

<211> 1005

<212> DNA

<213> Artificial Sequence

<220>

<223> SpDLA.

<400> 22

atggcttcct ccaacttact caccctagcc ctcttccttg tgcttctcac ccacgcaaac 60

tcagccgcac agtcattgtc atttagtttc accaagtttg atcctaacca agaggatctt 120

atcttccaag gtcatgccac ttctacaaac aatgtcttac aagtcaccaa gttagacagt 180

gcaggaaacc ctgtgagttc tagtgcggga agagtgttat attctgcacc attgcgcctt 240

tgggaagact ctgcggtatt gacaagcttt gacaccatta tcaactttga aatctcaaca 300

ccttacactt ctcgtatagc tgatggcttg gccttcttca ttgcaccacc tgactctgtc 360

atcagttatc atggtggttt tcttggactc tttcccaacg caaacactct caacaactct 420

tccacctctg aaaaccaaac caccactaag gctgcatcaa gcaacgttgt tgctgttgaa 480

tttgacacct atcttaatcc cgattatggt gatccaaact acatacacat cggaattgac 540

gtcaactcta ttagatccaa ggtaactgct aagtgggact ggcaaaatgg gaaaatagcc 600

actgcacaca ttagctataa ctctgtctct aaaagactat ctgttactag ttattatgct 660

gggagtaaac ctgcgactct ctcctatgat attgagttac atacagtgct tcctgaatgg 720

gtcagagtag ggttatctgc ttcaactgga caagataaag aaagaaatac cgttcactca 780

tggtctttca cttcaagctt gtggaccaat gtggcgaaga aggagaatga aaacaagtat 840

attacaagag gcgttctgtg atgatatatg tgtatcaatg attttctatg ttataagcat 900

gtaatgtgcg atgagtcaat aatcacaagt acagtgtagt acttgtatgt tgtttgtgta 960

agagtcagtt tgcttttaat aataacaagt gcagttagta cttgt 1005

<210> 23

<211> 286

<212> PRT

<213> Artificial Sequence

<220>

<223> SpDLA.

<400> 23

Met Ala Ser Ser Asn Leu Leu Thr Leu Ala Leu Phe Leu Val Leu Leu

1               5                   10                  15

Thr His Ala Asn Ser Ala Ala Gln Ser Leu Ser Phe Ser Phe Thr Lys

            20                  25                  30

Phe Asp Pro Asn Gln Glu Asp Leu Ile Phe Gln Gly His Ala Thr Ser

        35                  40                  45

Thr Asn Asn Val Leu Gln Val Thr Lys Leu Asp Ser Ala Gly Asn Pro

    50                  55                  60

Val Ser Ser Ser Ala Gly Arg Val Leu Tyr Ser Ala Pro Leu Arg Leu

65                  70                  75                  80

Trp Glu Asp Ser Ala Val Leu Thr Ser Phe Asp Thr Ile Ile Asn Phe

                85                  90                  95

Glu Ile Ser Thr Pro Tyr Thr Ser Arg Ile Ala Asp Gly Leu Ala Phe
            100                 105                 110
Phe Ile Ala Pro Pro Asp Ser Val Ile Ser Tyr His Gly Gly Phe Leu
        115                 120                 125
Gly Leu Phe Pro Asn Ala Asn Thr Leu Asn Asn Ser Ser Thr Ser Glu
    130                 135                 140
Asn Gln Thr Thr Thr Lys Ala Ala Ser Ser Asn Val Val Ala Val Glu
145                 150                 155                 160
Phe Asp Thr Tyr Leu Asn Pro Asp Tyr Gly Asp Pro Asn Tyr Ile His
                165                 170                 175
Ile Gly Ile Asp Val Asn Ser Ile Arg Ser Lys Val Thr Ala Lys Trp
            180                 185                 190
Asp Trp Gln Asn Gly Lys Ile Ala Thr Ala His Ile Ser Tyr Asn Ser
        195                 200                 205
Val Ser Lys Arg Leu Ser Val Thr Ser Tyr Tyr Ala Gly Ser Lys Pro

    210                 215                 220

Ala Thr Leu Ser Tyr Asp Ile Glu Leu His Thr Val Leu Pro Glu Trp

225                 230                 235                 240

Val Arg Val Gly Leu Ser Ala Ser Thr Gly Gln Asp Lys Glu Arg Asn

                245                 250                 255

Thr Val His Ser Trp Ser Phe Thr Ser Ser Leu Trp Thr Asn Val Ala

            260                 265                 270

Lys Lys Glu Asn Glu Asn Lys Tyr Ile Thr Arg Gly Val Leu

        275                 280                 285

<210> 24

<211> 8

<212> PRT

<213> Dolichos lablab

<220>

<221> MOD_RES

<222> (7)

<223> any amino acid.

<400> 24

Thr Asn Asn Val Leu Gln Xaa Thr

1               5

<210> 25

<211> 24

<212> DNA

<213> Artificial Sequence

<220>

<223> MutI primer.

<400> 25

ccataatcgg gatcaagata ggtg                                    24

<210> 26

<211> 24

<212> DNA

<213> Artificial Sequence

<220>

<223> MutII primer.

<400> 26

cacctatctt gatcccgatt atgg 24

<210> 27

<211> 24

<212> DNA

<213> Artificial Sequence

<220>

<223> M1 Forw primer.

<400> 27

aactcagccg cacagtcatt gtca 24

<210> 28

<211> 28

<212> DNA

<213> Artificial Sequence

<220>

<223> APEcoRI primer.

<400> 28

gaattcgacc acgcgtatcg atgtcgac 28

<210> 29

<211> 21

<212> DNA

<213> Artificial Sequence

<220>

<223> Sigforw primer.

<400> 29

gaattcatgg cttcctccaa c                                    21

<210> 30

<211> 28

<212> DNA

<213> Artificial Sequence

<220>

<223> Sigrev primer.

<400> 30

tgactgtgcg gctgagtttg cgtgggtg                             28

<210> 31

<211> 14

<212> PRT

<213> Artificial Sequence

<220>

<223> Peptide corresponding to Pv-FRIL.

<220>

<221> PEPTIDE

<222> (7)

<223> Asn, Cys, or Ser

<400> 31

Ala Gln Ser Leu Ser Phe Xaa Phe Thr Lys Phe Asp Leu Asp

1               5                   10

<210> 32

<211> 14

<212> PRT

<213> Artificial Sequence

<220>

<223> Polypeptide of 18 kDa.

<220>

<221> PEPTIDE

<222> (7)

<223> unknown amino acid.

<400> 32

Ala Gln Ser Leu Ser Phe Xaa Phe Thr Lys Asp Ala Leu Asp

1               5                   10

<210> 33

<211> 14

<212> PRT

<213> Artificial Sequence

<220>

<223> Aminoterminal sequence.

<220>

<221> PEPTIDE

<222> (12>

<223> unknown amino acid.

<400> 33

Thr Asp Ser Arg Val Val Ala Val Glu Phe Asp Xaa Phe Pro

1               5                   10

<210> 34

<211> 13

<212> PRT

<213> Artificial Sequence

<220>

<223> Aminoterminal polypeptide.

<220>

<221> PEPTIDE

<222> (7)

<223> unknown amino acid.

<400> 34

Ala Gln Ser Leu Ser Phe Xaa Phe Lys Phe Asp Pro Asn

1               5                   10

<210> 35

<211> 11

<212> PRT

<213> Artificial Sequence

<220>

<223> Aminoterminal polypeptide.

<400> 35

Thr Asp Ser Arg Val Val Ala Val Glu Asp Phe

1               5                   10

<210> 36

<211> 20

<212> DNA

<213> Artificial Sequence

<220>

<223> Degenerate oligonucleotide PVBeta1.

<220>

<221> misc_feature

<222> (18)

<223> any nucleotide.

<400> 36

ttyacyaart tygayytnga                                    20

<210> 37

<211> 17

<212> DNA

<213> Artificial Sequence

<220>

<223> Degenerate oligonucleotide PVBeta2.

<400> 37

atyttycarg gwgaygc                                    17

<210> 38

<211> 20

<212> DNA

<213> Artificial Sequence

<220>

<223> Degenerate oligonucleotide PVAlfa1.

<400> 38

ttracrtcra twccratrtg                                 20

<210> 39

<211> 23

<212> DNA

<213> Artificial Sequence

<220>

<223> Degenerate oligonucleotide PVAlfa2.

<400> 39

tarttwggrt cratrttrgc rtt 23

<210> 40

<211> 22

<212> DNA

<213> Artificial Sequence

<220>

<223> PV3 PCR-Anchor primer.

<400> 40

caatgtctta caactcacta ag 22

<210> 41

<211> 21

<212> DNA

<213> Artificial Sequence

<220>

<223> PV4 PCR-Anchor primer.

<400> 41

agtgtgggaa gagtgttatt c                                21

<210> 42

<211> 21

<212> DNA

<213> Artificial Sequence

<220>

<223> SPV2 Anchor primer.

<400> 42

accaaagctt tggttttcag a                                    21

<210> 43

<211> 21

<212> DNA

<213> Artificial Sequence

<220>

<223> SPV3 Anchor primer.

<400> 43

tctgaaaacg tttgagtaga g                                    21

<210> 44

<211> 32

<212> DNA

<213> Artificial Sequence

<220>

<223> PVEcoRI primer.

<400> 44

tacatgaatt cgctcagtca ttatctttta ac                    32

<210> 45

<211> 21

<212> DNA

<213> Artificial Sequence

<220>

<223> Sigfor Bg1II primer.

<400> 45

agatctatgg cttcctccaa c 21

<210> 46

<211> 32

<212> DNA

<213> Artificial Sequence

<220>

<223> Sigrev primer.

<400> 46

aaagataatg actgagcggc tgagtttgcg tg 32

<210> 47

<211> 32

<212> DNA

<213> Artificial Sequence

<220>

<223> SpM1forw primer.

<400> 47

cacgcaaact cagccgctca gtcattatct tt 32

<210> 48

<211> 27

<212> DNA

<213> Artificial Sequence

<220>

<223> APXhoI primer.

<400> 48

ctcgaggacc acgcgtatcg atgtcga 27

<210> 49

<211> 105

<212> PRT

<213> Artificial Sequence

<220>

<223> Beta-subunit of the mannose lectin of Gowda et al.

<400> 49

Ala Gln Ser Leu Ser Phe Ser Phe Thr Lys Phe Asp Pro Asn Gln

1               5                   10                  15

Glu Asp Leu Ile Phe Gln Gly Thr Ala Thr Ser Lys Leu Asp Ser Ala

                20                  25                  30

Gly Asn Pro Val Ser Ser Ser Ala Gly Arg Val Leu Tyr Ser Ala Pro

            35                  40                  45

Leu Arg Leu Trp Glu Asp Ser Ala Val Leu Thr Ser Phe Asp Pro Thr

        50                  55                  60

Ile Tyr Ile Phe Thr Asn Tyr Thr Ser Arg Ile Ala Asp Gly Leu Ala

    65                  70                  75

Phe Ile Ala Pro Pro Asp Ser Val Ile Ser Tyr His Gly Gly Phe Leu
80                  85                  90                  95
Gly Leu Phe Pro Asn Ala Ala Glu Ser Gly
                100                 105
<210> 50
<211> 123
<212> PRT
<213> Artificial Sequence
<220>
<223> Beta-subunit of D1-FRIL.
<400> 50
Ala Gln Ser Leu Ser Phe Ser Phe Thr Lys Phe Asp Pro Asn Gln Glu
1               5                   10                  15
Asp Leu Ile Phe Gln Gly His Ala Thr Ser Thr Asn Asn Val Leu Gln
            20                  25                  30

Val Thr Lys Leu Asp Ser Ala Gly Asn Pro Val Ser Ser Ser Ala Gly

        35                  40                  45

Arg Val Leu Tyr Ser Ala Pro Leu Arg Leu Trp Glu Asp Ser Ala Val

    50                  55                  60

Leu Thr Ser Phe Asp Thr Ile Ile Asn Phe Glu Ile Ser Thr Pro Tyr

65                  70                  75                  80

Thr Ser Arg Ile Ala Asp Gly Leu Ala Phe Phe Ile Ala Pro Pro Asp

                85                  90                  95

Ser Val Ile Ser Tyr His Gly Gly Phe Leu Gly Leu Phe Pro Asn Ala

            100                 105                 110

Asn Thr Leu Asn Asn Ser Ser Thr Ser Glu Asn

        115                 120

<210> 51

<211> 132

<212> PRT

<213> Artificial Sequence

<220>

<223> Alpha-subunit of the mannose lectin of Gowda et al.

<400> 51

Ile Ala Glu Ser Asn Val Val Ala Val Glu Phe Asp Thr Asp Tyr Leu

1               5                   10                  15

Asn Pro Asp Tyr Gly Asp Pro Asn Tyr Ile His Ile Gly Ile Asp Val

            20                  25                  30

Asn Ser Ile Arg Ser Lys Val Thr Ala Ser Trp Asp Trp Gln Asn Gly

        35                  40                  45

Lys Ile Ala Thr Ala His Ile Ser Tyr Asn Ser Val Ser Lys Arg Leu

    50                  55                  60

Ser Val Thr Thr Tyr Tyr Pro Gly Arg Gly Lys Pro Ala Thr Ser Tyr

65                  70                  75                  80

Asp Ile Glu Leu His Thr Val Leu Pro Glu Trp Val Arg Val Gly Leu

                85                  90                  95

Ser Ala Ser Thr Gly Gln Asn Ile Glu Arg Asn Thr Val His Ser Trp

            100                 105                 110

Ser Phe Thr Ser Ser Leu Trp Thr Asn Val Ala Lys Val Gly Val Ala

        115                 120                 125

Ser Ile Ser Gly

    130

<210> 52

<211> 141

<212> PRT

<213> Artificial Sequence

<220>

<223> Alpha-subunit of D1-FRIL.

<400> 52

Gln Thr Thr Thr Lys Ala Ala Ser Ser Asn Val Val Ala Val Glu Phe

1               5                   10                  15

Asp Thr Tyr Leu Asn Pro Asp Tyr Gly Asp Pro Asn Tyr Ile His Ile

            20                  25                  30

Gly Ile Asp Val Asn Ser Ile Arg Ser Lys Val Thr Ala Lys Trp Asp

        35                  40                  45

Trp Gln Asn Gly Lys Ile Ala Thr Ala His Ile Ser Tyr Asn Ser Val

    50                  55                  60

Ser Lys Arg Leu Ser Val Thr Ser Tyr Tyr Ala Gly Ser Lys Pro Ala

65                  70                  75                  80

Thr Leu Ser Tyr Asp Ile Glu Leu His Thr Val Leu Pro Glu Trp Val

                85                  90                  95

Arg Val Gly Leu Ser Ala Ser Thr Gly Gln Asp Lys Glu Arg Asn Thr

            100                 105                 110

Val His Ser Trp Ser Phe Thr Ser Ser Leu Trp Thr Asn Val Ala Lys

        115                 120                 125

Lys Glu Asn Glu Asn Lys Tyr Ile Thr Arg Gly Val Leu
    130                 135                 140

<210> 53
<211> 64
<212> DNA
<213> Artificial Sequence

<220>
<223> Recombinant expression vector.

<400> 53
ctggttccgc gtggatcccc ggaattcatg cccggttcga ctcgagcggc cgcatcgtga 60

ctga 64

<210> 54
<211> 54
<212> DNA

<213> Artificial Sequence

<220>

<223> Recombinant expression vector.

<400> 54

ctggttccgc gtggatcccc ggaattcatg ctcgagcggc cgcatcgtga ctga          54

<210> 55

<211> 237

<212> PRT

<213> Artificial Sequence

<220>

<223> Mannose lectin of Gowda et al.

<400> 55

Ala Gln Ser Leu Ser Phe Ser Phe Thr Lys Phe Asp Pro Asn Gln Glu

1               5                   10                  15

Asp Leu Ile Phe Gln Gly Thr Ala Thr Ser Lys Leu Asp Ser Ala Gly
            20                  25                  30
Asn Pro Val Ser Ser Ser Ala Gly Arg Val Leu Tyr Ser Ala Pro Leu
        35                  40                  45
Arg Leu Trp Glu Asp Ser Ala Val Leu Thr Ser Phe Asp Pro Thr Ile
    50                  55                  60
Tyr Ile Phe Thr Asn Tyr Thr Ser Arg Ile Ala Asp Gly Leu Ala Phe
65                  70                  75                  80
Ile Ala Pro Pro Asp Ser Val Ile Ser Tyr His Gly Gly Phe Leu Gly
                85                  90                  95
Leu Phe Pro Asn Ala Ala Glu Ser Gly Ile Ala Glu Ser Asn Val Val
            100                 105                 110
Ala Val Glu Phe Asp Thr Asp Tyr Leu Asn Pro Asp Tyr Gly Asp Pro
        115                 120                 125
Asn Tyr Ile His Ile Gly Ile Asp Val Asn Ser Ile Arg Ser Lys Val

    130                 135                 140

Thr Ala Ser Trp Asp Trp Gln Asn Gly Lys Ile Ala Thr Ala His Ile

145                 150                 155                 160

Ser Tyr Asn Ser Val Ser Lys Arg Leu Ser Val Thr Thr Tyr Tyr Pro

                165                 170                 175

Gly Arg Gly Lys Pro Ala Thr Ser Tyr Asp Leu Glu Leu His Thr Val

            180                 185                 190

Leu Pro Glu Trp Val Arg Val Gly Leu Ser Ala Ser Thr Gly Gln Asn

        195                 200                 205

Ile Glu Arg Asn Thr Val His Ser Trp Ser Phe Thr Ser Ser Leu Trp

    210                 215                 220

Thr Asn Val Ala Lys Val Gly Val Ala Ser Ile Ser Gly

225                 230                 235

<210> 56

<211> 279

<212> PRT

<213> Artificial Sequence

<220>

<223> PvFRIL.

<400> 56

Ala Gln Ser Leu Ser Phe Asn Phe Thr Lys Phe Asp Leu Asp Gln Lys

1               5                   10                  15

Asp Leu Ile Phe Gln Gly Asp Ala Thr Ser Thr Asn Asn Val Leu Gln

            20                  25                  30

Leu Thr Lys Leu Asp Ser Gly Gly Asn Pro Val Gly Ala Ser Val Gly

        35                  40                  45

Arg Val Leu Phe Ser Ala Pro Phe His Leu Trp Glu Asn Ser Met Ala

    50                  55                  60

Val Ser Ser Phe Glu Thr Asn Leu Thr Ile Gln Ile Ser Thr Pro His

65                  70                  75                  80

Pro Tyr Tyr Ala Ala Asp Gly Phe Ala Phe Phe Leu Ala Pro His Asp

                85                  90                  95

Thr Val Ile Pro Pro Asn Ser Trp Gly Lys Phe Leu Gly Leu Tyr Ser

            100                 105                 110

Asn Val Phe Arg Asn Ser Pro Thr Ser Glu Asn Gln Ser Phe Gly Asp

        115                 120                 125

Val Asn Thr Asp Ser Arg Val Val Ala Val Glu Phe Asp Thr Phe Pro

    130                 135                 140

Asn Ala Asn Ile Asp Pro Asn Tyr Arg His Ile Gly Ile Asp Val Asn

145                 150                 155                 160

Ser Ile Lys Ser Lys Glu Thr Ala Arg Trp Glu Trp Gln Asn Gly Lys

                165                 170                 175

Thr Ala Thr Ala Arg Ile Ser Tyr Asn Ser Ala Ser Lys Lys Ser Thr

            180                 185                 190

Val Thr Thr Phe Tyr Pro Gly Met Glu Val Val Ala Leu Ser His Asp

        195                 200                 205

Val Asp Leu His Ala Glu Leu Pro Glu Trp Val Arg Val Gly Leu Ser

    210                 215                 220

Ala Ser Thr Gly Glu Glu Lys Gln Lys Asn Thr Ile Ile Ser Trp Ser

225                 230                 235                 240

Phe Thr Ser Ser Leu Lys Asn Asn Glu Val Lys Glu Pro Lys Glu Asp

                245                 250                 255

Met Tyr Ile Ala Asn Val Val Arg Ser Tyr Thr Trp Ile Asn Asp Val

            260                 265                 270

Leu Ser Tyr Ile Ser Asn Lys

        275

<210> 57

<211> 254

<212> PRT

<213> Artificial Sequence

<220>

<223> PHA-E.

<400> 57

Ala Ser Gln Thr Ser Phe Ser Phe Gln Arg Phe Asn Glu Thr Asn Leu

1               5                   10                  15

Ile Leu Gln Arg Asp Ala Thr Val Ser Ser Lys Gly Gln Leu Arg Leu

            20                  25                  30

Thr Asn Val Asn Asp Asn Gly Glu Pro Thr Leu Ser Ser Leu Gly Arg

        35                  40                  45

Ala Phe Tyr Ser Ala Pro Ile Gln Ile Trp Asp Asn Thr Thr Gly Ala

    50                  55                  60

Val Ala Ala Ser Pro Thr Ser Phe Thr Phe Asn Ile Asp Val Pro Asn

65                  70                  75                  80

Asn Ser Gly Pro Ala Asp Gly Leu Ala Phe Val Leu Leu Pro Val Gly

                85                  90                  95

Ser Gln Pro Lys Asp Lys Gly Gly Leu Leu Gly Leu Phe Asn Asn Tyr

            100                 105                 110

Lys Tyr Asp Ser Asn Ala His Thr Val Ala Val Glu Phe Asp Thr Leu

        115                 120                 125

Tyr Asn Val His Trp Asp Pro Lys Pro Arg His Ile Gly Ile Asp Val

    130                 135                 140

Asn Ser Ile Lys Ser Ile Lys Thr Thr Thr Trp Asp Phe Val Lys Gly

145                 150                 155                 160

Glu Asn Ala Glu Val Leu Ile Thr Tyr Asp Ser Ser Thr Lys Leu Leu

                165                 170                 175

Val Ala Ser Leu Val Tyr Pro Ser Leu Lys Thr Ser Phe Ile Val Ser

            180                 185                 190

Asp Thr Val Asp Leu Lys Ser Val Leu Pro Glu Trp Val Ile Val Gly

        195                 200                 205

Phe Thr Ala Thr Thr Gly Ile Thr Lys Gly Asn Val Glu Thr Asn Asp

    210                 215                 220

Ile Leu Ser Trp Ser Phe Ala Ser Lys Leu Ser Asp Gly Thr Thr Ser
225                 230                 235                 240

Glu Ala Leu Asn Leu Ala Asn Phe Ala Leu Asn Gln Ile Leu
                245                 250

<210> 58
<211> 20
<212> PRT
<213> Artificial Sequence

<220>
<223> Synthetic Peptide

<220>
<221> MOD_RES
<222> (7)
<223> Variable Amino Acid

<400> 58
Asp Ser Ser Thr Ser Glu Xaa Gln Thr Thr Thr Lys Ala Ala Ser Ser
1               5                   10                  15

Asn Val Val Ala
            20

<210> 59
<211> 13
<212> PRT
<213> Artificial Sequence

<220>
<223> Synthetic Peptide

<220>
<221> MOD_RES
<222> (7)
<223> Variable Amino Acid

<400> 59
Asp Ser Ser Thr Ser Glu Xaa Gln Thr Thr Thr Lys Ala
1               5                   10

<210> 60

<211> 20

<212> PRT

<213> Artificial Sequence

<220>

<223> Synthetic Peptide

<400> 60

Thr Thr Thr Lys Ala Ala Ser Ser Asn Val Val Ala Val Glu Phe Lys

1               5                   10                  15

Thr Tyr Leu Asn

            20

<210> 61

<211> 30

<212> PRT

<213> Artificial Sequence

<220>

<223> Synthetic Peptide

<400> 61

Ala Gln Ser Leu Ser Phe Phe Ser Phe Thr Lys Phe Asp Pro Asn Gln

1               5                   10                  15

Glu Asp Leu Ile Phe Gln His Ala Thr Ser Thr Asn Asn Val

            20                  25                  30

YamFril: partial mRNA sequence
ACGAAGTTCGACAGCGACCAAAAGGATCTTATGTTCCAAGGTCATACCATTTCTAGCAGC
AATGTCATACAACTCACCAAGTTAGACAGTAATGGAAACCCTGTGAGTACCAGTGTGGGA
AGAGTGTTATACTCTGCACCATTGCGCCTTTGGGAAAGCTCTACAGTAGTGTCAACCTTT
GAGACCACTTTCACCTTTCAAATCTCAACACCTTACACTAGTCCTCCTGGTGATGGGCTC
GCCTTCTTCCTTGCACCATATGACACTGTCATCCCTCCAAATTCTGCTGGCAATCTTCTT
GGACTCTTTCCTAACTTAAATGCTTTAAGAAACTCCACCACCAGTAAAGAAACCACTATT
GATGTCAATGCTGCATCTAACAACGTTGTTGCCGTTGAATTTGACACCTACCCTAACGAC
AATATTGGTGATCCAAGATACAAACACATTGGAATCGATGTCAACTCTATCAGGTCCAAG
GCAACTGTTGCGTGGGACTGGCAAAATGGGAAAACAGCCACTGCACACATCAGCTATAAC
TCTGCCTCTAAAAGACTATCTGTTACTACTTTTTATCCTGGGGGTAAAGCTGTGAGTCTT
TCCCATGACGTTGAGCTCACTCAAGTGCTTCCTCAATGGATTAGAGTAGGGTTCTCTGCT
TCAACAGGATTAGAGAAA (SEQ ID NO: 7)

YamFril: deduced amino acid sequence
AQSVSFTFTKFDSDQKDLMFQGHTISSSNVIQLTKLDSNGNPVSTSVGRVLYSAPLRLWE
SSTVVSTFETTFTFQISTPYTSPPGDGLAFFLAPYDTVIPPNSAGNLLGLFPNLNALRNS
TTSKETTIDVNAASNNVVAVEFDTYPNDNIGDPRYKHIGIDVNSIRSKATVAWDWQNGKT

ATAHISYNSASKRLSVTTFYPGGKAVSLSHDVELTQVLPQWIRVGFSASTGLEK (SEQ ID
NO: 8)

## Claims

1. Use of a therapeutically effective amount of a composition containing a FRIL protein comprising an amino acid sequence that has at least about 95% amino acid sequence identity to the amino acid sequence set forth in SEQ ID NO:6,
wherein the FRIL protein binds to a normally glycosylated FLT3 receptor, and preserves hematopoietic cells,
for the preparation of a medicament for treatment of a condition **characterized by** hematopoietic progenitor cell-depleting activity of a radiotherapeutic or a chemotherapeutic treatment or a combination of a radiotherapeutic and a chemotherapeutic treatment,
wherein the therapeutically effective amount of said composition alleviates or reduces the hematopoietic progenitor cell-depleting activity of said therapeutic treatment in the patient;
wherein the medicament is to be administered prior to the radiotherapeutic or chemotherapeutic treatment; and
wherein the medicament is to be administered in an amount of between about 500ng of the FRIL protein/kg total body weight and about 5mg/kg total body weight per day.

2. The use of claim 1, wherein said FRIL protein is encoded by the nucleic acid sequence set forth in SEQ ID NO:5.

3. The use of claim 1, wherein said FRIL protein is encoded by a nucleic acid sequence that has at least about 95% nucleic acid sequence identity to the nucleic acid sequence set forth in SEQ ID NO:5.

4. The use of claim 1, wherein said FRIL protein has the amino acid sequence set forth in SEQ ID NO:6.

5. The use of claim 1, wherein said FRIL protein has at least about 95% amino acid sequence identity to the amino acid sequence set forth in SEQ ID NO:6.

6. The use of claim 1, wherein said FRIL protein is a recombinant protein.

7. The use of claim 1, wherein the hematopoietic progenitor cells in the patient being treated with the therapeutic treatment are depleted, either by killing the progenitor cells or by inducing the progenitor cells to undergo irreversible differentiation.

8. The use of claim 1, wherein the patient is human.

9. The use of claim 3, wherein the patient has cancer.

10. The use of claim 1, wherein the chemotherapeutic is selected from the group consisting of cytarabine, doxorubicin, and 5-fluorouracil.

11. The use of a FRIL protein as in claim 1, wherein said FRIL protein is administered daily for 1-5 days prior to the radiotherapeutic or chemotherapeutic treatment.

12. The use of a FRIL protein as in claim 1, wherein said FRIL protein is administered in an amount of between 500ng/kg and 500µg/kg total body weight per day.

13. The use of a FRIL protein as in claim 1, wherein said FRIL protein is administered in an amount of between 5µg/kg and 50µg/kg total body weight per day.

14. The use of a FRIL protein as in claim 1, wherein said FRIL protein is administered in an amount of 50µg/kg total body weight per day.
